(19) Europäisches Patentamt
European Patent Office
Office européen des brevets

(11) EP 4 233 851 A1

(12) EUROPEAN PATENT APPLICATION

(43) Date of publication:
**30.08.2023 Bulletin 2023/35**

(21) Application number: **22158721.5**

(22) Date of filing: **25.02.2022**

(51) International Patent Classification (IPC):
***A61K 31/00*** (2006.01) ***A61K 31/506*** (2006.01)
***A61K 31/5377*** (2006.01) ***A61P 9/00*** (2006.01)
***A61P 21/00*** (2006.01) ***A61P 25/28*** (2006.01)
***A61P 43/00*** (2006.01)

(52) Cooperative Patent Classification (CPC):
**A61K 31/00; A61K 31/506; A61K 31/5377;
A61P 9/00; A61P 21/00; A61P 25/28; A61P 43/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Charité - Universitätsmedizin Berlin
10117 Berlin (DE)**

(72) Inventor: **Scheibenbogen, Carmen
12203 Berlin (DE)**

(74) Representative: **Hertin und Partner
Rechts- und Patentanwälte PartG mbB
Kurfürstendamm 54/55
10707 Berlin (DE)**

(54) **A SOLUBLE GUANYLAT CYCLASE ACTIVATOR FOR TREATING CHRONIC VASCULAR DYSFUNCTION**

(57) The invention relates to a pharmaceutical composition comprising a soluble guanylate cyclase stimulator or salt thereof for use in the treatment of a medical condition comprising a chronic vascular dysfunction occurring or persisting after onset of an infection in a human subject. In preferred embodiments, the invention relates to a pharmaceutical composition comprising a soluble guanylate cyclase stimulator or salt thereof, preferably vericiguat, for use in the treatment of a chronic vascular dysfunction associated with vascular constriction, endothelial dysfunction and/or hypo-perfusion.

EP 4 233 851 A1

## Description

The invention relates to the field of pharmaceutical compositions, combinations, and the treatment of medical conditions associated with chronic vascular dysfunction, such as Chronic Fatigue Syndrome (ME/CFS) and Post COVID Syndrome.

The invention relates to a pharmaceutical composition comprising a soluble guanylate cyclase stimulator or salt thereof for use in the treatment of a medical condition comprising a chronic vascular dysfunction occurring or persisting after onset of an infection in a human subject. In preferred embodiments, the invention relates to a pharmaceutical composition comprising a soluble guanylate cyclase stimulator or salt thereof, preferably vericiguat, for use in the treatment of a chronic vascular dysfunction associated with vascular constriction, endothelial dysfunction and/or hypo-perfusion.

In preferred embodiments, the invention relates to the treatment of a medical condition of Chronic Fatigue Syndrome and/or Post COVID Syndrome. The invention further relates to a combination of a soluble guanylate cyclase stimulator or salt thereof, preferably vericiguat, and at least one other treatment, such as a guideline treatment for Chronic Fatigue syndrome or Post COVID Syndrome.

## BACKGROUND OF THE INVENTION

Chronic fatigue syndrome, myalgic encephalomyelitis (ME/CFS), and postviral fatigue syndrome are classified as neurological diseases according to the 10th version of the International Statistical Classification of Diseases and Related Health Problems, a medical classification list of the World Health Organization (WHO), with the number G93.3 in the ICD list. ME/CFS is a chronic disease that has as its leading symptom an exceptionally rapid physical and mental exhaustibility, and in extreme cases can lead to extensive disability and a need for long-term care. Despite unexplained causes and mechanisms of development, the syndrome is internationally recognized as an independent clinical picture. One of the most common recognized associations is a previous infection with human pathogens, particularly viral and bacterial infections.[1]

ME/CFS is considered a debilitating chronic disease with a worldwide prevalence of 0.3% to 0.8%. Severe mental and physical fatigue, sleep disturbances, chronic pain, shortness of breath and psychological symptoms - regardless of the severity of illness - are the main symptoms of ME/CFS. The best distinguishing symptoms that differentiate ME/CFS from chronic fatigue in multiple sclerosis are flu-like symptoms and intolerance to mental and physical exertion (PEM) that triggers PEM for more than 14 hours.[2]

In December 2019, a novel SARS coronavirus 2 (SARS-CoV-2) emerged in Wuhan, Hubei Province, China. Subsequently, SARS-CoV-2 spread rapidly in China and Asian countries before triggering a global pandemic of a new disease called 2019 coronavirus disease (COVID-19).

Soon after the outbreak of the pandemic, there were reports of patients with persistent or reoccurring symptoms referred to as Post COVID Syndrome (PCS) or long COVID-19. Although most patients with COVID-19 have only mild to moderate symptoms such as cough and fever or no symptoms at all, 10-30% of these patients experience persistent symptoms lasting longer than 6 months. PCS results in persistent fatigue, impaired physical and cognitive function, headache, shortness of breath, palpitations, and many other symptoms that interfere with daily activities in many patients and leads to the inability to work in 22% of patients, as well as causing other symptoms similar to those of ME/CFS.

The pathomechanism of ME/CFS, as well as that of PCS, are not fully understood, but immune dysregulation, inflammation, and vascular dysfunction have been observed. The disease not only has individual consequences but represents a sociological and economic burden due to the loss of family members and workers, increased burden on families and necessary health care measures and cost. Although the disease was first described in 1955, treatments are typically symptomatic but not causal and do not reliably lead to any significant improvement in patient wellbeing. Many patients suffer from the disease for decades with no prospect of a cure. Social and socioeconomic consequences are high.[1-4]

Despite the high medical need, established and efficacious treatment options for ME/CFS and PCS still do not exist and are urgently needed. Symptomatic treatments include physiotherapy and psychotherapy, as well as symptomatic drug treatment and vitamin supplementation, according to the S1 guidelines that exist for PCS and ME/CFS.

## SUMMARY OF THE INVENTION

In light of the prior art, the technical problem underlying the present invention is to provide alternative or improved means for the treatment of a medical condition comprising chronic vascular dysfunction occurring or persisting after onset of an infection. The technical problem may also be viewed as the provision of means for the treatment of a medical condition occurring or persisting after onset of first symptoms of an infection, more particularly a viral infection such as a SARS coronavirus infection. The technical problem may also be viewed as the provision of means for the treatment

of a post-infection-associated endothelial dysfunction and hypoperfusion, chronic fatigue syndrome, Post COVID Syndrome, myalgic encephalomyelitis, and/or postviral fatigue syndrome failure.

This problem is solved by the features of the independent claims. Preferred embodiments of the present invention are provided by the dependent claims.

In one aspect, the invention therefore relates to a pharmaceutical composition comprising a soluble guanylate cyclase stimulator for use in the treatment of a medical condition comprising a chronic vascular dysfunction occurring or persisting after onset of an infection in a human subject.

In some embodiments, the invention relates to the pharmaceutical composition comprising a soluble guanylate cyclase (sGC) stimulator or salt thereof, preferably vericiguat, for use in the treatment of chronic vascular dysfunction occurring or persisting after onset of an infection, such as a coronavirus infection, comprising the treatment of chronic fatigue syndrome (CFS), Post COVID Syndrome, myalgic encephalomyelitis (ME), and corresponding methods of treatment. As used herein, ME and CFS refer to the same syndrome and therefore can be summarized as ME/CFS.

In some embodiments, the subject is or has been treated additionally with a guideline treatment for ME/CFS or a guideline treatment for Post COVID Syndrome. In some embodiments, the guideline treatment is not or does not comprise a sGC stimulator. In some embodiments, the invention relates to the combined administration of a therapeutically effective amount of a sGC stimulator, preferably vericiguat, and a guideline treatment for ME/CFS or Post COVID Syndrome.

The invention further relates to a pharmaceutical composition comprising a sGC stimulator for use in the treatment of a chronic vascular dysfunction associated with an infection in a human subject.

In one embodiment, symptoms of a chronic vascular dysfunction occur after onset of first symptoms of an infection in a human subject. In one embodiment, symptoms of a chronic vascular dysfunction occur after onset of the first symptoms a viral infection in a human subject.

In one embodiment, symptoms of a chronic vascular dysfunction persist after an acute phase of an infection in a human subject. In one embodiment, symptoms of a chronic vascular dysfunction persist after an acute phase of a viral infection in a human subject.

The medical condition to be treated using the present invention may therefore be defined as any condition associated with a chronic vascular dysfunction. In embodiments, this dysfunction may have continued (persisted) from the acute phase of the infection, for example as a symptom of infection that did not reduce naturally after the acute phase of the infection. The dysfunction may have arisen after the acute phase of the infection, or may have returned at a later stage after having been present in the acute phase of the infection and subsequently lost.

In some embodiments, the infection is a viral infection, for example a viral infection caused by coronavirus, respiratory tract disease virus, herpesvirus (HHV6, HSV-1, HSV-2 or VZV), Epstein Barr virus. In some embodiments, the infection is a bacterial infection, for example a bacterial infection caused by legionella, chlamydia, borreliosis or coxial bacteria.

The invention further relates to a pharmaceutical composition comprising a sGC stimulator for use in the treatment of a medical condition associated with an infection-related chronic disturbance persisting or developing after an infection in a human subject. The invention further relates to a pharmaceutical composition comprising a sGC stimulator for use in the treatment of a medical condition associated with a viral infection-related chronic disturbance persisting or developing after a viral infection in a human subject.

The invention further relates to a pharmaceutical composition comprising a sGC stimulator for use in the treatment of a medical condition associated with a chronic syndrome occurring or persisting after a viral infection in a human subject, wherein the chronic syndrome is preferably a chronic vascular syndrome.

In some embodiments, the treatment recommended by the guidelines for ME/CFS or Post COVID Syndrome patients is a non sGC stimulator drug, wherein the non sGC stimulator drug drug is preferably a pain reliever, antibiotic or vitamin supplement.

As used herein, the non sGC stimulator drug recommended by the guidelines for ME/CFS or Post COVID Syndrome patients, may be referred to as a guideline drug. The invention also relates to the combined administration of a therapeutically effective amount of a sGC stimulator or salt thereof, preferably vericiguat, and a therapeutically effective amount of the guideline drug, in such treatment.

In one embodiment, one or more clinical parameters, for example those as described herein, are above or below a relevant reference level for ME/CFS or Post COVID Syndrome patients compared to a healthy control subject or healthy patient population.

In one embodiment, one or more laboratory parameters, as described herein, are out of a healthy range compared to one or more reference levels in ME/CFS or Post COVID Syndrome patients compared to a healthy control subject or healthy patient population.

In one embodiment, one or more biomarkers are above a reference level in ME/CFS and/or Post COVID Syndrome patients compared to a healthy control subject or healthy patient population, wherein said level is compared to a level in a healthy control subject or healthy patient population and is determined in a sample comprising and/or

derived from a bodily fluid and/or cells from the subject, preferably from blood, serum or plasma.

In one embodiment, one or more biomarkers are below a reference level in ME/CFS and/or Post COVID Syndrome patients compared to a healthy control subject or healthy patient population, wherein said level is compared to a level in a healthy control subject or healthy patient population and is determined in a sample comprising and/or derived from a bodily fluid and/or cells from the subject, preferably from blood, serum or plasma. For example, a biomarker can also be used to indicate a change in the expression or state of a protein that correlates with the risk or progression of a disease, syndrome or dysfunction, or even with the response of the disease to a given treatment.

In one embodiment, the reference level is also a value, such as value of a clinical or laboratory parameter, of a healthy control subject or a healthy patient population.

By way of example, increased levels of endothelin-1, NT-proBNP, interleukin 8, CK, cytokines, autoantibodies, and/or CrP in subjects can lead to or be associated with a ME/CFS or Post COVID Syndrome, particularly for patients who had or are suspected to have had an infection, such as COVID19, wherein the patient exhibits for example endothelial dysfunction, vascular constriction, vascular damage, cognitive impairment, mitochondrial dysfunction, vascular stiffness, retinal endothelial dysfunction, and/or hypoperfusion.

In embodiments, decreased levels of bilirubin, immunoglobins, ANG-2, ACE2, and/or ACE-1 in subjects can lead to or be associated with a ME/CFS or Post COVID Syndrome, particularly in patients who had or are suspected to have had an infection, such as COVID19, wherein the patient exhibits for example endothelial dysfunction, vascular constriction, vascular damage, cognitive impairment, mitochondrial dysfunction, vascular stiffness, retinal endothelial dysfunction, and/or hypo-perfusion.

The exact pathomechanism of ME/CFS and Post COVID Syndrome are not yet fully characterized and may include dysregulation of the immune system, autonomic nervous system, vascular system and/or metabolism.

Without being bound by theory, SARS-CoV-2 infection triggers a strong inflammatory response in a subject and there is evidence for autoimmunity triggered by COVID-19.

SARS-CoV-2 infects endothelial cells via ACE2. As shown in Example 3, endothelial dysfunction and dysregulated levels of the endothelial marker ET-1 and Ang-2 occur in Post COVID Syndrome patients on average 8 months after mild to moderate COVID-19, as well as in ME/CFS. Surprisingly, the inventors found a paradoxical positive correlation of RHI, a marker of endothelial dysfunction, with both age and blood pressure in Post COVID Syndrome, suggesting that endothelial dysfunction (ED) develops independently of classic cardiovascular risk factors in these patients and that vascular stiffness may even contribute to stabilization of vessel diameter.

Remarkably, PCS patients with and without ME/CFS showed increased levels of the endothelial biomarker ET-1, a measure of hypo-perfusion, whereas convalescents had normal levels. Hypo-perfusion also associates with ME/CFS and Post COVID Syndrome. Also, pro-inflammatory mediator IL-8 is elevated ME/CFS and Post COVID Syndrome patients, wherein IL-8 is mainly produced by endothelial cells and monocytes. Endothelial inflammation occurs and persists in ME/CFS and Post COVID Syndrome. ET-1 and IL-8 may therefore both be biomarkers of endothelial involvement and represent a promising therapeutic target in ME/CFS and Post COVID Syndrome.

Endothelial dysfunction and inflammation are associated with dysregulation of the NOsGC-cGMP pathway, having sGC as a key enzyme. The sGC has vasodilatory and anti-inflammatory effects via the production of cGMP. The inventors have thus for the first time elucidated a mechanism for ME/CFS and Post COVID Syndrome patients and for endothelial dysfunction in these patients and identified sGC as a target for therapy. sGC represents a promising anti-inflammatory and vasodilatory target in the therapy of endothelial dysfunction, vascular constriction, vascular damage, mitochondrial dysfunction, vascular stiffness, retinal endothelial dysfunction, and/or hypo-perfusion. Stimulators of sGC, such as vericiguat, increase the intracellular cGMP levels leading to relaxation and vasodilation.

It has proven challenging to elucidate the significance of clinical and laboratory parameters, such as endothelial dysfunction, activity level, fatigue, HGS, NT-pro BNP, IL-8, CrP, bilirubin, in pathogenesis and pathophysiology of ME/CFS and Post COVID Syndrome, and to identify compositions that have a therapeutic effect on these factors. In the prior art, ME/CFS and Post COVID Syndrome therapies have not proven effective in successfully addressing endothelial dysfunction in treated subjects.

Of note is that the inventors have further found that decreased handgrip strength (HGS) and Chalder Fatigue Score levels compared to healthy controls, thus indicating mental and physical fatigue, correlated with laboratory parameters associated with endothelial function and muscle perfusion and with the degree of subjective fatigue. The inventors further found that endothelial dysfunction (ED) is evident in the majority of ME/CFS and a subset of PCS patients.

In a Post COVID Syndrome cohort, a significant inverse correlation of Fmax1/2 and Fmean1/2 with IL-8 and CrP was found. IL-8 and CrP, which are inversely associated with HGS, have a negative effect on endothelial function.

In the ME/CFS cohort, values of HGS (Fmax1/2 and Fmean1/2) correlate significantly inversely with NT-pro BNP. As also shown, HGS values of ME/CFS and Post COVID Syndrome patients correlate positively with hemoglobin, erythrocytes, and creatinine.

HGS values of Post COVID Syndrome patients also correlate positively with ACE2 and bilirubin. The HGS levels of ME/CFS patients also correlate positively with ferritin levels.

NT-pro BNP is a marker of heart failure and is released by distension of cardiomyocytes, but no indication of impaired cardiac function was found in the patients employed in the examples. Furthermore, BNP is produced in ischemic skeletal muscle satellite cells as a potential paracrine regulator of vasodilation and vascular regeneration through activation of plasmatic guanylate cyclase receptors.

Of note is that all of NT-pro BNP, IL-8 and CrP, hemoglobin, erythrocytes, creatinine, ACE2, bilirubin and ferritin are biomarkers of endothelial dysfunction and hypo-perfusion. Hb/erythrocytes and creatinine (oxygen and energy supply, respectively), ACE2 (vasodilation), and bilirubin (vasodilation and antioxidant) have a protective role on endothelial and muscle function.

Therefore, the inventors have surprisingly found that endothelial dysfunction and hypoperfusion represent an underlying potentially causative factor in muscle fatigue, especially in the ME/CFS and PCS patient groups.

Without being bound by theory, the treatment of the present invention seeks to stimulate sGC and increase cGMP levels in ME/CFS and/or Post COVID Syndrome patients, thereby improving relaxation, vasodilation and/or endothelial function.

In other aspects or embodiments, the invention seeks to aid in the reduction of risk for progression of chronic vascular dysfunction after infection, preferably viral infection, more preferably coronavirus or Epstein Barr virus, even more preferably SARS-CoV-2.

The present invention provides a solution to these challenges, as described in further detail below.

As described at length herein, the composition comprising sGC or salt thereof, preferably vericiguat, represents a novel approach towards developing an effective measure to treat a medical condition comprising chronic vascular dysfunction, particularly endothelial dysfunction, in ME/CFS or Post COVID Syndrome subjects, wherein said subject preferably had or is suspected to has had an infection, such as Epstein Barr virus or SARS-COV-2 infection.

At present, said patients are not appropriately treated as no effective medication may be available for the patient group described herein. As evident from the experimental support provided (Examples 1-3), diminished hand grip strength, Reactive Hyperemia Index <1.8 and/or increased levels of ET-1, IL-8, CrP, hemoglobin, and/or NT-proBNP significantly correlates with endothelial dysfunction in ME/CFS or Post COVID Syndrome. sGC activity in ME/CFS or Post COVID Syndrome subjects is unexpectedly associated with increased inflammation and endothelial dysfunction in said subjects.

In one embodiment, hand grip strength, Reactive Hyperemia Index, ET-1, IL-8, CrP, hemoglobin, and/or NT-proBNP can be used as clinical and/or laboratory parameters to determine or otherwise assess endothelial dysfunction in ME/CFS or Post COVID Syndrome patients.

Through employing the treatments of the present invention, the mechanisms underlying disease progression may be directly addressed by stimulating sGC to produce cGMP and reduce inflammation and endothelial dysfunction.

In one embodiment, the physiological response of sGC stimulation for producing cGMP can be determined by quantifying one or more of ET-1, IL-8, CrP, hemoglobin, and/or NT-proBNP in cells, blood, plasma and/or serum from a patient, preferably blood or serum from ME/CFS or Post COVID Syndrome patients treated with a sGC stimulator, such as vericiguat.

In one embodiment, the physiological response of sGC stimulation for producing cGMP can be measured by evaluating clinical parameters of ME/CFS or Post COVID Syndrome patients treated with sGC stimulator, such as vericiguat. In some embodiments, the evaluation of clinical parameters includes monitoring persistence and/or progression of symptoms in ME/CFS or Post COVID Syndrome patients treated with a sGC stimulator, such as vericiguat. In some embodiments, the clinical parameters for monitoring physiological response comprise those of the guidelines described herein. In some embodiments, the clinical parameters for monitoring physiological response comprise one or more of questionnaires (SF-36 PF, Chalder Fatigue Score, CCC Symptom Score, Bell disabling scale, COMPASS-31), muscle fatigue (hand grip strength test, cognitive testing (SDMT), hemodynamics (passive standing test), ANS (heart rate variability), endothelial function (EndoPAT), retinal vessel function (dynamic vessel analysis, optical coherence tomography angiography and/or cerebral blood flow (arterial spin labeling).

In one embodiment, the effectiveness and physiological response of the treatment, as described herein, is determined by values of clinical and laboratory parameters compared to said value determined before treatment initiation. In one embodiment, values of clinical and laboratory parameters are determined as described herein, and in the Examples 1-3.

In some embodiments, the chronic vascular dysfunction occurs after the onset of first symptoms of the infection, or after the acute phase of the infection, for example within six months after the acute phase of said infection.

In one embodiment, a chronic condition of ME/CFS or Post COVID Syndrome occurs after the onset of first symptoms of the infection, or after the acute phase of the infection. In one embodiment, a chronic condition of ME/CFS or Post COVID Syndrome persists from the onset of first symptoms of the infection, or from the onset of the chronic vascular dysfunction.

In one embodiment, symptoms of an infection may persist after an acute phase of infection and lead to ME/CFS. In one embodiment, symptoms of an infection may occur or re-occur after an acute phase of infection and lead to

ME/CFS. In one embodiment, the subject that had an acute phase of infection was fully or nearly fully recovered before ME/CFS become evident. In one embodiment, the chronic vascular dysfunction occurs independently of an infection.

In one embodiment, symptoms of SARS-CoV-2 infection may persist after an acute phase of an infection and lead to Post COVID Syndrome. In one embodiment, symptoms of an infection may occur or re-occur after an acute phase of SARS-CoV-2 infection and lead to Post COVID Syndrome. In one embodiment, the subject that had an acute phase of SARS-CoV-2 infection was fully or nearly fully recovered before Post COVID Syndrome. In one embodiment, the chronic vascular dysfunction occurs independently of a SARS-CoV-2 infection.

In one embodiment, the time interval between the end of the acute phase of infection and before first symptoms of ME/CFS occur, comprises at least 1 week, 2 weeks, 3 weeks, 1 month, 2 months, 3 months, 4 months, 5 months, 6 months, or more than 6 months.

In one embodiment, ME/CFS comprises chronic vascular dysfunction. One aspect of the invention relates to the chronic vascular dysfunction occurring or persisting after onset of infection in a human subject.

In one embodiment, the chronic vascular dysfunction occurs after the onset of the first symptoms of an infection or after the acute phase of an infection, for example, within six months after the acute phase of the viral infection. In one embodiment, the time interval between the end of the acute phase of SARS-CoV-2 infection and before first symptoms of Post COVID Syndrome occur, comprises at least 1 week, 2 weeks, 3 weeks, 1 month, 2 months, 3 months, 4 months, 5 months, 6 months, or more than 6 months.

In one embodiment, sGC treatment improves symptoms of ME/CFS, wherein acute ME/CSF preferably occurs after the acute phase of an infection, such as an Epstein Barr virus infection. In one embodiment, ME/CFS is a postviral fatigue. In one embodiment, ME/CFS is a postbacterial fatigue.

In one embodiment, sGC treatment improves symptoms of Post COVID Syndrome, wherein acute Post COVID Syndrome preferably occurs after the acute phase of SARS-CoV-3 infection. In one embodiment, Post COVID Syndrome is a postviral fatigue.

In one embodiment, treatment comprises administering vericiguat to a subject who has had or is suspected of having had SARS-CoV-2 infection and said subject has chronic vascular dysfunction comprising fatigue, muscle fatigue, muscle pain, exercise intolerance, post exertional malaise, hypersensitivity, neurocognitive impairment (e.g. memory disturbance), headache, and/or decreased hand-grip strength compared to reference level for a healthy control subject or healthy patient population, wherein said chronic vascular dysfunction occurs or persist after the onset of first symptoms of SARS-CoV-2 infection, or after the acute phase of SARS-CoV-2 infection.

In some embodiments, the chronic vascular dysfunction persists from the onset of first symptoms of the infection, or from the onset of the dysfunction, for at least one month, preferably six months.

A chronic condition or course of the disease often lasts longer than one month, or longer than three months, usually affects several areas of the body, and may not respond at all or does not respond completely to a treatment. Said chronic condition, such as chronic vascular dysfunction, and symptoms thereof persist in a subject for a period of time, i.e. for at least one month, wherein said condition can persist continuously, periodically interrupt or re-occur after intervals of varying length. In one embodiment, a chronic condition of ME/CFS or Post COVID Syndrome persists from the onset of first symptoms of the infection, or from the onset of the chronic vascular dysfunction.

In one embodiment, the symptoms may be new, recurrent, intermittent, episodic, transitory, or persistent. In one embodiment, symptoms may be acute and/or chronic.

In one embodiment, the chronic vascular dysfunction persists from the onset of first symptoms of the infection for at least 1 month, 2 months, 3 months, 4 months, 5 months, 6 months, 7 months, 8 months, 9 months, 10 months, 11 months, 1 year, 2 years, 3 years or more than three years, preferably six months.

In one embodiment, the chronic vascular dysfunction persists from the onset of the dysfunction for at least 1 month, 2 months, 3 months, 4 months, 5 months, 6 months, 7 months, 8 months, 9 months, 10 months, 11 months, 1 year, 2 years, 3 years or more than three years, preferably six months.

In one embodiment, ME/CFS has a non-acute onset, wherein non-acute onset of ME/CSF preferably occurs after the acute phase of an infection, such as an Epstein Barr virus infection. In one embodiment, ME/CFS progresses episodically over years and may increase or decrease in severity.

In one embodiment, Post COVID Syndrome has a non-acute onset, wherein non-acute onset of Post COVID Syndrome preferably occurs after the acute phase of SARS-CoV-2 infection. In one embodiment, Post COVID Syndrome progresses episodically over years and may increase or decrease in severity over time.

In some embodiments, the chronic vascular dysfunction is associated with vascular constriction, endothelial dysfunction and/or hypo-perfusion.

Chronic vascular dysfunction is also an abnormality or perturbation of the vascular system that is persistent or persists over a period of time, e.g., at least 3 or 6 months, and is characterized by impaired blood vessel function. In one embodiment, chronic vascular dysfunction comprises dysfunction of large arteries (due to arterial stiffness), the microcirculation (microvascular dysfunction) and/or endothelium (endothelial dysfunction).

In one embodiment of the invention, chronic vascular dysfunction is associated with endothelial dysfunction,

vascular constriction, vascular damage, cognitive impairment, mitochondrial dysfunction, vascular stiffness, retinal endothelial dysfunction, and/or hypoperfusion.

The sGC for use in an interventional therapy as described herein is intended to encompass a slowing or reduction in disease progression, or a reduction of Post COVID Syndrome or ME/CSF, preferably due to a reduced likelihood of worsening endothelial dysfunction, vascular constriction, vascular damage, cognitive impairment, mitochondrial dysfunction, retinal endothelial dysfunction, and/or hypo-perfusion, via activation of sGC in endothelial cells and endothelial function within the vascular system. Surprisingly, in some embodiments, the treatment described herein improves endothelial dysfunction or even resolves endothelial dysfunction.

In one embodiment, the treatment described herein relates to ameliorating chronic vascular dysfunction, endothelial dysfunction, vascular constriction, vascular damage, cognitive impairment, mitochondrial dysfunction, vascular stiffness, retinal endothelial dysfunction, and/or hypo-perfusion, inflammation, thus reducing the risk of obtaining or of progression of ME/CSF or Post COVID Syndrome.

In some embodiments, the chronic vascular dysfunction comprises muscle weakness, muscle fatigue, muscle pain, moderate fatigue, severe fatigue, exercise intolerance, neurocognitive impairment (e.g. memory disturbance), post exertional malaise, hypersensitivity, and/or pain (e.g. headache), preferably decreased handgrip strength.

ME/CFS and Post COVID Syndrome are multisystem syndromes affecting multiple organs with hallmarks of fatigue, pain, cognitive symptoms and worsening of symptoms after minimal physical / mental exertion (PEM). Symptoms vary in severity and occurrence in both syndromes. The cardinal feature of ME/CFS and Post COVID Syndrome is fatigue. A typical feature of ME/CFS is the worsening of the condition, known as postexertional fatigue or malaise (PEM), which often only occurs on the day after exertion and can last for days. In one embodiment, the invention relates to treating postexertional fatigue or malaise (PEM), wherein PEM particularly occurs > 14h after exertion in ME/CFS patients. In one embodiment, PEM particularly occurs 2-14h after exertion in Post COVID Syndrome patients.

In one embodiment, symptoms of Post COVID Syndrome comprise symptoms of ME/CFS. In one embodiment, Post COVID Syndrome can present as a full-blown post-infectious chronic fatigue syndrome ME/CFS. In some embodiments, patient with symptoms of Post COVID Syndrome do not fulfill the CCC criteria for ME/CFS.

In one embodiment, symptoms of ME/CSF comprise rapid exhaustibility due to physical, intellectual, or psychosocial exertion compared to before the onset of the disease, prolonged debilitation after exertion, impaired orthostasis response, pain (e.g., headache, pain in muscles, tendons, joints, abdomen, or chest), neurocognitive impairment after exertion, neurocognitive impairment independent of exertion, wherein neurocognitive impairment includes e.g. for example, difficulties with information processing, slowed thinking, impaired concentration, clouding of consciousness, confusion or disorientation, cognitive overload, difficulty making decisions, slowed speech, dyslexia, word-finding difficulties, difficulties with short-term memory, sleep disturbances (e. g. disturbed sleep patterns, non-restorative sleep), neurosensory perception or movement disorders, urogenital complaints, susceptibility to viral infections, prolonged recovery periods from infections, flu-like symptoms, chronic or recurrent, activated or exacerbated by stress, intolerance to foods, medications, odors, or chemicals, cardiovascular intolerance, respiratory impairment, loss of thermostability (e. g. sensation of cold, flushing), intolerance to temperature extremes, intolerance to loudness, and/ or hypersensitivity.

In one embodiment, symptoms of Post COVID Syndrome comprise fatigue, shortness of breath, and both impaired physical, mental performance, sleep disturbance, depression, anxiety, stress intolerance, cognitive disturbance, orthostatic inolerance, dyspnea (resting/exertional) cough, olfactory disturbances, gustatory disturbances, muscle weakness, psychological complaints, anxiety, impaired performance, PEM, impaired activity, altered breathing pattern, obsessive-compulsive behavior, hair loss, stress, paralysis, sensory disturbances, dizziness, nausea, diarrhea, loss of appetite, tinnitus, ear pain, loss of voice, palpitations, tachycardia, and pain, particularly muscle pain and headache.

In one embodiment, medical scores above a reference level in Post COVID Syndrome comprise medical scores above a reference level in ME/CFS. In one embodiment, a Post COVID Syndrome patient can be a human subject suffering from symptoms of ME/CFS. In one embodiment, symptoms of Post COVID Syndrome particularly comprise fatigue, dyspnea (resting/exertional), impaired performance, impaired activity, PEM, headache, and/or olfactory and gustatory disturbances. Olfactory and gustatory disturbances are common phenomena in COVID-19.

In some embodiments, sGC stimulators or a pharmaceutically acceptable salt thereof improve one or more symptoms of the Post COVID Syndrome and/or ME/CSF, such as fatigue, neurocognitive impairment (e.g. memory disturbance), moderate fatigue, severe fatigue, exercise intolerance, pain (e.g., muscle pain, headache), muscle fatigue, muscle weakness, PEM, hypersensitivity, and/or hand grip strength.

In one embodiment, the severity of symptoms in the patient to be treated represent a significant reduction in the subject's activity level, as measured by the subject's previous activity level. In embodiments, the activity level of a ME/CSF or Post COVID Syndrome patient may be mildly, moderately, severely or very severely affected.

In one embodiment, the effectiveness and physiological response of the treatment, as described herein, is determined by measuring the activity level of a ME/CSF or Post COVID Syndrome patient. In some embodiments, sGC stimulators or a pharmaceutically acceptable salt thereof administered ME/CSF or Post COVID Syndrome patient improve an activity level of said patient, for example, said patient's activity level changes from severely affected to mildly affected

or from mildly affected to non-affected. In one embodiment, the effectiveness and physiological response of the treatment, as described herein, is determined by symptoms of ME/CSF or Post COVID Syndrome.

It could not have been drawn from the prior art that endothelial dysfunction is a causative factor in ME/CSF or Post COVID Syndrome, as shown in Example 2 and 3. Surprisingly, stimulation of sGC, particularly using sGC stimulator vericiguat, improved both symptoms and endothelial dysfunction.

In some embodiments, the chronic vascular dysfunction is associated with a depression, a medical score above a reference level, for example for a reference level for a healthy control subject or healthy patient population, wherein the medical score is selected from the group consisting of SF-36 health score, Chalder Fatigue Score, CCC Symptom Score, hand grip strength test, Bell disabling scale, MBSQ score, PEM score, COMPASS-31, cognitive function (SDMT), Blood pressure (BP), Heart Rate (HR) regulation in passive standing test [CDE NIH], HRV and in stress index (SI), pain scale, and reactive hyperemia index (RHI).

In one embodiment, one or more medical scores can be used to diagnose ME/CSF and/or Post COVID Syndrome. In one embodiment, diagnostics for ME/CFS and Post COVID Syndrome comprise determination of one or more of medical scores comprising questionnaires, wherein said questionnaires comprise screening questions on fatigue, persistent physical exhaustion, exercise intolerance/PEM (one of the most common symptoms in most post/long-COVID studies), pain, cognitive dysfunction, depressive mood, and anxiety disorder (seeFatigue and Psychosomatics section). In one embodiment, a measurable physiological response to treatment described herein, may include symptom assessment by questionnaires. In one embodiment, medical scores above reference level in Post COVID Syndrome comprise medical scores above a reference level in ME/CFS.

In one embodiment, the effectiveness and physiological response of the treatment, as described herein, is determined by one or more clinical parameters, laboratory parameters, and/or medical scores, preferably by comparing measured values to reference levels from a healthy control subject or healthy patient population.

An important treatment goal is to avoid the need for assistance and anti-depressive drugs. ME/CSF or Post COVID Syndrome patients treated with a sGC stimulator, such as vericiguat, may additionally benefit from independency in their daily lives (e.g., no assistance for walking) and discontinuation of supplementary drugs, such as an antidepressant drugs.

In some embodiments, the soluble guanylate cyclase stimulator is selected from the group consisting of vericiguat, riociguat, neliciguat, BAY-41-2272, BAY 41-8543, and etriciguat, preferably vericiguat. sGC stimulators, e.g. vericiguat, riociguat, have a high safety profile. Side effects rarely, if ever, occur, such as dizziness, nausea, and gastrointestinal (GI) disturbances.

Soluble guanylate cyclase (sGC) is a receptor for nitric oxide (NO) and primarily involved in vasodilation and inflammation. Two drugs targeting sGC are currently approved: riociguat and vericiguat. Vericiguat (Verquvo, 2021) is approved for medical use to reduce the risk of cardiovascular death and heart failure (HF) and for outpatient intravenous diuretic therapy in adults with symptomatic chronic HF and ejection fraction. In general, the safety profiles of vericiguat and riociguat correspond to each other and reflect its mechanism of action as a vasodilator.

In another aspect, the invention provides a pharmaceutical composition comprising a sGC stimulator or a pharmaceutically acceptable salt thereof, for use in the treatment of a chronic vascular dysfunction in a human subject. In another aspect, the invention provides pharmaceutical compositions comprising a sGC stimulator, or a pharmaceutically acceptable salt thereof, in combination with one or more additional therapeutic agents, particularly guideline drugs, for use in the treatment of a chronic vascular dysfunction in a human subject.

In some embodiments, one or more sGC stimulators are selected from a group of, but not limited to, vericiguat, riociguat, nelociguat, BAY-41-2272, BAY 41-8543, ataciguat (HMR-1766), cinaciguat (BAY 58-2667), A-350619, and etriciguat, HMR-1069, S 3448, CFM-1571, olinciguat (IW-1701), A-344905, A-778935, preferably vericiguat.

In one embodiment, the sGC stimulator is vericiguat. In one embodiment, the sGC stimulator is riociguat. In one embodiment, the sGC stimulator is nelociguat. In one embodiment, the sGC stimulator is ataciguat. In one embodiment, the sGC stimulator is cinaciguat. In one embodiment, the sGC stimulator is etriciguat. In one embodiment, the sGC stimulator is olinciguat.

In some embodiments, the treatment comprises administering the soluble guanylate cyclase stimulator, preferably vericiguat, or salt thereof, at 0.1-500 mg/day to a human subject, preferably 0.1-50 mg/day or at 1-10 mg/day.

In some embodiments, at least one sGC stimulator described herein or a pharmaceutically acceptable salt thereof can be selected for use in the treatment of chronic vascular dysfunction in a human subject, wherein the at least one sGC stimulator described herein or a pharmaceutically acceptable salt thereof is administered at a daily dose of 0.1 to 500 mg, preferably 0.1 - 50 mg or at 1 to 10 mg.

In one embodiment, riociguat is selected for use in the treatment of chronic vascular dysfunction in a human subject, wherein riociguat is administered orally at a daily dose of 0.1 to 500 mg, preferably 0.1 - 50mg or at 1 to 10 mg.

In some embodiments, the treatment comprises administration of vericiguat or a salt thereof once daily for at least one week, up to at least twelve weeks, preferably once daily for 10 weeks, wherein the route of administration is preferably oral, preferably 1 to 10 mg per dose via oral delivery.

In some embodiments, one or more sGC stimulators selected from the group described herein or a pharmaceutically acceptable salt are administered orally. In one embodiment, vericiguat is selected for use in the treatment of chronic vascular dysfunction in a human subject, wherein vericiguat is administered orally at a daily dose of 0.1 to 500 mg, preferably 0.1 - 50 mg or at 1 to 10 mg. In one embodiment, vericiguat is administered to ME/CSF and/or Post COVID Syndrome patients in the treatment of chronic vascular dysfunction at 2.5 mg for 2 weeks, 5 mg for 2 weeks, and 10 mg for 6 weeks.

The dosing rationale is supported by pharmacodynamic data obtained in a clinical phase I-III study as described below and in Example 1. In preferred embodiments, the compounds are administered in concentrations or amounts, or according to dosage regimes, already established in the art, such as those for which regulatory approval has been issued (e.g. by the FDA or EMA), or in doses currently being assessed during phase 2 clinical trials, and/or according to the maximum allowed dose according to a phase I trial.

The above embodiments, regarding particular relative amounts, are based on clinically allowed or currently trialed doses of the various compounds described herein, being combined into a pharmaceutical composition as described herein.

In some embodiments, sGC activators or a pharmaceutically acceptable salt thereof improve one or more symptoms of the ME/CSF and/or Post COVID Syndrome, as described herein. For example, an endothelial dysfunction and/or inflammation may be effectively treated by the administration orally at a daily dose about 0.1 to 500 mg, preferably 0.1 - 50 mg or at 1 to 10 mg per patient per day.

In some embodiments, the subject had or is suspected to have had an infection, preferably a viral infection selected from the group consisting of a SARS corona infection, SARS-CoV-2 infection, influenza virus infection, epstein barr virus infection, HSV-1 infection, HHV6 infection, dengue fever virus, and entero virus infection, preferably a SARS-CoV-2 infection.

In one embodiment, the subject suffering from ME/CFS had or is suspected to have had an infection with a human pathogen, such as by a bacteria, virus or fungi. In one embodiment, the subject suffering from ME/CFS had or is suspected to have had a viral infection, such as an influenza virus infection, epstein barr virus infection, HSV-1 infection, HHV6 infection, dengue fever virus or entero virus infection. In one embodiment, the subject suffering from ME/CFS had or is suspected to have had a bacterial infection, such as legionella, chlamydia, rickettsiae, bartonella or coxial bacteria.

In one embodiment, the chronic vascular dysfunction occurs independently of an infection. In one embodiment, the ME/CFS occurs independently of an infection. In one embodiment, the infectious agent is undetectable at the time of treatment.

In some embodiments, the treatment comprises administering vericiguat to a subject who has had or is suspected of having had SARS-CoV-2 infection and said subject has chronic vascular dysfunction comprising fatigue, muscle fatigue, muscle pain, exercise intolerance, post exertional malaise, hypersensitivity, neurocognitive impairment (e.g. memory disturbance), headache, and/or decreased hand-grip strength compared to reference level for a healthy control subject or healthy patient population, wherein said chronic vascular dysfunction occurs or persist after the onset of first symptoms of SARS-CoV-2 infection, or after the acute phase of SARS-CoV-2 infection.

In some embodiments, symptoms of a SARS-CoV-2 infection comprise one or more of fever, sore throat, cough, lack of taste and/or smell, shortness of breath and/or fatigue.

In one embodiment, the subject suffering from Post COVID Syndrome or ME/CSF had or is suspected to have had a SARS corona infection, e.g. SARS-CoV-2. In one embodiment, patients with a history of SARS-CoV-2 infection and a chronic condition comprising fatigue may not fulfil ME/CFS criteria, described herein. In one embodiment, patients not fulfilling the ME/CFS criteria and with a history of a SARS-CoV-2 are also referred to as Post COVID Syndrome patients. In one embodiment, symptoms of Post COVID Syndrome particularly comprise fatigue, dyspnea (resting/exertional), impaired performance, impaired activity, PEM, headache, and/or olfactory and gustatory disturbances. Olfactory and gustatory disturbances are common phenomena in COVID-19.

In some embodiments, the onset of the viral infection is characterized by the presence of symptoms of the viral infection, and/or by detection of the viral infection in said subject comprising detection of viral components, such as by a PCR-test or an antigen-test, and wherein said viral infection may be undetectable while the chronic vascular dysfunction persists.

Persistence of the SARS-CoV-2 virus or viral components over weeks and months may play a role. Other possible pathomechanisms are persistent postinfectious hypercoagulability, thrombosis, endothelial dysfunction, chronic immune dysregulation, (hyper)inflammation and autoimmunity. In one embodiment, methods for detecting an infectious agent or components thereof, such as SARS-CoV-2, comprise PCR, quantitative PCR, ELISA, FACS, whole genome sequencing, whole transcriptome sequencing, microscopic examination of specimens with special stains, antigen detection, western blots, or microbiological differential smear. In one embodiment, an infectious agent can be detected in a sample obtained from a ME/CFS patient.

In some embodiments, the subject has an elevated level of one or more of biomarkers selected from ET-1, IL-8, CrP, hemoglobin, and/or NT-proBNP, and/or a decreased level of one or more of biomarkers selected from ferritin, ANG-2, ACE2, ACE-1, and/or bilirubin, wherein said level is compared to a level in a healthy control subject or healthy patient population and is determined in a sample comprising and/or derived from a bodily fluid and/or cells from the subject, preferably from blood, serum or plasma.

Samples are collected and analysed as described in the Examples 1-3. In one embodiment, samples from patients, preferably blood and serum, for differential blood work, ET-1, IL-8, CrP, hemoglobin, NT-proBNP, ferritin, ANG-2, ACE2, ACE-1, and/or bilirubin are obtained on day 1, after 2 weeks, after 4 weeks, after 6 weeks, after 8 weeks, after 10 weeks of treatment and 30 days after last treatment dose has been administered. In one embodiment, nasopharyngeal swabs or blood samples are collected on day for detecting virus or components thereof.

As shown in Example 2, an association of HGS with hemoglobin (Hb), IL-8, and CRP, NT-pro BNP, bilirubin, and ferritin indicates low inflammation and hypoperfusion as possible pathomechanisms. The inventors surprisingly found, as shown below in Example 3, an association between diminished RHI and hand grip strength and endothelial dysfunction. The inventors further strikingly found correlating biomarker levels, including ET-1, IL-8, CrP, NT-proBNP, which are endothelial biomarkers.

In embodiments, the present invention is related to treating a chronic vascular dysfunction in ME/CFS and Post COVID Syndrome patients, particularly an endothelial dysfunction and inflammation.

The composition provided by the present disclosure may be capable of delaying or stopping the progression and/or persistence of a medical condition comprising chronic vascular dysfunction, particularly endothelial dysfunction. The use of samples received from ME/CFS and Post COVID Syndrome patients and controls in two cohorts is an advantage of the present disclosure and a strength of the clinical study. The present disclosure is the first to show an association with RHI, hand grip strength expression, endothelial biomarkers and chronic vascular dysfunction in ME/CFS and Post COVID Syndrome patients and providing means to treat said disease.

In some embodiments, the treatment is administered at a dose and frequency configured to prevent and/or to reduce a level of ET-1, reduce a level of blood pressure, reduce a level of vascular constriction, reduce a level of endothelial dysfunction, reduce a level retinal endothelial dysfunction, reduce a level of cognitive impairment, reduce a level of hypo-perfusion, and/or to reduce a score of one or more medical scores to a level of at least 20% below a level at treatment initiation.

In one embodiment, said treatment is administered at a dosage and frequency configured to reduce a level of ET-1, a level of blood pressure, a level of vascular constriction, a level of endothelial dysfunction, a level retinal endothelial dysfunction, a level of cognitive impairment, a level of hypo-perfusion, and/or to a score of one or more medical scores to a level of at least 5%, 10%, 20%, 30%, 40% or at least 50% below a level at treatment initiation.

In one embodiment, said treatment is administered at a dosage and frequency configured to prevent and/or to reduce a level of anti-inflammatory cytokines to a level of at least 20% below a level at treatment initiation, wherein said anti-inflammatory cytokines include preferably IL-8.

A skilled person is capable, based on the experimental and written support provided herein, to carry out the invention, in combination with common knowledge in the art. By employing the quantitative measures described in the examples, or by using alternative quantitative means for determining reductions in levels of one or more anti-inflammatory cytokines and/or endothelial marker and/or endothelial function parameters or cognitive function parameters, the combination of pharmaceutical compounds and their necessary doses can be determined and adjusted to obtain the beneficial effects as described herein.

In some embodiments, the treatment comprises administering vericiguat orally at 2.5 to 10 mg/day to a subject diagnosed with ME/CFS or Post COVID Syndrome, wherein said subject has chronic vascular dysfunction occurring or persisting after the onset of first symptoms of SARS-CoV-2 infection, or after the acute phase of SARS-CoV-2 infection.

In some embodiments, the subject is or has been treated additionally with a guideline treatment for Post-COVID/Long-COVID and/or for ME/CSF.

Guidelines recommend therapeutic options in patients with chronic fatigue, e.g. ME/CFS and Post COVID Syndrome. Guidelines for the treatment of Post COVID Syndrome or ME/CFS patients comprise S3 guideline "Fatigue" DEGAM Guideline No. 2 (AWMF register no. 053-002) or S1 Guideline Post-COVID/Long-COVID (AWMF Register No. 020/027). In one embodiment, a drug recommended by a guideline is a non-sGC stimulator guideline drug. The guidelines are established therapeutic approaches and are clear and enabled for a skilled person by reference to common practice in the field and the guidelines cited above.

In one embodiment, the non-sGC stimulator guideline drug is solely administered to the subject described herein. In one embodiment, the non-sGC stimulator guideline drug is administered in combination with a sGC stimulator, preferably vericiguat, to the subject described herein.

Further examples of pharmaceutical compositions, pharmaceutical combinations, treatments, subjects, biomarkers and targets are described in detail below.

The features of the invention regarding methods of treatment and diagnosis, and descriptions of the composition comprising sGC stimulator for use in the treatment of various medical conditions, apply to the composition itself, and

vice versa. Any of the features disclosed herein for any given embodiment are applicable to other embodiments, mas deemed reasonable by a skilled person.

## DETAILED DESCRIPTION OF THE INVENTION

Singular expressions should be understood to include the concept in the plural form, unless explicitly stated otherwise. Thus, singular articles (e.g., "a," "an," "the," and the like in English) should also be understood to include the concept in the plural form, unless specifically noted otherwise. Further, terms used herein shall be understood to have the meaning generally accepted in the art, unless otherwise expressly noted.

Soluble gyanylate cyclase, soluble guanylate cyclase activator, soluble guanylate cyclase stimulator

Soluble guanylate cyclase (sGC) is a receptor for nitric oxide (NO) and also the only one known at present. Nitric oxide (NO) signaling plays an essential role in many physiological processes. NO molecules are produced by NO synthase in an NO donor cell. NO then readily penetrates the membranes of a target cell, such as an endothelial cell, and binds to soluble guanylate cyclase (sGC). This increases the activity of sGC by a hundredfold to produce intracellular cGMP. Deregulation of NO signaling can lead to a spectrum of diseases.

sGC is soluble, present intracellularly, and primarily involved in vasodilation. The term "vasodilation" refers to "expansion" or "widening" of blood vessels, i.e. the enlargement of their lumen. Muscle cells are located in the walls of the vessels. If these muscle cells slacken, the blood vessel dilates, i.e. vasodilation. Dilatation and constriction of blood vessels serve to regulate blood flow to organs and tissues in the body. As a heterodimer composed of one $\alpha$ and one $\beta$ subunit, sGC is encoded in humans by the genes GUCY1A2, GUCY1A3, GUCY1B2 and GUCY1B3.

Recent high-resolution structures of human sGC in various functional states have shown that sGC is an allosteric enzyme consisting of three structural modules: the sensing module, the transducer module, and the catalytic module. Binding of saturated NO to sGC results in a conformational change of the sensor module. This signal is passed to the adjacent transducer module and allosterically transferred to the catalytic module to open the GTP binding cleft and increase its activity. The conformational changes within each module during sGC activation are related to the extensive structural reconfiguration of the entire enzyme: inactive sGC has a bent shape, whereas active sGC is elongated. Similar structural changes were also observed in the medium-resolution structures of sGC from the insect Manduca sexta, further highlighting the evolutionarily conserved structural mechanism of sGC activation by NO. The H-NOX domain of the $\beta$-subunit in the sensing module harbors a prosthetic iron-containing heme (Fe2+) unit essential for NO sensing. Structural and biochemical in vitro studies have shown that oxidation of ferrous heme to ferric heme (Fe3+) can trap sGC in an inactive state. In addition, the ferrous heme can readily dissociate from sGC; this results in heme-free sGC, which has low activity and responds poorly to NO stimulation. The processes of heme oxidation and dissociation have also been observed in vivo and may lead to attenuation of NO signaling under various pathological conditions, such as inflammation or oxidative stress.

NO increases the sGC activity about 200-fold. NO has a strong trans effect, wherein the histidineiron bond is weakened when the NO bond shifts electrons into the dz2 orbital towards the axial ligand. When nitric oxide binds iron-haem at the distal position, a His-Fe-NO complex is formed, which dissociates into a 5-coordinate Fe-NO complex. However, the identification of two distinct [NO]-dependent processes in sGC activation has led to the speculation that a proximal NO is responsible for the histidine shift, resulting in an intermediate 6-coordinated NO-Fe-NO complex. Depending on whether the reaction equilibrium is on the side of the product, the intermediate can then dissociate into either of two 5-coordinate forms, the more active distal NO-ligated form or the less active proximal NO-ligated form. An alternative hypothesis is that a second site, not bound to the haem, is responsible for the second NO-dependent activation process to obtain the fully active enzyme.

As such, due to the extensive information already obtained on the sGC, any functional effect on the sGC, such as stimulation of the sGC, can be easily determined without undue effort using methods established in the art.

Two drugs targeting sGC are currently approved: riociguat and vericiguat. Riociguat (trade name Adempas) is a sGC stimulator for the treatment of two forms of pulmonary hypertension (PH): chronic thromboembolic pulmonary hypertension (CTEPH) and pulmonary arterial hypertension (PAH). Vericiguat (Verquvo, 2021) is approved for medical use to reduce the risk of cardiovascular death and heart failure (HF) in adults with symptomatic chronic HF and reduced ejection fraction who required intravenous therapy due to decompensation.

Nelociguat (BAY 60-4552) has undergone phase II clinical trials by Bayer for the treatment of erectile dysfunction and heart failure. Etriciguat is a potent, selective, orally available sGC stimulator (described in WO 2003086407). Cinaciguat (BAY 58-2667) is a soluble guanylate cyclase activator in clinical development for the treatment of acute decompensated heart failure (ADHF). In patients with ADHF, intravenously administered cinaciguat results in potent unloading of the heart with arterial vasodilation.

Ataciguat (HMR1766) has undergone phase II clinical trials by Sanofi to determine whether it slows progression

of valve calcification in patients with moderate calcific aortic valve stenosis. Secondary and tertiary objectives are to determine whether Ataciguat slows progression of aortic valve function, reduces systemic inflammation, and prevents left ventricular dysfunction in patients with moderate calcific aortic valve stenosis.

Olinciguat (IW-1702) is currently being evaluated in a Phase II study by Ironwood Pharmaceuticals for achalasia.

In some embodiments, the sGC stimulator is selected from a group of, but not limited to, vericiguat, riociguat, nelociguat, BAY-41-2272, BAY 41-8543, ataciguat (HMR-1766), cinaciguat (BAY 58-2667), A-350619, etriciguat, HMR-1069, S 3448, CFM-1571, olinciguat (IW-1701), A-344905, A-778935. The preferred sGC stimulator is vericiguat.

In embodiments of the invention, the sGC stimulator or the sGC activator is one of the compounds depicted below in Table 1.

Table 1: sGC stimulators and sGC activators

| | | |
|---|---|---|
| 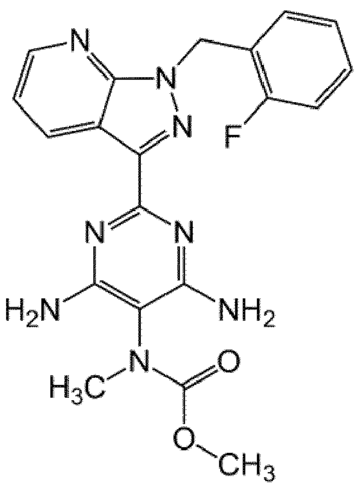 | 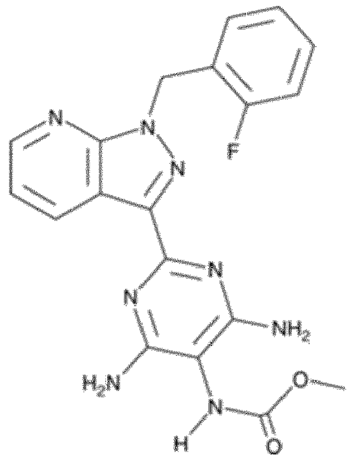 | 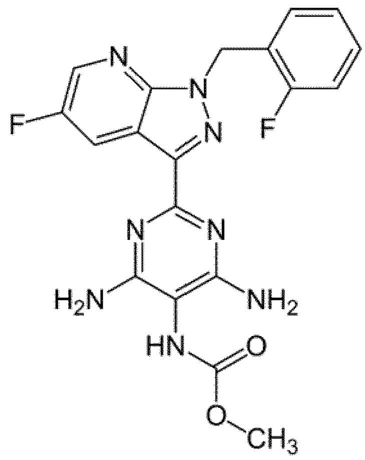 |
| Riociguat (BAY 63-2521, Adempas®, FDA approved drug, DE19834044) | Nelociguat (BAY 60-4552, described in WO 2003095451) | Vericiguat (BAY 1021189, FDA approved drug,) |
| 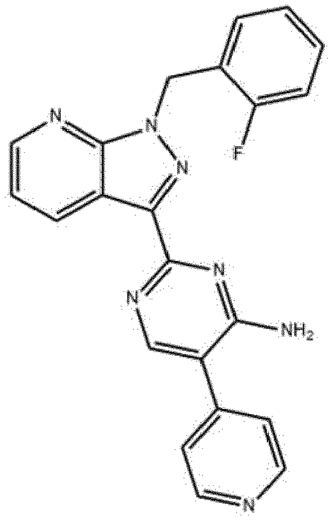 | 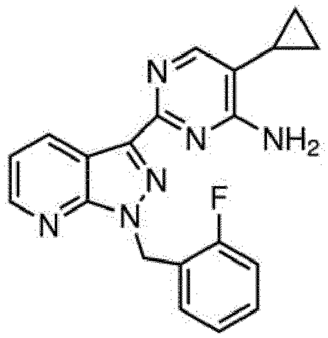 | 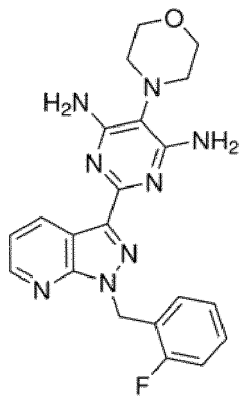 |
| Etriciguat (described in WO 2003086407) | BAY 41-2272 (DE19834047 and DE19942809) | BAY 41-8543 |

(continued)

| | | |
|---|---|---|
| 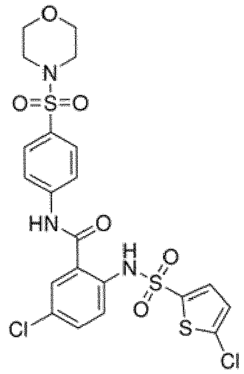 | 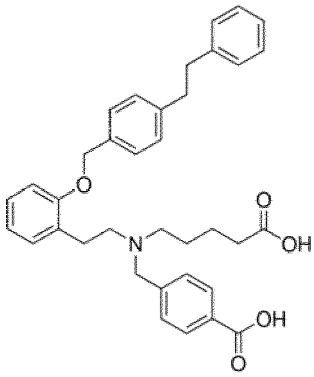 | 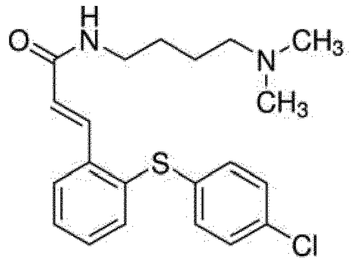 |
| Ataciguat (HMR-1766) | Cinaciguat, BAY 58-2667; | A-350619 |
| 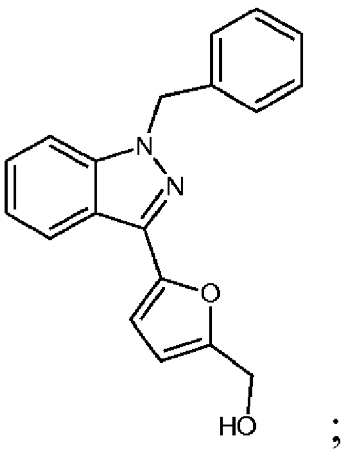 | 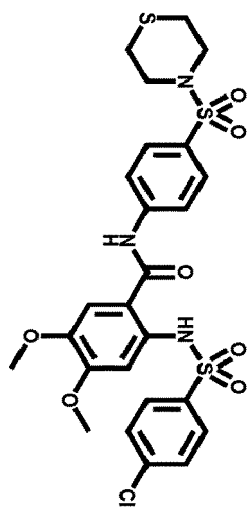 | 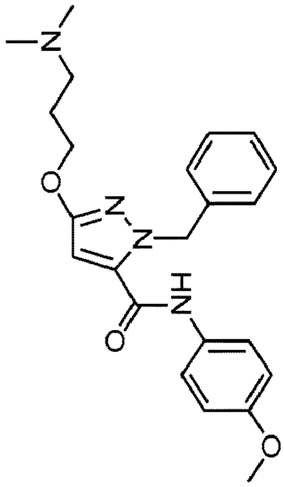 |
| HMR-1069 (Sanofi-Aventis). | S 3448 | CFM-1571 |
| 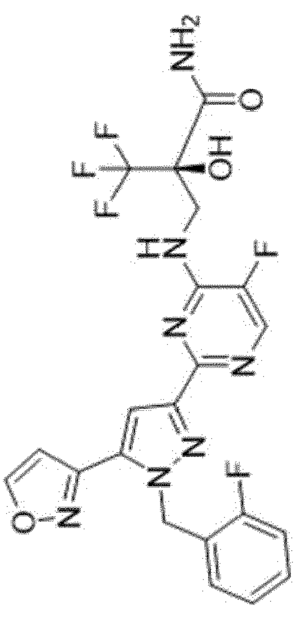 | 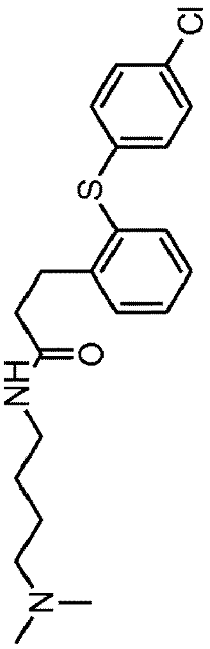 | 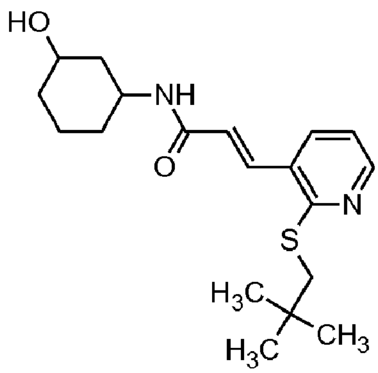 |
| Olinciguat (IW-1701) | A-344905 | A-778935 |

In some embodiments, at least one sGC stimulator described herein or a pharmaceutically acceptable salt thereof can be selected for use in the treatment of chronic vascular dysfunction in a human subject, wherein the sGC stimulator can be administered in doses as described herein.

In further embodiments, riociguat is selected for use in the treatment of chronic vascular dysfunction in a human subject, wherein riociguat is administered orally at a daily dose of 0.1 to 500 mg, preferably 0.1 to 50 mg or at 1 to 10 mg. In one embodiment, nelociguat is selected for use in the treatment of chronic vascular dysfunction in a human subject, wherein nelociguat is administered orally at a daily dose of 0.1 to 500 mg, preferably 0.1 - 50mg or at 1 to 10 mg. In one embodiment, etriciguat is selected for use in the treatment of chronic vascular dysfunction in a human subject, wherein etriciguat is administered orally at a daily dose of 0.1 to 500 mg, preferably 0.1 to 50mg or at 1 to 10 mg. In one embodiment, ataciguat is selected for use in the treatment of chronic vascular dysfunction in a human subject, wherein ataciguat is administered orally at a daily dose of 0.1 to 500 mg, preferably 0.1 to 50mg or at 1 to 10 mg. In one embodiment, cinaciguat is selected for use in the treatment of chronic vascular dysfunction in a human subject, wherein cinaciguat is administered orally at a daily dose of 0.1 to 500 mg, preferably 0.1 to 50mg or at 1 to 10 mg. In one embodiment, olinciguat is selected for use in the treatment of chronic vascular dysfunction in a human subject, wherein olinciguat is administered orally at a daily dose of 0.1 to 500 mg, preferably 0.1 to 50mg or at 1 to 10 mg.

Vericiguat

Vericiguat (Verquvo, 2021) is approved for medical use to reduce the risk of cardiovascular death and heart failure (HF) in adults with symptomatic chronic HF and reduced ejection fraction who required intravenous therapy due to decompensation. It has been extensively studied in preclinical and clinical studies in patients with symptomatic chronic HF and reduced ejection fraction as described herein. Nor vericiguat nor any other sGC stimulator or activator known in the prior art has been studied in context with ME/CSF and Post COVID Syndrome. Riociguat, vericiguat, nelociguat and are found to present similar pharmacology and safety features. In one embodiment, any sGC stimulator described herein, e.g. vericiguat or riociguat, can be used for the treatment of ME/CSF and Post COVID Syndrome in human subjects.

Vericiguat is typically well-tolerated in clinical studies, and most treatment-emergent adverse events were of mild-to-moderate severity and as expected from the mechanism of action of vericiguat. In clinical phase I studies, the PK interaction profile was estimated to be low in in vitro studies and confirmed in studies in healthy subjects. The results suggested that vericiguat is suitable for the treatment of a patient population with multiple comorbidities requiring polypharmacy, such as patients with HF. (Boettcher, M., Thomas, D., Mueck, W. et al. Safety, pharmacodynamic, and pharmacokinetic characterization of vericiguat: results from six phase I studies in healthy subjects (Eur J Clin Pharmacol 77, 527-537 (2021) https://doi.org/10.1007/s00228-020-03023-7).

In clinical phase II and III trials, the efficacy of vericiguat at an initial dose of 2.5 mg, titrated to 10 mg at two-week intervals to reach the 10-mg dose, has been shown to be superior to placebo in terms of cardiovascular mortality and HF hospitalization (EudraCT: 2016-000671-25). The initial dose of vericiguat of 2.5 mg was doubled every two weeks to 5 mg and finally to the target dose of 10 mg. Subjects with heart failure with reduced ejection fraction (HFrEF) were treated (Eur J Clin Pharmacol 77, 527-537 (2021). https://doi.org/10.1007/s00228-020-03023-7).

Dosing rationale has been developed in other therapeutic contexts. The randomized, placebocontrolled, double-blind, multicenter, 12-week parallel-group dose-finding study evaluated four vericiguat dosing regimens (1.25 mg, 2.5 mg, 2.5 up-titrated to 5 mg, and 2.5 up-titrated to 10 mg) compared with placebo in patients with worsening HFrEF. At 12 weeks, the primary efficacy endpoint, the change from baseline in log-transformed NT-proBNP, was not significantly different between the pooled vericiguat group and placebo. However, the secondary analysis showed a dose-response relationship in the reduction of NT-proBNP at 12 weeks in the vericiguat group. The initial dose of 2.5 mg vericiguat, titrated at two-week intervals to a target dose of 10 mg, resulted in the greatest reduction in NT-proBNP at 12 weeks compared to placebo (p=0.0483) (Table 2).

Table 2. Comparison of the Primary Efficacy Variable Change in Log transformed NT-proBNP From Baseline to Week 12 (Visit 5)

| Treatment Comparison | Difference of Means vs Placebo - log scale | 90% CI of Difference | Ratio of Geometric Means vs Placebo -Back Transformed | 90% CI of Ratio | t-test p-value (one-sided) |
|---|---|---|---|---|---|
| Vericiguat Pool | -0.1220 | -0.32 to 0.07 | 0.885 | 0.73 to 1.08 | 0.1506 |
| Vericiguat 1.25 mg | 0.0151 | -0.21 to 0.24 | 1.015 | 0.81 to 1.27 | 0.5444 |

(continued)

| Treatment Comparison | Difference of Means vs Placebo - log scale | 90% CI of Difference | Ratio of Geometric Means vs Placebo -Back Transformed | 90% CI of Ratio | t-test p-value (one-sided) |
|---|---|---|---|---|---|
| Vericiguat 2.5 mg | -0.0396 | -0.26 to 0.18 | 0.961 | 0.77 to 1.20 | 0.3841 |
| Vericiguat 2.5 to 5 mg | -0.0731 | -0.31 to 0.16 | 0.930 | 0.73 to 1.18 | 0.3402 |
| Vericiguat 2.5 to 10 mg | -0.2494 | -0.50 to -0.00 | 0.779 | 0.61 to 1.00 | 0.0483 |
| The 3 highest vericiguat dose groups (2.5 mg, 2.5 to 5 mg, 2.5 to 10 mg) were included in the pool.<br>Mean and SD are on the log scale (log [pg/mL]).<br>Geometric mean and SD are on the original scale (pg/mL).<br>Since the primary analysis was not significant, all p-values are only descriptive.<br>CI=confidence interval; NT-proBNP=N-terminal pro-brain natriuretic peptide | | | | | |

Immune cell and Inflammation

An "immune cell" includes any cell of the vertebrate immune system, including lymphocytes such as B-cells, killer T-cells (i.e., CD8+ T-cells), helper T-cells (i.e., CD4+ T-cells, including Th1 and Th2 cells), natural killer cells, and γδ T-cells, monocytes, macrophages, neutrophils, dendritic cells, mast cells, eosinophils, and basophils.

The term "Inflammation" also refers to the biological response of a tissue to noxious stimuli such as pathogens, damaged cells (e.g., wounds), and/or irritants. The term "inflammatory response" refers to a specific mechanism that causes and regulates inflammation and, in addition to other mechanisms known in the art, activation migration of immune cells, or cytokine production, vasodilatation. Ideally, inflammation is a protective function of the intended body that both eliminates harmful stimuli and initiates the process of healing one or more affected tissues. However, hyperinflammation or chronic inflammation, described herein, is associated with various diseases such as viral infections, e.g. SARS-CoV-2 infection, hay fever, atherosclerosis, rheumatoid arthritis and other diseases known in the medical community

Activator, and stimulator

As used herein, "activator" and "stimulator" can be used interchangeably and refer to an agent or molecule that binds to, interacts with, or contacts a structure (target) within the body of a subject, or otherwise indirectly influences a structure (target), particularly within a cell, and that leads to an increased activity of that structure, such as a chemical reaction or an enzyme activity. In embodiments, such activators, as described herein, may bind the structure (target) independently from its states, e.g. oxidized and non-ox oxidized, and may act independently from a second molecule that is involved or needed for an activity of such structure. In embodiments, such a stimulator, as described herein, may bind the structure in a certain state, e.g. oxidized, and may act synergistically with a second molecule that is involved or needed for an activity of such structure

According to the invention, the structure (target) enzyme that is increased in its activity by an activator and/or a stimulator is sGC. Such a second molecule may be a co-molecule for enzyme activity. In one embodiment, the second molecule is NO. In one embodiment, increase of cGMP levels by sGC stimulation decreases chronic vascular dysfunction, for example by increase of endothelial function, vessel density, perfusion, blood flow, decrease vascular constriction. The activity may also be gene transcription and/or translation or chemical reaction. For example, an activator binding to an enzyme, i.e. an enzyme activator, is a molecule that binds to an enzyme and increases enzyme's activity.

As stated above, dysregulation of NO signaling and consequently of sGC activity can lead to various diseases. To enhance downstream NO signaling by increasing sGC activity in associated diseases, two types of similar small pharmaceutical molecules may be employed, namely sGC stimulators and activators. In embodiments, sGC stimulators can activate sGC with iron-containing haem without NO and also have a strong synergistic activation effect with NO. Therefore, sGC stimulators can enhance downstream cGMP signaling both in the absence and presence of NO. In embodiments, the sGC stimulator riociguat is used clinically to treat pulmonary arterial hypertension and chronic thromboembolic pulmonary hypertension, and another stimulator, vericiguat, has been developed for the treatment of symptomatic chronic heart failure with reduced ejection fraction.

In some embodiments, sGC activators can bind to and activate heme-oxidised and heme-free sGC, independent of upstream NO signaling. They are used to activate sGC when NO signaling is impaired by heme oxidation and subsequent loss of heme in sGC.

In one embodiment, sGC stimulators can activate sGC with iron-containing heme without NO and also have a strong synergistic activation effect with NO. In one embodiment, vericiguat increases intracellular cGMP levels by activating soluble guanylate cyclase, independently and synergistically with NO. In one embodiment, sGC activators bind to and activate haem-oxidised and haem-free sGC, independent of upstream NO signalling. In one embodiment, the sGC activator interacts with sGC resulting in activation of an oxidized and heme-free and thus NOinsensitive form of soluble guanylate cyclase formed under oxidative stress. In one embodiment, sGC activation and/or sGC stimulation may cause smooth muscle relaxation and vasodilation.

Laboratory and clinical parameters

Laboratory and clinical parameters include physical parameters to be measured, the measurement of which indicates a quantity or function that cannot itself be accurately determined by direct methods, e.g. blood pressure and pulse rate are parameters of cardiovascular function and glucose level in blood and urine is a parameter of carbohydrate metabolism. Reference levels of laboratory and clinical parameters are known in art and can be easily found and interpreted by a person skilled in the art, such as in a reference book for laboratory and clinical parameters, e.g. "Praktische Labordiagnostik - Lehrbuch zur Laboratoriumsmedizin, klinische Chemie und Hämatologie", author Harald Renz, published by De Gruyter Verlag.

In some embodiments, clinical parameters for monitoring physiological responses to the treatment comprise blood pressure, level of vascular constriction, level of endothelial dysfunction, level retinal endothelial dysfunction, level of cognitive impairment, level of hypo-perfusion. In one embodiment, the effectiveness and physiological response of the treatment, as described herein, is determined by at least one clinical parameter, wherein a value of said clinical parameter is compared to the value determined before treatment initiation for that laboratory parameter. In one embodiment, ME/CFS or Post COVID Syndrome is associated with one or more clinical parameters. In one embodiment, one or more clinical parameters are out of range of healthy reference levels in ME/CFS or Post COVID Syndrome patients, when compared to a healthy control subject or healthy patient population.

In some embodiments, laboratory parameters for monitoring physiological response to the treatment comprise biomarkers selected from HbA1c, 25-OH-Vitamin D3 Se, ferritin, ferritin HP, transferrin, transferrin-saturation HP, Zinc, Folic acid, fT3, fT4, TSH, thyreoid peroxidase AB, thyreoid peroxidase AB serum, antinuclear AB (ANA)/HEp-2-IF, ENA-Screen, all ENA antibodies, IgG, IgA, IgM, IgE HP, IgG 1, IgG2, IgG3, IgG4, IgG4 serum, C3, C4 Complement HP, urine analysis: stick and sediment, CBC, hematocrit, albumin, creatinine, cystatin C, Na, K, Ca, phosphate, haemoglobin, creatin kinase muscle-brain type, creatin kinase, LDH, myoglobin, Renin, NT-proBNP , hsTnT, bilirubin, bilirubin total, plasma total protein, protein electrophoresis, C-reactive protein, tumor necrosis factor alpha 4hLPS, soluble IL-2 receptor, IL-8, lipase, leukocytes, PLT, PCT, PPT (Quick)/INR, ACE , GPT/ALT, GOT/AST, gamma GT, AP, BNP, and/or troponin.

In one embodiment, ME/CFS or Post COVID Syndrome is associated with one or more laboratory parameters. In one embodiment, one or more laboratory parameters are out of range in ME/CFS or Post COVID Syndrome patients compared to a healthy control subject or healthy patient population. As used herein, the range of healthy control subject or healthy patient population values are known by a skilled person or can be determined without undue effort.

In one embodiment, the effectiveness and physiological response of the treatment, as described herein, is determined by at least one laboratory parameter, wherein a value of said laboratory parameter is compared to the value determined before treatment initiation for that laboratory parameter. In one embodiment, a laboratory parameter for determining effectiveness and physiological response of the treatment described herein, comprise one or more of the biomarkers listed above for monitoring physiological response. In one embodiment, samples from a human subject for measuring said biomarkers are preferably blood samples, serum samples, plasma samples and/or urine samples.

Biomarkers

The term "biomarker" refers to a measurable parameter of biological processes that has prognostic or diagnostic value and can be used as a measurable indicator of the presence or severity of a disease state or other physiological condition of an organism. Biomarkers can be molecules that can be detected and measured in parts of the body (such as blood or tissue). Biomarkers can also be specific molecules or genes, gene products, enzymes, or hormones. Complex organ functions or characteristic changes in biological structures can also serve as biomarkers. In clinical diagnostics and preclinical research, biomarkers are used to indicate normal or pathological processes in the body. For example, a biomarker indicates a change in the expression or state of a protein that correlates with the risk or progression of a disease, syndrome or dysfunction, or even with the response of the disease to a given treatment.

Biomarkers measured in samples, preferably blood, serum or plasma, obtained from a human subject, comprise without limitation endothelin-1, NT-proBNP, ACE2, ACE-1, bradykinin receptor1, β2 adrenergic receptor, arginase 1, ANG-2, and/or PK.

Endothelin 1 (ET-1) is released from endothelial cells during acute and chronic vascular injury and is the most potent vasoconstrictor known. Its action is due to a particular linkage with specific receptors: Endothelin ETA and ETB receptors.

Interleukin 8 (IL-8) is an important proinflammatory cytokine, but also an important α-chemokine. It is released by many inflammatory cells such as lymphocytes, monocytes, and mast cells, and is produced by endothelial cells.

N-terminal pro-brain natriuretic peptide (NT-proBNP) is a 76 amino acid molecule that is secreted in equimolar unit with brain natriuretic peptide (BNP) during hypoxia and/or stretching of ventricular muscle cells. There is strong evidence for its role as a novel marker of acute myocardial ischemia in unstable angina. It is also a marker of ischemic muscle cells.

Angiotensin-converting enzyme 1 (ACE1) is involved in the regulation of blood pressure and electrolyte balance. It catalyzes the conversion of angiotensin I to the physiologically active peptide angiotensin II. Angiotensin II is a potent vasopressor and aldosterone-stimulating peptide that controls blood pressure and fluid-electrolyte balance. ACE also inactivates the vasodilator protein bradykinin. Accordingly, the encoded enzyme increases blood pressure and is a target for ACE inhibitors, which are often prescribed to lower blood pressure.

ACE2 has been identified as a critical negative modulator of ANG2 bioactivity, counterbalancing the effects of ACE. ACE2 is highly expressed in vascular endothelial cells.

Bradykinin receptor1 expression is induced during injury by pro-inflammatory cytokines such as interleukin-1β and acts primarily on endothelial cells in the peripheral and coronary vasculature.

β2 adrenergic receptor is also expressed in vascular smooth muscle cells and endothelial cells. It acts on vasodilation via Gs/AC/increased cAMP.

Angiopoietin-2 (ANG2) is a growth factor involved in angiogenesis, inflammation and its dysregulation can cause vessel regression and endothelial cell death. ANG2 is additionally one of the main effectors of endothelial dysfunction. Arginase 1 upregulation can lead to suppression of nitric oxide (NO) formation by endothelial NOS (eNOS) resulting in superoxide production, endothelial dysfunction, platelet aggregation, and leukocyte activation and attachment to the vessel wall.

In embodiments, a biomarker for determining the effectiveness and physiological response of the treatment described herein, comprise one or more of endothelin-1, NT-proBNP, ACE2, ACE-1, bradykinin receptor1, β2 adrenergic receptor, arginase 1, ANG-2, and/or PK. In one embodiment, samples from a human subject for measuring said biomarkers are preferably blood samples, serum samples, plasma samples and/or urine samples.

Medical Indications

As used herein, the "patient" or "subject" may be a vertebrate. In the context of the present invention, the term "subject" includes both humans and animals, particularly mammals, and other organisms.

In the present invention "treatment" or "therapy" generally means to obtain a desired pharmacological effect and/or physiological effect. The effect may be prophylactic in view of completely or partially preventing a disease, syndrome, medical condition and/or a symptom, for example by reducing the risk of a subject having a disease, syndrome, medical condition or symptom or may be therapeutic in view of partially or completely curing a disease and/or adverse effect of the disease. In the present invention, "therapy" includes arbitrary treatments of diseases or conditions in mammals, in particular, humans, for example, the following treatments (a) to (b): (a) Inhibition of a symptom of a condition, that is, prevention of progression of the symptom; (b) Amelioration of a symptom of a condition, that is, induction of regression of the disease, syndrome or symptom.

A "disease course" or "course of a disease" is the quantitative (temporal) and qualitative (symptomatic) profile of a disease. The course of a disease can - depending on the aspect considered - be given different attributes that characterise its speed, severity or other properties. For example, "acute" refers to a rapid onset of the disease, but also to a rather short duration of the disease, wherein "chronic" refers primarily to the duration of the disease. Chronic diseases can also have an acute onset, e.g. ME/CSF with pain and fatigue.

The course of the disease is characterised by the symptoms and their occurrence, wherein symptoms can be characterised according to their temporal course (tempo and duration, rhythmicity), progress of the symptomatology, severity of the symptomatology and/or pathophysiology. In one embodiment, the symptoms may be new, recurrent, intermittent, episodic, transitory, persistent. In one embodiment, symptoms may be acute and/or chronic.

The terms "dysfunction" or "dysfunctionality" as used herein, which can be used interchangeably, refer to a perturbation, preferably pathological, i.e., absent or deficient function of the normal structure or function of a body part, organ, tissue, or cell assembly. Endothelial dysfunction or vascular dysfunction is also an abnormality or perturbation in which the endothelial cells of the blood vessels no longer fully perform their normal function. Chronic vascular dysfunction is also an abnormality or perturbation of the vascular system that is persistent or persists over a period of time, e.g., at least 6 months, and is characterized by impaired blood vessel function. In patients having chronic vascular dysfunction, the structure of the blood vessels may be normal, and there are no obvious lesions such as plaques. The

function can be disturbed and the patient suffers from an abnormality, perturbation of blood flow or hypoperfusion.

*ME/CSF and diagnostic criteria for ME/CSF*

Myalgic encephalomyelitis, also called chronic fatigue syndrome (ME/CFS), is a severe, multisystemic chronic disease with dysregulation of the immune system, nervous system, and cellular energy metabolism, manifested by unexplained severe fatigue, symptom aggravation after physical or mental exertion, sleep disturbances, or cognitive dysfunction lasting six months or longer and often accompanied by orthostatic intolerance, pain, digestive symptoms, hypersensitivity to light, sound, odors, or chemical substances, or the like.

In most patients, ME/CSF has an acute onset, commonly following an acute infection, such as a late Epstein-Barr virus (EBV) initial infection, i.e., Pfeiffer glandular fever, can trigger ME/CFS. But other infectious agents have also been described in connection with ME/CFS: SARS-Cov2, HSV-1 and HHV6, enteroviruses, influenza, but also intracellular bacteria such as chlamydia, legionella, borreliose, and coxiella.

An infection can only be detected in a small proportion of ME/CSF patients. In one embodiment, an infection is detected in ME/CSF patients. In one embodiment, an infection is undetectable in ME/CSF patients. Pathogenetically, a disturbed immune regulation with persistent immune activation is suspected. In addition, many patients show a persistent T-cell activation. Fatigue also frequently occurs in patients with autoimmune diseases, such as rheumatoid arthritis or multiple sclerosis, in chronic infectious diseases, such as hepatitis or viral cardiomyopathy, under immunomodulatory treatment with interferon, tumor fatigue may become chronic after completion of tumor treatment.

ME/CFS is typically characterized by the triad of fatigue, neurocognitive, and immunologic symptoms. The leading symptom ME/CFS is an exceptionally rapid physical and mental exhaustibility, which in extreme cases can lead to extensive disability and the need for long-term care. Frequently, ME/CSF begins acutely with flu-like symptoms, often accompanied by sore throat, headache, myalgias, arthralgias, impaired concentration, memory, and sleep. ME/CFS also initially resembles postviral fatigue, a fatigue lasting weeks to months after a viral infection, but it can also occur after bacterial infections. ME/CSF can also begin insidiously, progress in episodes, and increase or decrease in severity over years. It is not uncommon for a period of physical or mental overexertion to coincide with the onset of the disease. In one embodiment, ME/CFS has an acute onset. In one embodiment, ME/CFS has an insidiously onset. In one embodiment, ME/CFS has a progress in episodes. In one embodiment, ME/CFS increases in severity over years. In one embodiment, sGC stimulator treatment improves symptoms of ME/CFS, wherein acute ME/CSF preferably occurs after the acute phase of an infection, such as Epstein Barr virus infection. In one embodiment, ME/CFS is a postviral disease. In one embodiment, ME/CFS is a postbacterial disease.

In one embodiment, ME/CFS has a non-acute onset, wherein non-acute onset of ME/CSF preferably occurs after the acute phase of an infection, such as Epstein Barr virus infection. In one embodiment, ME/CFS progresses episodically over years and may increase or decrease in severity.

A typical feature of ME/CFS is the worsening of the condition, known as postexertional fatigue or malaise, which is sometimes impressively described by patients and often only occurs on the day after exertion and can last for days. The clinical picture is almost always accompanied by neurocognitive symptoms. Medical conditions of ME/CFS comprise post-exertional malaise (PEM), post-exertional neuroimmune exhaustion, neurological impairment, immunological, gastrointestinal or urogenital impairment, and impairment of energy production/transport. Symptoms of ME/CSF comprise rapid exhaustibility due to physical, intellectual, or psycho-social exertion compared to before the onset of the disease, prolonged debilitation after exertion, impaired orthostasis response, pain (e.g., headache, pain in muscles, tendons, joints, abdomen, or chest), neurocognitive impairment after exertion, neurocognitive impairment independent of exertion, wherein neurocognitive impairment includes e.g. for example, difficulties with information processing, slowed thinking, impaired concentration, clouding of consciousness, confusion or disorientation, cognitive overload, difficulty making decisions, slowed speech, dyslexia, word-finding difficulties, difficulties with short-term memory, sleep disturbances (e. g. disturbed sleep patterns, non-restorative sleep), neurosensory perception or movement disorders, urogenital complaints, susceptibility to viral infections, prolonged recovery periods from infections, flu-like symptoms, chronic or recurrent, activated or exacerbated by stress, intolerance to foods, medications, odors, or chemicals, cardiovascular intolerance, respiratory impairment, loss of thermostability (e. g. sensation of cold, flushing), intolerance to temperature extremes, intolerance to loudness, and/ or hypersensitivity. Exhaustion or PEM may occur immediately after activity or may be delayed for hours or days. The recovery period after physical exertion is prolonged. It is often 24 hours, but may last days or weeks. Exhaustion and fatigability are not a result of unusual exertion and do not improve significantly with rest. Despite severe exhaustion, there are often problems falling asleep and sleeping through the night.

Diagnosis of ME/CFS is primarily by clinical symptoms. For diagnosis, the so-called "Canadian criteria" are useful, which also include CFS-typical symptoms and the less extensive CDC or FUKUDA criteria. For differentiation from other forms of fatigue, the DEGAM guidelines "Fatigue" are also to be considered. Further diagnostics to exclude internal and neurological diseases including a cranial MRI should always be performed. A comprehensive endocrinological evaluation must be part of any ME/CFS diagnosis.

In one embodiment, diagnostics for ME/CFS and Post COVID Syndrome comprise one or more determination of the laboratory parameters described herein, including cranial MRI, endocrinologic diagnostics, Schellong test (detection of orthostatic intolerance), psychological differential diagnostics (e.g., exclusion of depression), immunodiagnostic (e.g. detection of CRP, T-cell cytokines, mannose-binding lectin, immunoglobulins, blood cell sedimentation rate, NK cell function), infection diagnostics (e.g. detection of SARS-CoV-2, EBV, EBV-VCA-IgM, EBV-VCA-IgG, EBNA-antibodies, HSV-1-, HSV-2, coxiella burnetii, legionella, denguevirus, rickettsia, bartonella, chlamydia or enterovirus), and/or mitochondrial dysfunction.

*Post COVID Syndrome and diagnosis:*

Post COVID Syndrome occur after an SARS-CoV-2 infection, wherein said SARS-CoV-2 infection may have an asymptomatic, mild, moderate, or severe course of COVID19. Post COVID Syndrome can present as a full-blown post-infectious chronic fatigue syndrome ME/CFS (ICD-10 G93.3). The exact causes of Post COVID Syndrome are not yet known. Persistence of the SARS-CoV-2 virus or viral components over weeks and months may play a role. Other possible pathomechanisms are persistent postinfectious hypercoagulability, thrombosis, chronic immune dysregulation, (hyper)inflammation and autoimmunity.

According to the WHO criteria from October 2021, Post COVID Syndrome is defined as: "Post COVID-19 condition occurs in individuals with a history of probable or confirmed SARS CoV-2 infection, usually 3 months from the onset of COVID-19 with symptoms and that last for at least 2 months and cannot be explained by an alternative diagnosis. Common symptoms include fatigue, shortness of breath, cognitive dysfunction but also others and generally have an impact on everyday functioning. Symptoms may be new onset following initial recovery from an acute COVID-19 episode or persist from the initial illness. Symptoms may also fluctuate or relapse over time."

Post COVID Syndrome may also comprise (i) new symptoms that occurred after the end of the acute phase but are understood to be a consequence of COVID-19 disease,

(ii) Worsening of a pre-existing underlying condition (Ceravolo MG, Arienti C, de Sire A et al. Rehabilitation and COVID-19: the CochraneRehabilitation 2020 rapid living systematic review. Eur J Phys Rehabil Med 2020; 56: 642-651.DOI: 10.23736/S1973-9087.20.06501-6).

Post COVID Syndrome is a patient group with moderate to severe fatigue and exertion intolerance persistent for more than 6 months. About 50% of these patients meet the CCC 2003 diagnostic criteria of ME/CFS (Kedor C, Freitag H, Meyer-Arndt L, Wittke K, Zoller T, Steinbeis F, et al. Chronic COVID-19 Syndrome and Chronic Fatigue Syndrome (ME/CFS) following the first pandemic wave in Germany - a first analysis of a prospective observational study. medRxiv. 2021:2021.02.06.21249256.). The other half of these patients were similarly affected, but with a PEM duration <14 h, therefore not fulfilling the ME/CFS criteria. These patients are referred to as Post COVID Syndrome.

Both patient groups, ME/CFS and Post COVID Syndrome, have a high burden of symptoms that severely limit daily life (approx. 50 % incapable of working). The median age of the patients is 36 and 42 years, respectively, with a female to male ratio of 2:1. Most patients are strongly impaired in daily life with cognitive dysfunction, loss of muscle strength, headache and muscle pain. There is currently no causal therapy for ME/CFS nor for the Post COVID Syndrome patients (Nacul L, Authier FJ, Scheibenbogen C, Lorusso L, Helland IB, Martin JA, et al. European Network on Myalgic Encephalomyelitis/Chronic Fatigue Syndrome (EUROMENE): Expert Consensus on the

Diagnosis, Service Provision, and Care of People with ME/CFS in Europe. Medicina. 2021;57(5):510.).

In one embodiment, Post COVID Syndrome diagnostics comprise physical examination including neurological, psychological and functional status, and determining clinical parameters and laboratory parameters as described herein, such as persistence of SARS-CoV-2 virus or viral components, chronic (hyper)inflammation, and autoimmunity. In one embodiment, clinical parameters for Post COVID Syndrome diagnostics comprise blood pressure, heart rate, temperature, respiratory rate, pulmonary function analysis, SpO2, D-dimers, ECG, chest x-ray, and oxygen saturation. In one embodiment, laboratory parameters for Post COVID Syndrome diagnostics comprise BB, CRP, creatinine, urea, transaminases, TSH, urine stix (optional: CK,troponin, ferritin, D-dimers, NT-proBNP, anti-phospholipid autoantibodies, autoantibodies against interferons, neutrophils, citrulline peptides and cell nuclei, lymphopenia, and D-dimer levels. In one embodiment, medical score for Post COVID Syndrome diagnostics using questionnaires comprise screening questions on fatigue, persistent physical exhaustion, exercise intolerance/PEM (one of the most common symptoms in most post/long-COVID studies), pain,cognitive dysfunction, depressive mood, and anxiety disorder (seeFatigue and Psychosomatics section).

*Treatment of Post COVID Syndrome and/or ME/CSF:*

In particular, the treatment described herein relates to either reducing or curing a Post COVID Syndrome or ME/CSF or symptoms thereof via the activation or stimulation of soluble guanylate cyclase in endothelial cells and endothelial function within the vascular system. The interventional therapy as described herein is intended to encompass

reduced progression or reduction of a Post COVID Syndrome or ME/CSF, preferably due to a reduced likelihood of endothelial dysfunction, vascular constriction, vascular damage, cognitive impairment, mitochondrial dysfunction, retinal endothelial dysfunction, and/or hypo-perfusion via activation of sGC in endothelial cells and endothelial function within the vascular system thereof described herein.

In the present invention, as the "fatigue symptoms" of "myalgia encephalomyelitis/chronic fatigue syndrome with fatigue symptoms", for example, fatigue symptoms satisfying the following (a) can be exemplified; preferably, the following can be exemplified (a) and (b) fatigue symptoms; more preferably, satisfy the following fatigue symptoms (a), (b) and (c); more preferably, satisfy the following fatigue symptoms (a), (b) and (c), and satisfy the fatigue symptoms of (d) and/or (e):

(a) Compared with the occupational, academic, social activities, and personal activity levels before the onset, there is a significant decline in function or dysfunction, which lasts for more than 6 months and is accompanied by a sense of fatigue. Its severity is often serious, it is a state of obvious disease, and it is not due to recent overwork. In addition, the symptoms will not disappear even if they are resting. (b) will aggravate after exertion (c) Will not recover even when sleeping (d) Impairment of cognitive function (e) Unable to get up and stand.

As used herein, a "patient with symptoms of an infectious disease" refers preferably to a subject who presents with one or more of, without limitation, fever, diarrhea, fatigue, muscle aches, coughing, having trouble breathing, severe headache with fever, rash or swelling, unexplained or prolonged fever or vision problems. Other symptoms may be fever and chills, very low body temperature, decreased output of urine (oliguria), rapid pulse, rapid breathing, nausea and vomiting. In preferred embodiments, the symptoms of an infectious disease are fever, diarrhea, fatigue, muscle aches, rapid pulse, rapid breathing, nausea and vomiting and/or coughing.

As used herein, "infectious disease" comprises all diseases or disorders that are associated with bacterial and/or viral and/or fungal infections.

As used herein, a patient within an "acute phase" of an infectious disease is a subject who is symptomatic for an infectious disease and actively presents a group of physiologic changes that occur shortly after the onset of an infection and may include an increase in the blood level of various proteins, fever, and other metabolic changes. An acute phase includes conditions or symptoms that usually occur and develop rapidly, and where these conditions or symptoms persist for a limited time, usually resolving within less than one month, less than 3 months, or less than six months. In one embodiment, the acute phase affects one part of the body and responds to treatment. As used herein, "onset of an infection" also means the occurrence of one or more first symptoms associated with that infection occurring.

As used herein, "chronic" comprises a human health condition, syndrome or disease that has reoccurring, persistent or otherwise long-lasting conditions, or a disease that comes with time. A chronic condition or course of the disease often lasts longer than one month, preferably three months, and usually affects several areas of the body, and may potentially not respond at all or not respond completely to a relevant treatment. In one embodiment, a chronic condition of ME/CFS or Post COVID Syndrome occurs after the onset of first symptoms of the infection, or after the acute phase of the infection. In one embodiment, a chronic condition of ME/CFS or Post COVID Syndrome persists from the onset of first symptoms of the infection, or from the onset of the dysfunction.

As used herein, a "patient with symptoms of chronic vascular dysfunction", in particular in the vascular system and endothelium, is a subject who presents with one or more of, without limitation, chronic vascular dysfunction phenotype, including endothelial dysfunction, retinal endothelial dysfunction, vascular stiffness, vascular constriction, hypo-perfusion characterized by elevated endothelial marker ET-1, IL-8, CrP, hemoglobin, and/or NT-proBNP compared to a healthy subject, and/or decreased level of one or more of, without limitation, ferritin, ANG-2, ACE2, ACE-1, and/or bilirubin.

As used herein, a "patient with symptoms of ME/CSF", is a subject who presents with one or more of, without limitation, rapid exhaustibility due to physical, intellectual, or psycho-social exertion compared to before the onset of the disease, prolonged debilitation after exertion, impaired orthostasis response, pain (e.g., headache, pain in muscles, tendons, joints, abdomen, or chest), neurocognitive impairment after exertion, neurocognitive impairment independent of exertion, wherein neurocognitive impairment includes e.g. for example, difficulties with information processing, slowed thinking, impaired concentration, clouding of consciousness, confusion or disorientation, cognitive overload, difficulty making decisions, slowed speech, dyslexia, word-finding difficulties, difficulties with short-term memory, sleep disturbances (e. g. disturbed sleep patterns, non-restorative sleep), neurosensory perception or movement disorders, urogenital complaints, susceptibility to viral infections, prolonged recovery periods from infections, flu-like symptoms, chronic or recurrent, activated or exacerbated by stress, intolerance to foods, medications, odors, or chemicals, cardiovascular intolerance, respiratory impairment, loss of thermostability (e. g. sensation of cold, flushing), intolerance to temperature extremes, intolerance to loudness, and/ or hypersensitivity, characterized by diminished hand grip, PEM, immune cell activation, decreased ferritin, detection of an infectious agent, autoantibodies, altered immunoglobulins and/or medical scores out of reference level, compared to a healthy subject.

As used herein, a "patient with symptoms of Post COVID Syndrome", is a subject who presents with one or more of, without limitation, fatigue, shortness of breath, and both impaired physical, mental performance, sleep disturbance, depression, anxiety, stress intolerance, cognitive disturbance, orthostatic inolerance, dyspnea (resting/exertional)

cough, olfactory disturbances, gustatory disturbances, muscle weakness, psychological complaints, anxiety, impaired performance, PEM, impaired activity, altered breathing pattern, obsessive-compulsive behavior, hair loss, stress, paralysis, sensory disturbances, dizziness, nausea, diarrhea, loss of appetite, tinnitus, ear pain, loss of voice, palpitations, tachycardia, and pain, particularly muscle pain and headache characterized by diminished hand grip, detecting of SARS-CoV-2 virus or viral components, chronic (hyper)inflammation, autoimmunity, B cell abundance, T cell abundance, increased interleukins, decreased blood pressure, increased heart rate, decreased respiratory rate, decreased endothelial function, autoantibodies, immunoglobulins and/or medical scores out of reference level compared to a healthy subject. In some embodiments, symptoms of Post COVID Syndrome comprise muscle weakness, muscle fatigue, muscle pain, PEM 2-14 hours, moderate fatigue, severe fatigue, exercise intolerance, neurocognitive impairment (e.g. memory disturbance), post exertional malaise, hypersensitivity, and/or pain (e.g. headache), preferably decreased handgrip strength, fatigue. In some embodiments, patient with symptoms of Post COVID Syndrome do not fulfill the CCC criteria for ME/CFS.

As used herein, a patient with "symptoms of a SARS infection" is a subject who presents with one or more of, without limitation, fatigue/malaise, fever/higher temperature, cough, respiratory problems, loss of smell, loss of taste, headache, arthralgia, chest pain, and/or gastrointestinalsymptoms. In some embodiments, symptoms of infection with a SARS-virus are fever, sore throat, cough, myalgia or fatigue, and in some embodiments, additionally, sputum production, headache, hemoptysis and/or diarrhea. In some embodiments, symptoms of an infection with a SARS-coronavirus, for example SARS-CoV-2, are fever, sore throat, cough, lack of taste and/or smell, shortness of breath and/or fatigue.

As used herein, the term "a patient that is at risk of developing a Post COVID Syndrome" relates to a subject, preferably distinct from any given person in the general population, who has an increased (e.g. above-average) risk of developing Post COVID Syndrome after onset of SARS infection. In some embodiments, the patient had symptoms of SARS or symptoms of a SARS Coronavirus infection. In some embodiments, the patient had no symptoms of SARS or symptoms of a SARS Coronavirus infection. In some embodiments, the subject has been in contact with people with SARS Coronavirus infections or symptoms. In some embodiments, the person at risk of developing SARS has been tested for the presence of a SARS Coronavirus infection. In some embodiments, the person at risk of developing SARS has tested positive for the presence of a SARS Coronavirus infection, preferably a coronavirus infection, who has had or is suspected of having had SARS-CoV-2 infection

In embodiments, the patient at risk of developing Post COVID Syndrome is an asymptomatic patient who had not shown specific symptoms of SARS. An asymptomatic patient may be at risk of developing Post COVID Syndrome because the patient has been in contact with a person infected with a SARS Coronavirus. Methods of determining contact/physical proximity to an infected person are known to the skilled person and equally apply to the method of the invention.

Further, a diagnosis of ME/CSF and Post COVID Syndrome can be made when the activity level of a subject averages 50% or less, as determined using standard methods of the art. At least 25% of subjects are unable to leave their homes or are even bedridden at least once in their lives. The severity of symptoms must result in a significant reduction in the subject’s activity level, as measured by the subjective previous activity level. Reduction in activity level can be divided into four levels: (i) Mild: about a 50% reduction in activity level, (ii) Moderate: mostly housebound, (iii) Severe: mostly bedbound, (iv) Very severe: completely confined to bed and dependent on assistance for basic activities.

As used herein, a "therapeutically effective amount" of a sGC stimulator, preferably vericiguat, refers to an amount that is capable of improving the health of a patient in the relevant patient group. For example, increasing activity level, reducing the need assistance, reducing the number of bedridden days, increase in time (minutes, hours, days, weeks, months) spent outside home, reducing fatigue, increase endothelial function (e.g. level of perfusion, blood flow, contractility, vasoconstriction, vascular stiffness), increase mitochondrial function, increase level of retinal endothelial function, increase of the concentration time, increase of the memory capacity, increase hand grip strength, increasing muscle strength, reducing the level of pain, reduction in time having pain, increasing survival, modulating or regulating an inflammatory response or inflammation, decreasing psychological complaints (e.g., depression, anxiety), resolving psychological complaints (e.g., depression, anxiety), increase in time of exercise tolerance, decrease in time of physical exhaustion, increasing oxygen saturation, increase in level of cGMP, reducing autoantibodies, recovering olfactory/gustatory qualities, clearance of the infectious agent and/or normalizing laboratory parameters and medical scores, described herein, to reference level of healthy control subject or healthy patient population.

A "therapeutically effective amount" of a non-soluble guanylate cyclase activator drug (drug that is not a sGC activator) recommended by the S1 guidelines for Post COVID Syndrome and/or ME/CSF refers to an amount that is capable of reducing disease symptoms, such as the number of bedridden days, reduction in time having pain, modulating or regulating an inflammatory response or inflammation, reducing autoantibodies, and/or reducing vascular dysfunction.

The term "modulating" includes "increasing" or "stimulating," as well as "decreasing" or "reducing," typically in a physiologically relevant amount.

The terms "enhance" or "enhancing," or "increase" or "increasing," or "stimulate" or "stimulating," refer generally to the ability of one or more agents or compositions to produce or cause a greater physiological response (i.e., downstream effects) in a cell or a tissue, as compared to the response caused by either no treatment, or a non-soluble guanylate

cyclase activator drug treatment recommended by the S1 guidelines for Post COVID Syndrome and/or recommended by the guidelines for ME/CSF, vitamin supplementation, pain relievers, or physical activity.

An "increased" or "enhanced" number may include an increase that is 1.1, 1.2, 2, 3, 4, 5, 6, 7, 8, 9, 10, 15, 20, 30 or more times greater (e.g., 500, 1000 times greater), including all integers and decimals in between.

Examples include increased sGC activation and increased endothelial function. A measurable physiological response may include increasing activity level, increase in time (minutes, hours, days, weeks, months) spent outside home, reducing fatigue, increase of the concentration time, increase of the memory capacity, increase hand grip strength, increasing muscle strength, reducing the level of pain, reduction in time having pain, modulating or regulating an inflammatory response or inflammation, increase in time of exercise tolerance, decrease in time of physical exhaustion, increase in level of cGMP, recovering olfactory/gustatory qualities, and/or normalizing medical scores, decreased number of bedridden days, decreased fatigue, decreased need for assistance in daily life, or clinical marker of endothelial dysfunction, vasoconstriction, hypoperfusion. Relevant physiological or cellular responses (in vivo or in vitro) will be apparent to persons skilled in the art.

The term "reduce" may relate generally to the ability of soluble guanylate cyclase activator drug, preferably vericiguat, or in combination with non-soluble guanylate cyclase activator drug treatment recommended by the S1 guidelines for Post COVID Syndrome and/or recommended by the guidelines for ME/CSF, such as vitamin supplementation or pain relievers, to "decrease" a relevant physiological or cellular response, such as a symptom of a disease or condition described herein, as measured according to routine techniques in the diagnostic art.

A "decrease" in a response may measured compared to the response produced by no treatment, or a non-soluble guanylate cyclase activator drug treatment recommended by the S1 guidelines for Post COVID Syndrome and/or recommended by the guidelines for ME/CSF, such as vitamin supplementation or pain relievers, and may include, for example, a 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% decrease, including all integers in between.

In some embodiments, the term "biomarker of interest" refers to a nucleic acid sequence coding for a protein, or the protein itself, involved in endothelial function, perfusion, vasomotor regulation, hypertension, vasoconstrictor activity, endothelial vasodilator capacity, endothelial integrity, endothelial vessel density, vascular function, vasodilatation, immune response and/or inflammation, preferably IL-8, ET-1, ACE2, ACE1, ANG2, bradykinin receptor1, β2 adrenergic receptor, arginase 1.

For example, elevated levels of the potent vasoconstrictor ET-1 were found in ME/CFS patients.

The expression of the biomarker of interest in the sample is compared to the expression of the biomarker in a control sample (e.g., a sample of target cells in culture that express the biomarker) that is not exposed to a soluble guanylate cyclase activator drug, e.g. vericiguat. A value of 100% may be assigned to the expression of the biomarker in a control sample. In particular embodiments, inhibition or reduction of expression of a biomarker is achieved when the level of biomarker expression or biomarker protein in the test sample or test mammal relative to the level of biomarker expression or biomarker protein in the control sample is about 95%, 90%, 85%, 80%, 75%, 70%, 65%, 60%, 55%, 50%, 45%, 40%, 35%, 30%, 25%, 20%, 15%, 10%, 5%, or 0%. In other words, the soluble guanylate cyclase activator treatment, e.g. vericiguat, reduces or inhibits expression of a biomarker by at least about 5%, 10%, 15%, 20%, 25%, 30%, 35%, 40%, 45%, 50%, 55%, 60%, 65%, 70%, 75%, 80%, 85%, 90%, 95%, or 100% in a test compared to the level of biomarker expression in a control sample or control mammal that has not been exposed to a sGC stimulator drug, e.g. vericiguat.

In particular embodiments, induction or increase of expression of a biomarker is achieved when the level of biomarker in the test sample or test mammal relative to the level of biomarker expression or biomarker protein in the control sample is more than 5%, 10%, 15%, 20%, 25%, 30%, 35%, 40%, 45%, 50%, 55%, 60%, 65%, 70%, 75%, 80%, 85%, 90%, 95%, 100% or more than 100%, above the level in the control sample. In other words, the soluble guanylate cyclase activator treatment, e.g. vericiguat, increases or induces expression of a biomarker or biomarker protein by at least about 5%, 10%, 15%, 20%, 25%, 30%, 35%, 40%, 45%, 50%, 55%, 60%, 65%, 70%, 75%, 80%, 85%, 90%, 95%, 100% or more than 100% in a test compared to the level of biomarker expression in a control sample or control mammal that has not been exposed to a sGC stimulator drug, e.g. vericiguat.

In one embodiment, samples for endothelial function monitoring for evaluating systemic effects of sGC stimulation are also collected every day, every second day, every 3rd day, every 4th day, every 5th day, every 6th day, every week, every second week, every 3rd week, or every month.

Post COVID Syndrome associated with SARS corona virus infection

In some embodiments, the patient that has or is at risk of developing a severe acute respiratory syndrome (SARS) has a coronavirus infection. Coronaviruses are a group of related viruses that cause diseases in mammals and birds. The scientific name for coronavirus is Orthocoronavirinae or Coronavirinae. Coronavirus belongs to the family of Coronaviridae. The family is divided into Coronavirinae and Torovirinae sub-families, which are further divided into six

genera: Alphacoronavirus, Betacoronavirus, Gammacoronavirus, Deltacoronavirus, Torovirus, and Bafinivirus. In humans, coronaviruses cause respiratory tract infections that can be mild, such as some cases of the common cold, and others that can be lethal, such as SARS, MERS, and COVID-19.

Various species of human coronaviruses are known, such as, without limitation, Human coronavirus OC43 (HCoV-OC43), of the genus β-CoV, Human coronavirus HKU1 (HCoV-HKU1), of the genus β-CoV, Human coronavirus 229E (HCoV-229E), α-CoV, Human coronavirus NL63 (HCoV-NL63), α-CoV, Middle East respiratory syndrome-related coronavirus (MERS-CoV), Severe acute respiratory syndrome coronavirus (SARS-CoV), Severe acute respiratory syndrome coronavirus 2 (SARS-CoV-2). Advances in nucleic acid sequencing technology (commonly termed Next-Generation Sequencing, NGS) are providing large sets of sequence data obtained from a variety of biological samples and allowing the characterization of both known and novel virus strains. Established methods are therefore available for determining a Coronavirus infection.

SARS is induced via droplet transmission and replication of the virus could be shown in the upper and lower respiratory tract or gastrointestinal mucosa. In parallel the virus is also capable of directly invading cells of different organs such as liver, kidney, heart and brain. Another distinct mechanism appears to be the direct invasion of the virus in T-cells. A significant number of SARS patients, who suffer COVID-19, have a clinically low concentration of lymphocytes in the blood, also known as Lymphocytopenia. Clinically, patients show respiratory symptoms such as dry cough and shortness of breath, fever or diarrhea. But symptoms associated with acute liver injury, heart injury or kidney injury can also occur. In a less severe state of SARS, patients can show mild or even no symptoms.

Clinical and scientific investigations show that SARS-CoVs bind to the epithelial cells via the Angiotensin converting enzyme 2 receptor (ACE2). ACE2 is a cell membrane linked carboxypeptidase which is expressed in vascular endothelia, kidney, bladder, heart, nasal mucosa, bronchus and lung. As one consequence binding of the virus leads to an epithelial and endothelial cell damage along with vascular leakage, which triggers a secretion of proinflammatory cytokines and chemokines. The virus also mediates ACE2 downregulation and shedding which further promotes a dysfunctional renin-angiotensin system (RAS). Once the RAS is disturbed it can lead an inflammatory response and to vascular permeability. Focusing on the respiratory system, ACE2 shedding can lead to pulmonary vascular permeability and subsequently to pulmonary edema.

Older patients (above 60 years), patients with chronic diseases (e.g. cardiovascular diseases, diabetes, cancer, COPD) or immune compromised patients are considered to be at a higher risk to face a severe development of SARS. Smoking and obesity are also considered as risk factors. Embodiments of a SARS Coronavirus include, without limitation, any coronavirus that induces a SARS or SARS-similar pathology. Particular embodiments include, without limitation, the SARS Coronavirus (SARS-CoV-1), MERS-CoV, and the SARS-CoV-2, which causes COVID-19. The strain SARS-CoV-2 causes COVID-19, a disease which brought about the ongoing 2019-2020 coronavirus pandemic. The disease was first identified in December 2019 in Wuhan, the capital of China's Hubei province, and spread globally. Common symptoms include fever, cough, and shortness of breath. Other symptoms may include muscle pain, diarrhea, sore throat, loss of taste and/or smell, and abdominal pain. While the majority of cases result in mild symptoms, some progress to viral pneumonia, multi-organ failure, Post COVID Syndrome.

## Medical score

A medical score is selected from a group consisting of SF-36 health score, Chalder Fatigue Score, CCC Symptom Score, hand grip strength test, Bell disabling scale, MBSQ score, PEM score, COMPASS-31, cognitive function (SDMT), Blood pressure (BP), Heart Rate (HR) regulation in passive standing test [CDE NIH], HRV and in stress index (SI), pain scale, and reactive hyperemia index (RHI).

One or more medical score can be used to assess symptom severity of ME/CSF and/or Post COVID Syndrome.

## Treatment outcome

Diagnosis of ME/CFS can be also assessed based on the 2003 Canadian Consensus Criteria CCC in accordance with the European guidelines (Carruthers BM, et al Journal of Chronic Fatigue Syndrome, 2003;11:7-115. doi: 10.1300/J092v11n01_01, Nacul L, et al, European Network on Myalgic Encephalomyelitis/Chronic Fatigue Syndrome (EUROMENE): Expert Consensus on the Diagnosis, Service Provision, and Care of People with ME/CFS in Europe. Medicina (2021) 57(5):510. Published online 2021 May 19. doi: 10.3390/medicina57050510).

A key symptom of ME/CFS is exertion intolerance with aggravation of all symptoms upon minor mental and physical exertion, typically lasting for more than 14 hours up to several days, the post exertional malaise (PEM). This symptom can be assessed by the PEM questionnaire (Cotler, 2018) determining frequency (up to 20 points), severity (up to 20 points), and length (up to 6 points) of PEM, and treatment outcomes. Both fatigue and PEM can severely impair physical activities of daily life, which can be assessed by Short Form Health Survey 36 physical function (SF-36-PF) (Ware & Sherbourne, 1992). This questionnaire has been used in most RCT in ME/CFS as primary aim.

Table 3 comprises medical scores used to determine subjective changes of symptoms in subjects suffering from ME/CSF and/or Post COVID Syndrome while or after being treated with one or more sGC stimulators, e.g. vericiguat.

Table 3: Clinical scores that can be used to assess (subjective) changes in symptoms to described treatment effectiveness

| Score | Use | Range | Source / Reference |
|---|---|---|---|
| Bell Disabling Scale | Disease severity and disability | 0 (total loss of selfdependence) to 100 (without restrictions) | https://cfc.charite.de/fileadmin/user_upload/micros ites/kompetenzzentren/cfc/Landing_Page/Kanadis che_Kriterien_Bell.pdf |
| Self-reported Symptoms (MBSQ) | Disease and symptom severity | To be added | |
| SF-36 Health Status Questionnaire | Physical function | 0 (greatest possible health restrictions) to 100 (no health restrictions) | https://clinmedjournals.org/articles/jmdt/jmdt-2-023-figure-1.pdf Expected change: Improvement of physical function bv 20 points |
| Postexertional malaise (PEM) criteria | Frequency, seve rity and duration of PEM symptoms, such as muscle | 0 (no PEM) to 46 (frequent, severe and long PEM) | Cotler J, Holtzman C, Dudun C, Jason LA. A Brief Questionnaire to Assess Post-Exertional Malaise. Diagnostics (Basel, Switzerland). 2018;8(3). |
| | weakness, pain or mental tiredness | | Expected change: PEM frequency and severity reduction by 30 %, each; improvement by one category. |
| Chalder Fatigue Score | Severity of fatigue | 0 (no fatigue) to 33 (heavy fatigue) | Chalder T, Berelowitz G, Pawlikowska T, Watts L, Wessely S, Wright D, et al. Development of a fatigue scale. Journal of psychosomatic research. 1993;37(2):147-53. Expected change: Improvement by 20 % |
| Canadian Consensus Criteria (CCC) Symptom Score | Quantification of symptoms | 1 (no symptoms) to 10 (extreme symptoms) | https://cfc.charite.de/fileadmin/user_upload/micros ites/kompetenzzentren/cfc/Landing_Page/Kanadis che_Kriterien_Bell.pdf (Fluqe 2015. Scheibenboqen 2021) Expected change: Improvement |
| COMPASS-31 | Autonomic dysfunction including heart, vasomotor, orthostatic, ocular, bladder and gastrointestinal symptoms | 0 (without symptoms) to 100 (strong autonomic dysfunction) | Sletten et al. COMPASS 31: a refined and abbreviated Composite Autonomic Symptom Score. Mayo Clinic proceedings. 2012;87(12): 1196-201. Expected change: improvement means a reduction in score |
| SDMT-Test | Cognitive function including dimensions of processing speed and working memory, in addition of visual search and scanning, oculomotor functioning | number of correct responses (out of a maximum of 110) in 90 seconds is recorded | Smith A. Symbol digit modalities test: Manual. Los Angeles, CA: Western Psychological Services, 1982. Expected change: higher numbers mean better cognitive performance; clinically meaningful change in test performance: 3-4 points or greater change in scores |

A "SF-36 health score" or "SF-36 score" is also derived from the Short Form (SF-36) Health Questionnaire, a disease-unspecific measurement instrument for surveying health-related quality of life. SF-36 health score can also be used for ME/CFS and Post COVID Syndrome diagnostics and treatment response measure. The SF-36 can describe the individual health status of patients and measure and compare disease-related burdens in the course. The SF-36 is composed of eight domains assessed with scales corresponding to the weighted sums of the responses in each section. To score the SF-36, scales are standardized with a scoring algorithm or by the SF-36v2 scoring software to obtain a

score ranging from 0 to 100. Higher scores indicate better health status, and a mean score of 50 has been articulated as a normative value for all scales (Ware, J.E., Jr. & Sherbourne, C.D. The MOS 36-item short-form health survey (SF-36).

"Chalder Fatigue Score" or also "Chalder Fatigue Scale" is an 11-question questionnaire developed by Trudie Chalder's research team at King's College London to measure the severity of fatigue in fatiguing illness and also defines clinical criteria for the diagnosis of ME/CFS, which was published in 2003 and can also be applied to Post COVID Syndrome and treatment response measure. The Fatigue Scale has been used in several randomized trials of behavioral interventions in patients with ME/CFS (Cella, M. & Chalder, T. Measuring fatigue in clinical and community settings. Journal of psychosomatic research 69, 17-22 (2010), Chalder T, et al, Development of a fatigue scale. J Psychosom Res. 1993;37(2):147-53. doi: 10.1016/0022-3999(93)90081-p. PMID: 8463991).

"Canadian Consensus Criteria" (CCC) or "Canadian working definition" "CCC Symptom Score" also defines clinical criteria for the diagnosis of ME/CFS, which was published in 2003 and can also be applied to Post COVID Syndrome and treatment response measure. CCC is more stringent than the Fukuda criteria and represents a more impaired population, according to Leonard Jason. Adults are diagnosed after at least six months of persistent impairment, and children are diagnosed after at least three months. In CCC, a patient with ME/CFS meets criteria for fatigue, malaise and/or exhaustion after exertion, sleep disturbances, and pain, has two or more neurological/cognitive manifestations, and one or more symptoms from two of the categories (i) autonomic, (ii) neuroendocrine, and (iii) immune manifestations. (Carruthers, Bruce M. et al (2003), "Myalgic Encephalomyelitis/Chronic Fatigue Syndrome: Clinical Working Case Definition, Diagnostic and Treatment Protocols", Journal of Chronic Fatigue Syndrome, 11 (2): 7-115). In one embodiment, Canadian Consensus Criteria score value of ME/CFS and Post COVID Syndrome patients can be 1 or 2 or 3 or 4 or 5 or 6 or 7 or 8 or 9 or 10. In one embodiment, the effectiveness and physiological response of the treatment, as described herein, is determined by a decreased Canadian Consensus Criteria score value compared to said value determined before treatment initiation.

Hand grip strength test: "Hand grip strength test" is also used for ME/CFS and Post COVID Syndrome diagnostics and treatment response measure. Hand grip strength is a general strength test and measures the maximum isometric strength of the hand and forearm muscles of the human subject. Human subjects with strong hands are usually also strong in other areas. While performing the hand grip strength test, a human subject holds the dynamometer in the hand to be tested. Said subject squeezes the force gauge with maximum isometric force, and maintain for about 5 seconds. No other body movements are allowed. For example, muscle fatigue and fatigability can be assessed by ten repeat hand grips at maximum force (Fmax1 and Fmean1) and can be repeated after 60 minutes (Fmax2 and Fmean2). Values obtained during the test (i.e. Fmax1 and Fmean1, Fmax2 and Fmean2) are compared to reference values of healthy subjects. ME/CFS or Post COVID Syndrome patients generally present values below reference values for Fmax1/2 and Fmean1/2. (Jakel, B., et al. Hand grip strength and fatigability: correlation with clinical parameters and diagnostic suitability in ME/CFS. J Transl Med 19, 159 (2021)). In one embodiment, the effectiveness and physiological response of the treatment, as described herein, is determined by an increased Hand grip strength score value compared to said value determined before treatment initiation.

Bell disabling scale: "Bell disabling scale", also known as "Bell CFIDS disability scale" or "Bell disability scale" or "Bell fatigue scale", is also a questionnaire used as symptom scale for ME/CFS and Post COVID Syndrome diagnostics and treatment response measure. The Bell disabling scale is also a self-scoring test comprising eleven statements, wherein said scale is scored from 0 (very severe, bedridden constantly) - 100 (healthy). (Bell DS. The Doctor's Guide to Chronic Fatigue Syndrome: Understanding, Treating and Living with CFIDS. Boston: Da Capo Lifelong Books; 1995.). In one embodiment, the effectiveness and physiological response of the treatment, as described herein, is determined by an increased Bell disabling scale score value compared to said value determined before treatment initiation.

MBSQ score: Munich Berlin Symptom Questionnaire (MBSQ) is also a questionnaire used for ME/CFS and Post COVID Syndrome diagnostics and treatment response measure. MBSQ is a German-language version of the DePaul Symptom Questionnaire Short Form (DSQ-SF; Jason 2018) and was developed by TU Munich and Charité Berlin for use in the German ME/CFS Register. MSBQ is complementary to and compared with the questionnaires on disease symptoms which can be collected in visits. Severity of a symptom is assessed on a scale from 0 = symptom not present to 4 = very severe. (Sunnquist M, Lazarus S, Jason LA. The development of a short form of the DePaul Symptom Questionnaire. Rehabil Psychol. 2019 Nov;64(4):453-462. doi: 10.1037/rep0000285. Epub 2019 Jul 18. PMID: 31318234; PMCID: PMC6803042.) In one embodiment, the effectiveness and physiological response of the treatment, as described herein, is determined by a decreased MBSQ score value compared to said value determined before treatment initiation.

PEM score: Postexertional malaise (PEM) is also one of the main symptoms of ME/CFS and Post COVID Syndrome and treatment response measure. PEM is "a delayed and significant worsening of ME/CFS symptoms that always follows physical and often cognitive activities". PEM is considered a cardinal symptom by a number of the different diagnostic criteria for ME/CFS and Post COVID Syndrome, including the CCC and International Consensus Criteria (ICC). In one embodiment, the effectiveness and physiological response of the treatment, as described herein, is determined by a decreased PEM score value compared to said value determined before treatment initiation.

COMPASS-31: "Composite Autonomic Symptom Score-31" ("COMPASS-31") is also used in ME/CFS and Post COVID Syndrome diagnostics and treatment response measures and used to score the severity of autonomic symptoms a (Sletten DM, et al, (2012) COMPASS 31: A refined and abbreviated Composite Autonomic Symptom Score. Mayo Clinic Proceedings 87:1196-120; Nacul L, et al, European Network on Myalgic Encephalomyelitis/Chronic Fatigue Syndrome (EUROMENE): Expert Consensus on the Diagnosis, Service Provision, and Care of People with ME/CFS in Europe. Medicina (2021) 57(5):510). In one embodiment, the COMPASS-31 score value in ME/CFS and Post COVID Syndrome patients can be 0, > 5, > 10, > 15, > 20, > 25, > 30, > 35, > 40, > 45, > 50, > 55, > 60, > 65, > 70, > 75, > 80, > 85, > 90, > 95 or 100. In one embodiment, the effectiveness and physiological response of the treatment, as described herein, is determined by a decreased COMPASS-31 score value compared to said value determined before treatment initiation.

SDM: "Symbol Digit Modalities Test" (SDMT) is further used in ME/CFS and Post COVID Syndrome diagnostics and treatment response measures and assesses cognitive function, particularly to detect cognitive impairment. SDMT can be used to assess changes in cognitive function over time. SDMT is a validated and established measure of cognition in multiple sclerosis capturing impairments such as processing speed and working memory, visual search and scanning and oculomotor functioning. A human subject is asked to report verbally the number that corresponds with each symbol provided by a sequential assortment at the top of the test page. The dependent variable is the total number correct in 90 s. (Strober LB, et al, A new look at an old test: Normative data of the symbol digit modalities test - Oral version Multiple Sclerosis and Related Disorders Volume 43, August 2020, 102154). In one embodiment, the effectiveness and physiological response of the treatment, as described herein, is determined by an increased SDMT scale value compared to said value determined before treatment initiation.

Heart Rate (HR) and Heart rate variability (HRV): HRV is further used in ME/CFS and Post COVID Syndrome diagnostics to and is a measure of the variation in time between each heartbeat. This variation is controlled by the autonomic nervous system (ANS). Low Heart Rate Variability (HRV) is a sign for sympathetic overdrive. In most ME/CFS and Post COVID Syndrome patients, ANS is dysregulated by an overactivity of the sympathicus. HRV can be measured with an VNA Analysis Program of COMMIT, licensed as Medical Device. VNA Analysis Program of COMMIT system provides information on HR, HRV, stress index (SI; as index for sympathetic activity), and RMSSD (root mean square of successive differences, as index for parasympathetic activity) (Baevsky RM and Chernikova AG. Heart rate variability analysis: physiological foundations and main methods; Cardiometry; No.10 May 2017; p.66-76;). In one embodiment, the effectiveness and physiological response of the treatment, as described herein, is determined by a decreased HR, decreased HRV, and/or decreased SI value compared to said value determined before treatment initiation.

Passive standing test: Passive standing test (CDE NIH) can also be used in ME/CFS and Post COVID Syndrome diagnostics and treatment response measure. CDE NIH provides orthostatic tolerance values and can be performed based on CDE CRF module instruction F2791 (ME/CFS CDE Version 1.0). Blood pressure and heart rate will be assessed upon sitting and 0, 2, 5 and 10 minutes standing while leaning against the wall. In one embodiment, passive standing test is used for ME/CFS and Post COVID Syndrome diagnostics. In one embodiment, blood pressure is decreased in ME/CFS and Post COVID Syndrome patients. In one embodiment, heart rate is faster in ME/CFS and Post COVID Syndrome patients compared to heart rate in healthy subjects using the passive standing test. In one embodiment, heart rate regulation is decelerated in ME/CFS and Post COVID Syndrome patients. In one embodiment, the effectiveness and physiological response of the treatment, as described herein, is determined by a decreased HR, accelerated HRV, and/or decreased SI value compared to said value determined before treatment initiation.

Pain scale: Presence and pain level using a pain scale can further used in ME/CFS and Post COVID Syndrome diagnostics. The pain scale measures a patient's pain intensity, for example using a numerical one dimensional rating scale from 1 to 10, wherein 1 = no pain and 10 = excruciating pain. In one embodiment, the pain level of ME/CFS and Post COVID Syndrome patients can be 1 or 2 or 3 or 4 or 5 or 6 or 7 or 8 or 9 or 10. In one embodiment, the effectiveness and physiological response of the treatment, as described herein, is determined by a decreased pain scale value compared to said value determined before treatment initiation.

Cerebral Blood Flow: "Cerebral Blood Flow" may also be used in ME/CFS and Post COVID Syndrome diagnostics and treatment response measure. Arterial Spin Labeling (ASL) MRI may be used to noninvasively measure the cerebral tissue perfusion during rest. ASL MRI provides a quantitative physiological measure of brain perfusion, the cerebral blood flow (CBF; ml/100g/min). ASL MR images may be acquired according to established methods in the art. In one embodiment, the average CBF value is a value for chronic vascular dysfunction, preferably endothelial dysfunction, in subjects suffering from ME/CSF and/or Post COVID Syndrome compared to healthy subjects. In one embodiment, the average CBF value is decreased in subjects suffering from ME/CSF and/or Post COVID Syndrome compared to healthy subjects. In one embodiment, the effectiveness and physiological response of the treatment, as described herein, is determined by an increased average CBF value compared to said value determined before treatment initiation.

Reactive hyperemia index: Determining peripheral microvascular function can also be used in ME/CFS and Post COVID Syndrome diagnostics and treatment response measure. "Reactive hyperemia" (RH) is a well-established technique for noninvasive assessment of peripheral microvascular function. It is preferably used to predict cardiovascular

morbidity and mortality. In its simplest form, reactive hyperaemia represents the extent of limb reperfusion following a brief period of ischaemia triggered by arterial occlusion. In one embodiment, the RHI value is a value for chronic vascular dysfunction, preferably endothelial dysfunction, in subjects suffering from ME/CSF and/or Post COVID Syndrome compared to healthy subjects, such as RHI < 1.8. In one embodiment, the RHI value is decreased in subjects suffering from ME/CSF and/or Post COVID Syndrome compared to healthy subjects. In one embodiment, the effectiveness and physiological response of the treatment, as described herein, is determined by an increased RHI value compared to said value determined before treatment initiation.

Endothelial dysfunction

An "endothelial dysfunction" refers to a pathological state of an endothelium, for example a systemic pathological state. The endothelium is a single layer of squamous cells that lines the inner surface of blood and lymphatic vessels. The endothelium forms an interface between the circulating blood or lymph in the lumen and the rest of the vessel wall. Endothelial cells form the barrier between vessels and tissues and control the flow of substances and fluid into and out of a tissue. Endothelial cells that are in direct contact with blood are called vascular endothelial cells, while those that are in direct contact with lymph are called lymphatic endothelial cells. Vascular endothelial cells line the entire circulatory system, from the heart to the smallest capillaries. These cells have unique functions such as fluid filtration, e.g. in the glomerulus of the kidney, blood vessel tension, haemostasis, neutrophil recruitment and hormone trafficking. The endothelium of the inner surfaces of the heart chambers is called the endocardium. Dysfunction can lead to serious health problems throughout the body.

Endothelial dysfunction is a form of non-obstructive coronary artery disease (CAD) in which there is no blockage of the arteries of the heart, but the large blood vessels on the surface of the heart narrow instead of dilating. The endothelium not only functions as a semi-permeable membrane, but is also responsible for maintaining vascular tone and regulating oxidative stress by releasing mediators such as nitric oxide, prostacyclin and endothelin, and controlling local angiotensin II activity. Endothelial dysfunction is usually associated with decreased NO bioavailability due to impaired NO production by the endothelium. A feature of endothelial dysfunction is the inability of arteries and arterioles to dilate fully in response to an appropriate stimulus, such as exogenous nitroglycerin, which stimulates the release of vasodilators such as NO from the endothelium.

A non-invasive method of measuring endothelial dysfunction is percent flow-mediated dilation (FMD), which is measured by brachial artery ultrasound. Another non-invasive, FDA-approved device for measuring endothelial function that uses the Reactive Hyperemia Index (RHI) is Itamar Medical's EndoPAT. Based on previous studies and the manufacturers analysis instructions, in some embodiments ED is defined as RHI ≤1.8 (Itamar Medical Ltd., 2002), on other studies as ≤1.67. The automatic batch analysis of individual measurements can be performed using the manufacturers analysis version 3.1.2 (2.0). Evidence for endothelial dysfunction may be an RHI < 1.8 and/or Endothelin-1 level > 90 percentile of healthy/age/gender matched controls or muscle fatigue and/or pathological values of the OCTA/dynamic vessel analyser. In one embodiment, endothelial function is impaired in subjects suffering from ME/CSF and/or Post COVID Syndrome compared to healthy subjects. In one embodiment, EndoPAT is used for measuring endothelial function that uses the Reactive Hyperemia Index (RHI). In one embodiment, the effectiveness and physiological response of the treatment, as described herein, is determined by an increased RHI value compared to said value determined before treatment initiation, wherein said RHI is determined using EndoPAT.

Cerebral blood flow may also be affected in subjects suffering from ME/CSF and/or Post COVID Syndrome. As surrogate for cerebral blood flow, retinal vessel density and/or perfusion can be determined. "Dynamic vessel analysis" (DVA) is a non-invasive method used to examine the condition and function of retinal vessels. This method quantifies the microvascular endothelial dysfunction by flicker light-induced dilatation (Wagner J, et al, Functional aging in health and heart failure: the COmPLETE Study. BMC Cardiovascular Disorderns (2019) 19:180, DOI 10.1186/s12872-019-1164-6).

"Optical coherence tomography angiography" (OCTA) is also a non-invasive imaging technique, which is able to acquire volumetric angiography images in a relatively short amount of time. In difference to conventional OCT, OCTA not only analyzes the intensity of the reflected light, but also the changes of OCT signal over time. By repeating OCT-scans at the same location, temporal signal changes are measured and blood flow of retina can be detected (De Carlo T E, Romano A, Waheed N K, Duker J S. A review of optical coherence tomography angiography (OCTA), International Journal of Retina and Vitreous (2015) 1:5).

In one embodiment, subjects suffering from ME/CSF and/or Post COVID Syndrome may present a retinal endothelial dysfunction compared to healthy subjects. In one embodiment, retinal endothelial function is associated with foveal avascularized zones, macular perfusion, ONH perfusion, macula vessel density, peripapillary flow, and/or peripapillary vessel density in ME/CSF and/or Post COVID Syndrome patients. In one embodiment, subjects suffering from ME/CSF and/or Post COVID Syndrome present enlarged foveal avascularized zones, decreased macular perfusion, decreased ONH perfusion, reduced macula vessel density, reduced peripapillary flow, and/or reduced peripapillary

vessel density. In one embodiment, OCTA and/or DVA are used for measuring retinal endothelial function and retinal vessel density. In one embodiment, the effectiveness and physiological response of the treatment, as described herein, is determined by a smaller sized avascularized zone, increased macular perfusion, increased ONH perfusion, increased macula vessel density, increased peripapillary flow, and/or increased peripapillary vessel density value compared to said value determined before treatment initiation, wherein said values are determined using OCTA and/or DVA.

Viral infection and viral load:

As used herein, the term "viral infection" describes a disease state in which a virus invades healthy cells. Viruses use the cell's reproductive machinery to multiply or replicate and finally dissolve the cell, leading to cell death, the release of viral particles and infection of other cells by the newly produced progeny viruses. The term "viral load" refers to a quantitative measure of viral genomes per invaded cell. The determination of viral genomic material may be employed in such a method. A latent infection by certain viruses is also a possible consequence of a viral infection. The term "viral growth" relates to the infection and replication of a virus and the production of viral particles during and after the infection of a host cell.

Detection of virus, bacteria and/or fungus

Infectious agents can be detected in suitable test samples. Examples of samples include swabs from wounds, stool samples, body fluids such as urine, cerebrospinal fluid, secretions (secretions) from the respiratory tract, and a special form of blood sample (blood cultures). A skilled person is capable of finding a method for detecting an infectious agent of interest without any effort. Typical methods detecting an infectious agent comprise PCR, quantitative PCR, ELISA, FACS, whole genome squencing, whole transcriptome sequencing, microscopic examination of specimens with special stains, antigen detection, western blots, or microbiological differential smear. In one embodiment, an infectious agent can be detected in a sample obtained from a ME/CFS patient. A frequently recurrent HSV-1, HSV-2 or VZV infection can be diagnosed clinically. If there is a history of tick infections, a Borrelia ELISA and, if positive, a complementary Western blot should be performed. Suitable diagnostic approaches are known to one skilled in the art.

Guideline - Recommendations for Post COVID Syndrome or ME/CFS patients

The term "guideline treatment for severe respiratory disease" encompasses any treatment, either via administration of a substance or other treatment, included in a standardized protocol for treatment of the indicated disease. For example, guideline treatment for chronic fatigue, e.g. ME/CFS and Post COVID Syndrome, encompasses those treatments provided in the "Drug therapeutic intervention and vaccination" or "Therapeutic options" or "Drug therapy" as published by the AWMF, dated 12.07.2021. Further examples of "guideline treatments for Post COVID Syndrome" or "guideline treatments for ME/CFS" are those described below, in the context of terms "guideline drug", "drug recommended by the guidelines", "non-sGC stimulator guideline drug", and "non-sGC activator guideline drug".

The terms "guideline drug", "drug recommended by the guidelines", "non-sGC stimulator guideline drug", and "non-sGC activator guideline drug", as used herein, refers to any drug therapy recommended in S1, S2k or S3 guideline.

A guideline is known to be modified and adapted to the current state of medical and scientific knowledge. The term "guideline drug" also comprises any prospective recommendations, provided they do not include a sGC stimulator or sGC activator. Preferably, the guideline is used for the treatment of Post COVID Syndrome or ME/CFS patients. However, other countries and nationalities published guidelines, protocols, data collection, and other measures put in place at the national and regional levels to address post-COVID conditions (such as Denmark, France, Portugal). Their drug therapy recommendations may be included herein.

Guidelines for the treatment of Post COVID Syndrome or ME/CFS patients comprise S3 guideline "Fatigue" DEGAM Guideline No. 2 (AWMF register no. 053-002) or S1 Guideline Post-COVID/Long-COVID (AWMF Register No. 020/027).

Treatment

The treatment of the present invention may be employed or administered as deemed suitable by a skilled practitioner. For example, in some embodiments, a time interval between treatments comprises a period of several hours (2, 3, 4, 5, 6, 7, 8, 9, 10, 11 or 12) several days (2, 3, 4, 5, 6, or 7), several weeks (1, 2, 3, 4, 5, 6, 7, or 8), or months (2, 3, 4, 5, or 6), preferably 1 day. In some embodiments, the time interval from one treatment to the next subsequent treatment can be the same, nearly the same or can change. In some embodiments, the treatment period in which the sGC stimulator or salt thereof, preferably vericiguat, and/or the drug recommended by one or more guidelines are administered to the subject can be >1 day, >2 days, >3 days, >4 days, >5 days, >6 days, >7 days, >8 days, >9 days,

>10 days, >11 days, >12 days, >13 days, >2 weeks, >3 weeks, >1 month, > 2 months, > 3months, > 4 months, > 6 months, preferably 10 weeks.

Subject

As used herein, the term "subject" refers to a mammal, such as humans, but can also be another animal, such as a domestic animal (e.g. a dog, cat or the like), a farm animal (e.g. a cow, sheep, pig, horse or the like) or a laboratory animal (e.g. a monkey, rat, mouse, rabbit, guinea pig or the like). The term "patient" refers to a "subject" suffering from or suspected of suffering from ME/CSF or Post COVID Syndrome comprising a chronic vascular dysfunction.
In one embodiment, treatment criteria comprise a positively diagnosed Post COVID Syndrome by mild to moderate acute COVID-19 course, non-hospitalized, ME/CFS CCC criteria with PEM > 14 hours, Bell Score: 30-60, symptom persistence ≥ 6month, evidence for endothelial dysfunction
(as indicated by RHI < 1.8 and/or Endothelin-1 level > 90 percentile of healthy/age/gender matched controls or muscle fatigue (below cut-off values of AUC reference values for age-matched healthy controls), pathological OCTA/dynamic vessel analyser, normal thyroid function, and/or laboratory or clinically confirmed SARS-CoV-2 infection.
In one embodiment, treatment criteria comprise a positively diagnosed Post COVID Syndrome by mild to moderate acute COVID-19 course, non-hospitalized, ME/CFS CCC criteria with PEM 2 - 14 hours, Bell Score: 30-60, symptom persistence ≥ 6month, evidence for endothelial dysfunction (as indicated by RHI < 1.8 and/or Endothelin-1 level > 90 percentile of healthy/age/gender matched controls or muscle fatigue (below cut-off values of AUC reference values for age-matched healthy controls), pathological OCTA/dynamic vessel analyser, normal thyroid function, and/or laboratory or clinically confirmed SARS-CoV-2 infection.
In one embodiment, treatment criteria comprise a positively diagnosed ME/CFS by mild to moderate acute COVID-19 course, ME/CFS CCC criteria with PEM > 14 hours, Bell Score: 30-60, symptom persistence ≥ 6month, evidence for endothelial dysfunction (as indicated by RHI < 1.8 and/or Endothelin-1 level > 90 percentile of healthy/age/gender matched controls or muscle fatigue (below cut-off values of AUC reference values for age-matched healthy controls), pathological OCTA/dynamic vessel analyser, normal thyroid function, and/or laboratory or clinically confirmed infection.
In one embodiment, treatment criteria comprise a positively diagnosed ME/CSF or Post COVID Syndrome by ME/CFS CCC criteria with PEM > 14 hours or PEM 2-14 hours, Bell Score: 30-60, symptom persistence ≥ 6month, evidence for endothelial dysfunction (as indicated by RHI < 1.8 and/or Endothelin-1 level > 90 percentile of healthy/age/gender matched controls or muscle fatigue (below cut-off values of AUC reference values for age-matched healthy controls), pathological OCTA/dynamic vessel analyser, normal thyroid function, with no laboratory or clinically confirmed infection.

Host cell and target cell

The term "host cell" and "target cell", as used herein, refers to a single cell or cell culture that may be or has been a recipient of at least one of the agents described herein, individually or in combination. Host cells include progeny of a single host cell, which progeny may not necessarily be completely identical (in morphology or overall DNA complement) to the original parent cell due to natural, random or intentional mutations and/or changes. In one embodiment, the host cell also refers to a cell into which an infectious agent (e.g. a virus) has invaded or is capable of invading.

Combined administration:

According to the present invention, the term "combined administration", otherwise known as coadministration or joint treatment, encompasses in some embodiments the administration of separate formulations of the compounds described herein, whereby treatment may occur together, within minutes of each other, in the same hour, on the same day, in the same week or in the same month as one another. Alternating administration of two agents is considered as one embodiment of combined administration. Staggered administration is encompassed by the term combined administration, whereby one agent may be administered, followed by the later administration of a second agent, optionally followed by administration of the first agent, again, and so forth. Simultaneous administration of multiple agents is considered as one embodiment of combined administration. Simultaneous administration encompasses in some embodiments, for example the taking of multiple compositions comprising the multiple agents at the same time, e.g. orally by ingesting separate tablets simultaneously. A combination medicament, such as a single formulation comprising multiple agents disclosed herein, and optionally additional anti-viral and/or anti-inflammatory or autoantibody-targeting medicaments, may also be used in order to coadminister the various components in a single administration or dosage.
A combined therapy or combined administration of one agent may occur together or precede or follow treatment with the other agent to be combined, by intervals ranging from minutes to weeks. In embodiments where the second

agent and the first agent are administered separately, one would generally ensure that a significant period of time did not expire between the time of each delivery, such that the first and second agents would still be able to exert an advantageously combined synergistic effect on a treatment site. In such instances, it is contemplated that one would contact the subject with both modalities within about 12-24 h of each other and, more preferably, within about 3-12 h of each other, with a delay time of only about 6 h being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

In the meaning of the invention, any form of administration of the multiple agents described herein is encompassed by combined administration, such that a beneficial additional therapeutic effect, preferably a synergistic effect, is achieved through the combined administration of the two agents.

Pharmaceutically acceptable salts, pharmaceutical compositions and methods of administration

In some embodiments, the sGC stimulator or sGC activator may be provided as (i) the compound itself (e.g., as a free base); (ii) a pharmaceutically acceptable salt of the compound; or (iii) part of a pharmaceutical composition. In some embodiments of the above methods, uses and pharmaceutical compositions, the additional therapeutic means may be provided as (i) the compound itself (e.g. as a free base); (ii) a pharmaceutically acceptable salt of the compound; (iii) or as part of a pharmaceutical composition.

"Pharmaceutically acceptable salts" of the compounds described herein include those resulting from said compounds when mixed with inorganic or organic acids or bases. In some embodiments, the salts may be produced in situ during the final isolation and purification of the compounds. In other embodiments, the salts may be produced from the free form of the compound in a separate synthesis step. The preparation of the pharmaceutically acceptable salts described above and other typical pharmaceutically acceptable salts is described in more detail in Berg et ah, "Pharmaceutical Salts", J. Pharm. ScL, 1977:66:1-19, which is incorporated herein by reference in its entirety. The pharmaceutically acceptable salts of an SGC stimulator are those that can be used in medicine. However, salts that are not pharmaceutically acceptable may be useful in producing an sGC stimulator or their pharmaceutically acceptable salts.

For acidic sGC stimulators and sGC activators, suitable "pharmaceutically acceptable salts" refer to salts produced from pharmaceutically acceptable non-toxic bases, including inorganic and organic bases. Salts derived from inorganic bases include aluminium, ammonium, calcium, copper, ferric iron, ferrous iron, lithium, magnesium, manganese, manganese(II), potassium, sodium, zinc and the like. Specific embodiments include ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines, arginine, betaine, caffeine, choline, N,N'dibcnzylcthylcncdiaminc, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

For basic sGC stimulators and sGC activators, salts can be produced from pharmaceutically acceptable non-toxic acids, including inorganic and organic acids. These acids include acetate, acetic acid, acid citrate, acid phosphate, ascorbate, benzenesulfonate, benzenesulfonate, benzoic acid, benzoate, bromide, bisulfate, bitartrate, camphorsulfonic acid, chloride, citrate, citric acid, ethanesulfonate, ethanesulfonic acid, formate, fumarate, fumaric acid, gentisinate, gluconate, gluconic, glucuronate, glutamate, glutamic, hydrobromic, hydrochloric, iodide, isethionic, isonicotinate, lactate, lactic, maleate, maleic, malic, mandelic, methanesulfonic, methanesulfonate, mucic, nitrate, nitric, oleate, oxalate, pamoic, pamoate (i. e., 1,1'-methylenebis-(2-hydroxy-3-naphthoate)), pantothenic acid, pantothenate, phosphate, phosphoric acid, saccharate, salicylate, succinic acid, succinate, sulfuric acid, sulfate, tannate, tartrate, tartaric acid, p-toluenesulfonate, p-toluenesulfonic acid and the like. Particular embodiments include citric, bromic, hydrochloric, maleic, phosphoric, sulphuric and tartaric acids.

In addition to a sGC stimulator and a sGC activator, their pharmaceutically acceptable salts may also be used in compositions for treating or preventing the chronic vascular dysfunction described herein.

The present invention also relates to a pharmaceutical composition comprising the compounds described herein. The invention also relates to pharmaceutically acceptable salts of the compounds described herein, in addition to enantiomers and/or tautomers of the compounds described.

The term "pharmaceutical composition" refers to a combination of the agent as described herein with a pharmaceutically acceptable carrier. The phrase "pharmaceutically-acceptable" refers to molecular entities and compositions that do not produce a severe allergic or similar untoward reaction when administered to a human. As used herein, "carrier" or "carrier substance" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Supplementary

active ingredients can also be incorporated into the compositions.

The pharmaceutical composition containing the active ingredient may be in a form suitable for oral use, for example, as tablets, chewing tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions. Tablets contain the active ingredient in admixture with non-toxic pharmaceutically acceptable excipients which are suitable for the manufacture of tablets. The tablets may be uncoated or they may be coated by known techniques to delay disintegration and absorption in the gastrointestinal tract and thereby provide a sustained action over a longer period.

In one embodiment, the pharmaceutical composition comprising the sGC stimulator, preferably vericiguat or salt thereof, described herein is supplied to the subject once daily in a form suitable for oral use, preferably 1-10 mg vericiguat per single dose, preferably administered as tablet that is swallowed by the subject.

Administration of the compounds of the invention, or their pharmaceutically acceptable salts, in pure form or in an appropriate pharmaceutical composition, can be carried out via any of the accepted modes of administration or agents for serving similar utilities. Thus, administration can be, for example, orally, nasally, parenterally, topically, transdermally, or rectally, sublingually, intramuscular, subcutaneously, or intravenously in the form of solid, semi-solid, lyophilized powder, or liquid dosage forms, such as for example, tablets, suppositories, pills, soft elastic and hard gelatin capsules, powders, solutions, suspensions, or aerosols, or the like, preferably in unit dosage forms suitable for simple administration of precise dosages. The compositions will include a conventional pharmaceutical carrier or excipient and a compound of the invention as the/an active agent, and, in addition, may include other medicinal agents, pharmaceutical agents, carriers, adjuvants, etc.

Dosage levels of the order of from about 0.01 mg to about 50mg per kilogram of body weight per day are useful in the treatment of the indicated conditions. For example, an endothelial dysfunction, hyper-perfusion and/or vascular constriction may be effectively treated by the administration of from about 0.1 mg to about 500mg, preferably 0.5 mg to about 100 mg, more preferably 1 mg to about 10 mg, of the sGC stimulator, preferably vericiguat, per patient per day.

Dosage levels of the order of from about 0.01 mg to about 500 mg per kilogram of body weight per day are useful in the treatment of the indicated conditions with a drug recommended by the guidelines for ME/CSF patients and/or guidelines for Post COVID Syndrome patients, preferably nutritional supplements, immune modulators and/ or pain relievers, more preferably omega-3 fatty acids, ferritin, vitamin D, vitamin B, alpha interferon, ribavirin, combination of alpha and gamma interferon, acetaminophen, aspirin, and/or ibuprofen. For example, fatigue, pain or muscle weakness may be effectively treated by the administration of from about 0.001 mg to about 1 g, preferably 0.005mg to about 500 mg, more preferably 0.01 mg to about 400 mg with a drug recommended by the guidelines for ME/CSF patients and/or guidelines for Post COVID Syndrome patients, preferably nutritional supplements, immune modulators and/ or pain relievers, more preferably omega-3 fatty acids, ferritin, vitamin D, vitamin B, alpha interferon, ribavirin, combination of alpha and gamma interferon, acetaminophen, aspirin, and/or ibuprofen, per patient per day. A skilled person knows the correct dosage information from the stand technique or from the recommendations for the treatment of ME/CSF and Post COVID Syndrome patients.

Another example, endothelial dysfunction, hypoperfusion and/or vascular constriction may be effectively treated by the administration of from about 0.1 mg to about 500mg, preferably 0.5 mg to about 100 mg, more preferably 1 mg to about 10 mg, of the sGC stimulator, preferably vericiguat, per patient per day, combined with a drug recommended by the guidelines for guidelines for ME/CSF patients and/or guidelines for Post COVID Syndrome patients, preferably nutritional supplements, immune modulators and/ or pain relievers, more preferably omega-3 fatty acids, ferritin, vitamin D, vitamin B, alpha interferon, ribavirin, combination of alpha and gamma interferon, acetaminophen, aspirin, and/or ibuprofen.

The amount of active ingredient that may be combined with the carrier materials to produce a single dosage form will vary depending upon the host treated and the particular mode of administration. For example, a formulation intended for the oral administration of humans may vary from about 5 to about 95% of the total composition. Dosage unit forms will generally contain between from about 0.001 mg to about 500 mg of active ingredient. It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy. The dosage effective amount of compounds according to the invention will vary depending upon factors including the particular compound, toxicity, and inhibitory activity, the condition treated, and whether the compound is administered alone or with other therapies.

## FIGURES

The invention is further described by the figures. These are not intended to limit the scope of the invention.

Short description of the figures:

**Figure 1: Severity of fatigue and disability**

**Figure 2: Frequency, severity and length of post exertional malaise (PEM)**

**Figure 3: Hand grip strength (HGS)**

**Figure 4: Sitting and standing heart rate and blood pressure**

**Figure 5: Correlation of HGS with laboratory parameter**

**Figure 6: Endothelial dysfunction (ED) assessed by RH-PAT**

**Figure 7: Serum ET-1 concentrations**

**Figure 8: Serum Ang-2 level**

Detailed description of the figures:

**Figure 1: Severity of fatigue and disability.** Bell disability scale (score 0-100), CFQ = Chalder fatigue scale (score 0-33), and SF-36 physical function, vitality, role limitations, and social function (scores 0-100) were assessed in PCS cohorts and non-COVID-ME/CFS by questionnaires. Data was analyzed with nonparametric all-pairs Dunn-type multiple contrast tests (Bell, Chalder, SF-36 physical functioning) and Brunner-Munzel tests. The pvalues were adjusted for multiplicity across endpoints with the Benjamini-Hochberg (BH) correction.

**Figure 2: Frequency, severity and length of post exertional malaise (PEM).** Frequency and severity of PEM was assessed on a five items scale with 0 - 20 points (none to severest) and the length in seven categories (from <1h to 2 - 3 days), according to Cotler 9. Median plus range in 19 PCS/CFS, 23 PCS, and 17 non COVID ME/CFS patients is shown. n=22 Data was analyzed with nonparametric all-pairs Dunn-type multiple contrast tests (Bell, Chalder, SF-36 physical functioning). The p-values were adjusted for multiplicity across endpoints with the Benjamini-Hochberg (BH) correction.

**Figure 3: Hand grip strength (HGS).** Hand grip strength (HGS) was assessed in PCS cohorts and non-COVID-ME/CFS. Fmax1 and Fmean1 of ten pulls in female patients and repeat assessment after 60 minutes (Fmax2 and Fmean2 of ten pulls) are shown (median, range). Cut-off values of AUC reference values for age-matched healthy females are displayed as dashed lines: <40 years black dots and narrower dashed lines; >40 years white dots and wider dashed lines. Sitting and standing heart rate and blood pressure. n=13 Data was analyzed with nonparametric all-pairs Dunn-type multiple contrast tests. The p-values were adjusted for multiplicity across endpoints with the Benjamini-Hochberg (BH) correction.

**Figure 4: Sitting and standing heart rate and blood pressure.** Heart rate and systolic and diastolic BP (blood pressure) sitting, standing, after two, five, and ten minutes standing in female PCS/non ME/CFS and PCS/ME/CFS females. Full dots represent POTS and orthostatic hypotension.

**Figure 5: Correlation of HGS with laboratory parameter.** Correlation of hand grip strength (HGS) parameter Fmax1, Fmean1, Fmax2 and Fmean2 with laboratory parameters of relevance for oxygen supply, inflammation and vasoregulation. We analysed the data within a correlation analysis using spearman's $\rho$ and Benjamini-Hochberg correction for multiplicity. Correlations that were significant are indicated by blue (positive) or red (negative).

**Figure 6: Endothelial dysfunction (ED) assessed by RH-PAT.** ED was found in five of 14 ME/CFS and in five of 16 PCS patients but not in healthy controls (HC). The RHI value for each patient is plotted. The dotted line indicates the RHI cut-off value of $\leq$1.67 defining ED. [RHI = reactive hyperaemia index; RH-PAT = reactive hyperaemia peripheral arterial tonometry] **Figure 7: Serum ET-1 concentrations.** Serum ET-1 concentration was measured in the PAT study cohort (a) and in a second validation cohort (b). Median (IQR) serum ET-1 concentration is shown. For statistical analysis Kruskal-Wallis with Dunn's post-hoc multiple comparisons test was used. P values <0.1 are plotted and p values $\leq$ 0.05 were considered statistically significant. [ET-1 = Endothelin-1; IQR = interquartile range; RH-PAT = reactive hyperaemia peripheral arterial tonometry]

**Figure 8: Serum Ang-2 level.** Serum Ang-2 levels were measured in the PAT study cohort (a) and in a second

validation cohort (b). Levels are depicted as median (IQR) of fold change (FC) to HC as standard was 1.5 fold higher in the ELISA of the 2nd compared to the 1st cohort. For statistical analysis Kruskal-Wallis with Dunn's post-hoc multiple comparisons test were used. P values <0.1 are plotted and p values ≤ 0.05 were considered statistically significant. [Ang-2 = Angiopoietin-2; IQR = interquartile range; RH-PAT = reactive hyperaemia peripheral arterial tonometry]

## EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

Described in more detail below is:

- Clinical trial protocol
- Methods, study design, clinical parameter of patient cohorts analyzed
- Laboratory parameter and biomarkers correlating with symptoms in ME/CSF and/or Post COVID Syndrome patients
- Endothelial dysfunction present in two patient cohorts

### Example 1: Clinical trial protocol for vericiguat in ME/CSF and Post COVID Syndrome patients

About every 10th person who had mild or moderate COVID-19 not requiring hospitalization has persistent symptoms after 6 months referred to as Long or Post COVID (Davis 2021). Predominantly healthy young and middle-aged adults with female preponderance are affected exhibiting a diverse spectrum of symptoms most without detectable organ damage. A parallel-group treatment, phase 2a, double-blind, placebo-controlled 2-arm study to show improvement in physical function in SF-36 in participants treated with Vericiguat compared to placebo tablets in male and female participants aged 18-50 years with Post COVID-19 Syndrome without (PCS) or with (PCS/CFS) fulfillment of ME/CFS criteria.

### Objectives and endpoints

The purpose of this study is to show improvement in SF-36 with Vericiguat compared with Placebo in Long COVID participants with PCS/PCS-CFS.

The pathomechanism of Long-COVID is not well understood yet and probably not uniform. Most PCS patients had severely diminished hand grip strength (HGS) and enhanced muscle fatiguability. In preclinical endothelial dysfunction (ED) models and in heart failure (HF) patients, it was shown that the sGC stimulator Vericiguat compensates ED-associated NO deficiency and restores vascular function.

Administration of Vericiguat in PCS / CFS and PCS patients may bridge the ED via direct sGC stimulation restores microvascular function and thus improves the health status.

Primary endpoints include intra-patient change in SF-36-PF from baseline to week 10. The SF-36-PF is a total score (ranging from 0 to 100) and computed from 10 physical functioning items. The higher the score, the better the clinical record (100=excellent). Scaling is interpreted as metric.

Other clinical objectives (secondary objectives):
In addition to the primary objective and endpoint, other clinical parameters are collected and documented for a more detailed assessment of the clinical course under vericiguat or placebo treatment. An improvement in secondary endpoints refers to an intra-patient change from baseline to week 10 and is analyzed via summary statistics in total and by study arm; the mean difference between both groups is provided. The tables providing the summary statistics and mean differences is presented overall as well as by diagnosis (PCS vs PCS/CFS).

Additional clinical endpoints (secondary endpoints) comprise difference in responder rate in PF in SF-36-PF (20 point increase) at week 10 comparing Vericiguat with placebo, improvement in Chalder Fatigue Score based on change from baseline to week 10 comparing vericiguat with placebo based on mean differences, improvement in severity of muscle pain and headache assessed by CCC Symptom Score, improvement in muscle fatigue test (repetitive hand grip strength (HGS) test), improvement in other SF-36 sub-domains from baseline to week 10 when comparing Vericiguat with placebo based on mean differences, improvement in functional disability [Bell disabling scale], improvement in disease severity based on self-reported symptoms [MBSQ], improvement in post exertional malaise (PEM) frequency, strength and severity in total and subcategories, improvement in symptom severity (CCC Symptom Scores), improvement

in COMPASS-31 (autonomic dysfunction), improvement in cognitive function (SDMT), improvement in Blood pressure (BP), Heart Rate (HR) regulation in passive standing test [CDE NIH], improvement in HR-Variability (HRV) [vnsanalyse© system; COMMIT GmbH], improvement in endothelial dysfunction [EndoPAT®], improvement in retinal endothelial function (OCTA and dynamic vessel analyzer), improvement in endothelin-1 (ET-1) serum levels.

The Chalder Fatigue score is a total score resulting from 11 items on an ordinal 0 to 3 scale, which are summed to a total score (range 0-33; 33 denotes maximal symptoms) and two subscales: physical fatigue (seven items, range 0-21) and mental fatigue (four items, range 0-12). Conventionally, fatigue case-status (fatigued vs. non-fatigued) is defined using this scale with a cut-off at <4 vs. ≥4. Scaling of the total score is interpreted as metric. The Chalder Fatigue total score at baseline and week 10 is described via summary statistics in total and by study arm. The intra-patient change from baseline to week 10 is analyzed via summary statistics in total and by study arm; the mean difference between both groups will be provided. The tables providing the summary statistics and mean differences is presented overall as well as by diagnosis.

The severity of muscle pain and headache is assessed by CCC score based on a Likert scale (1=no symptoms up to 104 = severest symptoms). Scaling is interpreted as metric.

CCC Symptom total score at baseline and week 10 is described via summary statistics in total and by study arm. The intra-patient change from baseline will be analyzed via summary statistics in total and by study arm; the mean difference between both groups will be provided. The tables providing the summary statistics and mean differences is presented overall as well as by diagnosis.

Muscle fatigue is monitored by (repeated) hand grip strength tests as objective measures for muscular strength and measured as Fmax, Fmean, fatigue ratio (=Fmax/Fmean), recovery rate and related computations. Measures at baseline and week 10 is described via summary statistics in total and by study arm. The intra-patient change from baseline is analyzed via summary statistics in total and by study arm; the mean difference between both groups is provided. The tables providing the summary statistics and mean differences is presented overall as well as by diagnosis.

Optional clinical endpoints comprise changes in cerebral blood flow by mean difference of arterial spin labeling (ASL).

Besides the careful and intensive clinical monitoring, additional scientific analyses are performed to define the pharmacokinetics of the vericiguat as tablet in PCS/PCS-CFS patient and to better understand the sGC stimulatory effect of vericiguat.

Study design: The clinical phase II trial is conducted in compliance with this protocol, Good Clinical Practice (GCP) guidelines, and all applicable regulatory requirements. The trial starts with approval by the relevant Institutional Review Board (IRB; ethics vote) and competent authority (CA). The overall design of the study is:

- 2-arm parallel-group, randomized, placebo-controlled, double-blind, single center study in participants with PCS/PCS-CFS.

- Total duration of study participation for each participant: up to 28 days screening phase, randomization, 10 weeks of treatment with Vericiguat (including titration phase) or Placebo, followed by 30 days Follow-up period.

- The effectiveness of Vericiguat for the treatment of PCS patients with proven ED is investigated. Subjects are assigned randomly in a 1:1 ratio to Vericiguat or matching placebo and stratified according to diagnosis and sex.

- The active substance is synthesized by Bayer AG under GMP conditions and made available to the study center as tablets as a verum preparation together with a corresponding placebo preparation.

- Vericiguat, a novel oral soluble guanylate cyclase stimulator that enhances the cyclic guanosine monophosphate (GMP) pathway by directly stimulating soluble guanylate cyclase (sGC) through a binding site independent of nitric oxide, and it sensitizes sGC to endogenous nitric oxide by stabilizing nitric oxide binding to the binding site has been developed for once-daily treatment of reduction in CV-death and HF hospitalizations in patients with reduced ejection fraction.

The patients included in the study are examined by patient questionnaires, which include symptoms, activity and cognitive function and objective measurements on day 1 and follow-up.

Oral treatment takes place on day 1 (start of therapy), whereby the dose is increased in a controlled and tolerated manner:

- first 2 weeks 2.5 mg / day,
- then dose increase for 2 weeks with 5.0 mg / day,
- then the target dose of 10 mg / day for 6 weeks.

After end of treatment, the patients are observed for additional 30 days.

In order to be able to describe mechanistically the effect of Vericiguat in the patients, accompanying extensive quantitative examinations and accompanying analyzes of blood samples (cardiac, vascular, immunological and inflammatory biomarker measurements) are carried out.

Scientific Rationale for Study Design

The study design was chosen to assess the effectiveness of Vericiguat in improvement in physical function of the SF-36 in patients with Chronic COVID-19 Syndrome of whom about half fulfills CCC diagnostic criteria for ME/CFS. The stepwise increase from 2.5 to 10 mg ensures tolerability and the duration of the treatment of 10 weeks ensures the achievement of steady state plasma concentrations. Placebo is used as control to evaluate safety, tolerability and efficacy and to account of any placebo bias in the study. In general, early clinical trials with molecular targeting agents should include the measurements of biomarkers in order to assess the pharmacodynamic effect of the molecular target and the relevant downstream components that may correlate with the pharmacological response. Therefore, several exploratory biomarker for endothelial dysfunction is part of the present study. An improvement in physical function (PF) in SF-36-PF in verum vs placebo arm is used as primary clinical endpoint. The SF-36 PF has been most frequently applied as primary endpoint in clinical studies on ME/CFS. A minimum of 10 point increase in the SF36-PF (range 0 - 100 = healthy) defines a clinical relevant improvement ("a little better") and an increase of 20 points defines a major clinical improvement ("much better"). Therefore, an increase of 20 points is defined as response and will be evaluated as secondary endpoint.

Patients aged 18 to 50 years are included in the study. Older patients will be excluded due to the increased prevalence of endothelial dysfunction beyond 50 years of age and to avoid a potential confounder as it will not be stratified for age. Participants are eligible to be included in the study only if all of the following ME/CFS CCC criteria apply:

1. Confirmed (PCR/serology), non-hospitalized, mild to moderate acute COVID-19 cases according to WHO criteria

2. Patients with PCS: either

- ME/CFS CCC criteria (Nacul) with PEM > 14 hours = PCS/CFS or

- ME/CFS CCC criteria with PEM 2 - 14 hours = PCS

3. Bell Score: 30-60

4. Ongoing symptoms since ≥ 6 months

5. Evidence for endothelial dysfunction (as indicated by RHI < 1.8 and/or Endothelin-1 level > 90 percentile of healthy/age/gender matched controls or muscle fatigue (below cut-off values of AUC reference values for age-matched healthy controls, Jaekel 2021) and/or pathological OCTA/dynamic vessel analyzer

6. Normal thyroid function

Pharmacokinetic Endpoints: PK assessments obtained from the data collected during this study are exploratory and is investigated under a separate detailed PK evaluation and analysis plan.

Planned Exploratory Biomarker Research: The exploratory biomarker analyses provide insights into the potential mechanisms of therapeutic effect in PCS and PCS/CFS beyond what is currently understood about how vericiguat affects cellular function. Endothelial biomarker serum endothelin-1, and RNA levels of bradykinin 1 receptor, β2 adrenergic receptor and arginase 1 are assessed. Endothelin-1 is the most important and potent vasoconstrictors and is produced by endothelial cells. The β2 adrenergic receptor plays a key role in mediating vasodilatation in muscle and cerebral vessels and is downregulated in ME/CFS (Light, and unpublished findings from C.S.). Bradykinin is released by ischemic muscles as a response to excessive vasoconstrictor and is also an important pain mediator assumed to cause angina pectoris pain in cardiac ischemia. As bradykinin has a very short half-live and is rapidly cleaved in serum it cannot be reliably measured. Therefore bradykinin receptor 1 is assessed as marker for activation of the bradykinin pathway.

Arginase is a key factor in endothelial dysfunction by mediating nitric oxide bioavailability and oxidative stress. Arginase is thought to modulate eNOS and this is most likely to occur when eNOS is translocated from the caveolae to the cytosol and perhaps even the mitochondria under proinflammatory states. In hypertension arginase activity and expression studies demonstrate increased arginase activity reduces NO mediated dilation in hypertensive pigs, which was then normalized using an arginase inhibitor.

**Study assessments**

Efficacy Assessments:

Symptom Assessment by Questionnaires: The diagnosis of ME/CFS is assessed based on the 2003 Canadian Consensus Criteria CCC in accordance with the European guidelines. Symptoms are assessed by questionnaires (e.g. SF-36 PF, Chalder Fatigue Score, CCC Symptom Score, Bell disabling scale, COMPASS-31).

Muscle fatigue test: Hand grip strength (HGS), as objective measure to assess muscular strength, is associated and is used as objective marker of the disease severity, PEM and muscle pain in ME/CFS patients.

We measure the HGS with a digital hand dynamometer (CAMRY, model: SCACAM-EH101) in two sessions with a recovery break of 60 minutes between the sessions. The participant has to sit in an upright position and place the forearm of the dominant hand on a standard table in full supination. Before the start of the measurement, all participants had the opportunity to pull the handle twice to become familiar with the device. The handle is pulled with maximum force for three seconds followed by a five second relaxation phase under supervision by the study nurse. Within one session, this procedure is repeated ten times with the dominant hand. After 60 minutes without any strenuous physical activity, a second session is conducted. Participants are verbally motivated during the measurement to continue using their maximum strength and to perform all repetitions. The dynamometer measures the highest value reached within the three seconds (force measurement in kg). The attempt with the highest measurement out of the ten repetitions was recorded as maximum strength. Strength, fatigability and recovery will be assessed as listed in table 4.

Table 4. Parameters of muscle strength assessed by handgrip measurements

| Parameter/Ratio | Formula | Explanation |
|---|---|---|
| Fmax | Fmax [kg] | maximum grip strength within one session (ten repeat trials) |
| Fmean | $\frac{\sum 10\text{ pulls}}{10}$ | mean grip strength of all ten trials |
| Fatigue Ratio (assessment of fatigability) | $\frac{\sum 10\text{ pulls}}{10}$ | higher values indicate stronger decrease of force during one session |
| Recovery Ratio (assessment of recoverability) | $\frac{\text{Fmean2}}{\text{Fmean1}}$ | low values indicate impaired recovery |

Cognitive testing: The Symbol Digit Modalities Test (SDMT, oral version) detects cognitive impairment in less than five minutes and is used in the study to assess change in cognitive function over time.

Hemodynamics: Passive Standing Test (CDE NIH) as measures of orthostatic tolerance is performed based on CDE CRF module instruction F2791 (ME/CFS CDE Version 1.0). Blood pressure and heart rate is assessed upon sitting and 0, 2, 5 and 10 minutes standing while leaning against the wall.

Assessement of the ANS: Heart rate variability (HRV) is a measure of the variation in time between each heartbeat. This variation is controlled by the autonomic nervous system (ANS). Low HRV is a sign for sympathetic overdrive. The HRV is measured with the VNA Analysis Program of COMMIT, licensed as Medical Device. The system provides information on HR, HRV, stress index (SI; as index for sympathetic activity), and RMSSD (root mean square of successive differences, as index for parasympathetic activity).

Assessment of endothelial function: Peripheral endothelial function is assessed using the EndoPAT2000 device. Endothelium-mediated changes in peripheral Arterial Tone (PAT) are recorded using plethysmographic probes on the index finger of each hand during reactive hyperaemia. Hyperaemia is induced by occlusion of the left brachial artery over five minutes using an inflatable blood pressure cuff. The RHI is calculated from the change in the pulse wave amplitude (PWA) relative to baseline in the occluded arm and corrected for corresponding changes in PWA relative to baseline in the contra-lateral, non-occluded arm in order to minimize the influence of non-endothelial dependent systemic effects using the equation: RHI = (A/B) ÷ (C/D). ED is defined as RHI ≤1.8. The automatic batch analysis of individual measurements is performed using the manufacturers analysis version 3.1.2 (2.0).

Retinal vessel analysis: Retinal endothelial function is assessed using the non-invasive dynamic vessel analyser (DVA, Imedos, Jena, Germany). DVA exposes the eye to flicker light and measures the diameter change of vessels as a function if time. This principle enables the functional examination of the vascular endothelium its flow-induced autoregulation. The maximal arteriolar (ADmax) and venular (VDmax) dilatation are analysed as well as the arteriolar (AFarea) and venular (VFarea) area under the flicker curve and the arteriolar-to-venular diameter ratio (AVR). Additionally to retinal vessel analysis, an ophthalmic screening is performed with assessment of refraction error, ocular blood pressure, fundoscopy, and optical coherence tomography.

Optical coherence tomography angiography (OCTA) is a non-invasive light-based high-resolution method to visualize retinal and choroidal microcirculation in 3D without dye injection. Vessel density may be reduced in post COVID-19 using OCTA. OCTA offers also a promising outcome parameters for therapy response. Vessel densities and perfusion (VD) of the superficial vascular plexus (SVP) and the area of the foveal avascularized zone (AVZ) are obtained from OCTA as markers of microvascular damage.

Analysis of cerebral blood flow: Arterial Spin Labeling (ASL)ASL MRI is used to noninvasively measure the cerebral tissue perfusion during rest as described herein.

Clinical scores used to assess (subjective) changes in symptoms are in Table 1. An overview of mechanistic measurements evaluating pharmacodynamic effects of Vericiguat is shown in Table 5.

Table 5: Mechanistic measurements used to assess (objective) changes in (micro-)vascular and organ/ tissue-related function(s) to evaluate the pharmacodynamic effects of Vericiguat

| | Use | Range | Read-outs | Device | Reference |
|---|---|---|---|---|---|
| EndoPAT-Endothelial Function Test | Endothelial function / dysfunction | RHI ≥ 1.8 (normal endothelial function) vs. RHI < 1.8 (endothelial dysfunction) | Reactive hyperemia index (RHI) | EndoPAT | Expected change: Recovery of reactive hyperemic response by 20 %. |
| Handgrip-Muscle-Fatigue Test | | | muscle strength (Fmax), muscle fatigue ratio Fmax/ Fmean), muscle recovery (ratio Fmean2/Fmean1) | | Expected change: Decrease in muscle fatigue and increase in muscle recovery by 10 %, each. |
| Orthostatic Intolerance Test | Explorative | Passive standing Test | | | Expected change: Improvement of tachycardia sitting and standing |
| HRV | Explorative | descriptive | SI, RMSSD | VNSAnalyse Commit | Expected change: Improvement of stress index (sympaticus acvitity) |
| | | | | | |
| Retinal Dynamic Vessel Analysis | Explorative; use of light indused change to assess endothelial function | AD-max: 0%-100% VD-max: 0%-100% AF-area: 0-100 VF-area: 0-100 AVR: 0-10 | arteriolar dilation (ADmax), venular dilation (VDmax), the arteriolar area under the flicker curve (AFarea), venular area under the flicker curve (VFarea), arteriolar-to-venular diameter ratio (AVR) | IMEDOS Dynamic vessel analyzer | Expected change: Improved response to light impulse |

(continued)

| | Use | Range | Read-outs | Device | Reference |
|---|---|---|---|---|---|
| Retinal optical coherence tomography angiography | Explorative; use of retinal vessel density and perfusion as surrogate for cerebral blood flow | Macular VD: 0-100 mm/mm2, Macular Perfusion: 0-100% ONH flow index: 0-1 ONH Perfusion: 0-100% FAZ area: 0-2 $mm^2$ | Vessel Densities (VD) Perfusion, FAZ area | Carl Zeiss Meditec Cirrus Angioplex OCTA | Expected change: Improvement of macula and peripapillary (ONH) vessel densities, smaller avascularized zone |
| OPTIONAL | | | | | |
| Cerebral Blood Flow (CBF) measured with | Explorative; | Unknown | Voxel-wise CBF in grey matter brain | ASL | Unknown |
| ASL (arterial spin labeling) | | | regions during rest | | |

Pharmacokinetics

Sample collection, storage, and shipment instructions for plasma samples are provided in the Operations/Laboratory Manual. Plasma samples of approximately 3,5 mL (7 x 500 µL) are collected for measurement of plasma concentrations of Vericiguat as specified in the SoA.

For the investigation of systemic exposure to Vericiguat and its relationship with treatment effects, the plasma concentrations of Vericiguat is determined at different time points using a sparse sampling approach in all participating subjects.

Blood samples is collected at the following timepoints:

1. Visits 2: 1-3 h and 4-6 h (towards the end of the visit) after drug intake (exact collection time must be documented).
2. Visit 3 and 4: baseline / trough (prior to drug intake) and 2-6 h (towards the end of the visit) after drug intake (exact collection time must be documented).
3. Visit 7: baseline / trough.

Biomarkers

In this study, non-genetic endothelial biomarkers are investigated. Serum ET-1 concentration is determined by QuantiGlo™ ELISA from R&D Systems, Inc. according to the manufacturers' protocols. Ang-2 is measured by Quantikine Human Angiopoietin-2 Immunoassay purchased from R&D Systems, Inc. Serum ACE2 level is determined using ELISA (Roche, Germany). Bradykinin receptor1, β2 adrenergic receptor and arginase 1 are determined by quantitative PCR.

**Example 2: Clinical parameter and Laboratory parameter of Post COVID Syndrome patients correlating with hand grip strength - first pandemic cohort**

A subset of patients has long-lasting symptoms after mild to moderate COVID- 19. In a prospective observational cohort study, we analysed clinical and laboratory parameters in 42 post COVID-19 syndrome (PCS) patients (29 female/13 male, median age 36.5 years) with persistent moderate to severe fatigue and exertion intolerance six months following COVID-19. Further an age- and sex-matched postinfectious non-COVID-19 myalgic encephalomyelitis/chronic fatigue syndrome (ME/CFS) cohort was comparatively evaluated. Most PCS patients were moderately to severely impaired in daily live. 19 PCS patients fulfilled the 2003 Canadian Consensus Criteria for ME/CFS (PCS/ME/CFS). Disease severity and symptom burden was similar in PCS/ME/CFS and non-COVID-19 ME/CFS patients. Hand grip strength (HGS) was diminished in most patients compared to normal values in healthy. Association of HGS with hemoglobin (Hb), IL-8 and CRP in PCS/non-ME/CFS and with Hb, NT-pro BNP, bilirubin, and ferritin in PCS/ME/CFS indicates low level inflammation

and hypoperfusion as potential pathomechanisms.

Methods:

Diagnostic criteria for ME/CFS and symptom assessment Severity of mental and physical fatigue was assessed using the Chalder Fatigue Scale (CFQ). The first seven questions assess mental fatigue (CFQA), the last four physical fatigue (CFQB). Disability and daily physical function were assessed by the Bell disability scale and Short Form Health Survey- 36 (SF-36 Version 1). The Bell disability scale is scored from 0 (very severe, bedridden constantly) to 100 (healthy). Frequency and severity of PEM symptoms were assessed. Symptoms of autonomic dysfunction were assessed by the Composite Autonomic Symptom Score (COMPASS 31). Depression and sleepiness were assessed by Patient Health Questionnaire 9 (PHQ9) and Epworth Sleepiness Scale (ESS). According to PHQ9, patients were classified as having minimal depressive symptoms (1-4), mild depressive symptoms, moderate depressive symptoms (10-14), moderately severe depressive symptoms (15-19), or severe depressive symptoms (20-27). According to ESS, patients were classified as no evidence of sleep apnea (0-9), possible mild to moderate sleep apnea (11-15), or possible severe sleep apnea (>16) of ME/CFS was based on Canadian Consensus Criteria (CCC) and exclusion of other diseases, which may be considered as potential confounding comorbidities serving as an alternative explanation for chronic fatigue18. In contrast to the original classification and in accordance with the studies of L. Jason and his team, a minimum of 14 hours of PEM instead of 24 hours was required for diagnosis of ME/CFS. In addition, key symptoms of CCC were quantified using a 1-10 scale to assess severity of symptoms. All data was recorded using the Research Electronic Data Capture (REDCap) database. Supplementary material shows complete questionnaires, criteria, and assessments with interpretation.

Functional studies, imaging and laboratory values: Hand grip strength (HGS) was assessed using an electric dynamometer assessing maximal and mean force of maximal pulls (Fmax1 and Fmean1) repeated ten times and a second assessment 60 minutes later (Fmax2 and Fmean2). Blood pressure and heart rate were assessed in sitting position as well as in standing position immediately after standing up and after two, five, and ten minutes. Postural tachycardia syndrome (POTS) is defined as increase of >30 bpm or over 120 bpm within ten minutes after standing up and signs of orthostatic intolerance. Postural hypotension is defined as a decrease of over 20mmHg of systolic or over 10mmHG in diastolic blood pressure. Laboratory parameters including full (CBC) and differential (DBC) blood count, lymphocyte subsets, IL-8 in erythrocytes, MBL, CRP, immunoglobulin subsets, ANA, ENA, C3/4, anti-TPO antibodies, TSH, fT3/4, ferritin, creatinine, liver enzymes, ACE1/2, NT-pro BNP were determined at the Charité diagnostics laboratory (Labor Berlin GmbH, Berlin, Germany). ACE2 was assessed by an enzyme-linked immunoassay (ELISA; R&D Systems).

Statistical analysis: For descriptive purpose, we illustrate the outcomes of all variables using median and range (if not indicated otherwise). Inferentially, we analyzed the data using purely nonparametric all-pairs Dunntype multiple contrast tests (accounting for variance heteroscedasticty). Two samples are compared with the Brunner-Munzel test. All methods used are ranking methods testing hypotheses formulated in terms of so-called relative effects (Wilcoxon-Mann-Whitney parameters). Effect estimators and their standard errors, test statistics and 95% simultaneous confidence intervals are provided in the supplementary material. Furthermore, since the number of endpoints and comparisons is pretty large (and to provide conservative estimates), we additionally adjust p-values for multiplicity across endpoints with the Benjamini-Hochberg (BH) correction. We performed all statistical computations using the statistical software package R using the R-packages nparcomp and the p.adjust function. Furthermore, we estimated Spearman's p and illustrate the results from correlation analysis in correlation plots using the R-package corrplot. The amount of missing values is very low and we therefore used all-available cases for data analysis in the correlation analysis. All results are interpreted in an exploratory manner at 5% level of significance (two-sided).

Demographic and baseline clinical characteristics: We report on a total of 42 patients who presented to the Charité Fatigue Center with PCS all suffering from persistent moderate to severe fatigue and exertion intolerance six months after diagnosis of COVID-19. Most patients had mild COVID-19 (n=32) and ten had moderate COVID-19 due to pneumonia, according to WHO criteria. Three of the 10 patients with pneumonia were treated in hospital but none of them required oxygen or mechanical ventilation thus making critical illness myopathy (CIM) unlikely to explain any symptoms. Ten most frequent initial symptoms of COVID-19 reported by the patients are fatigue, PEM, need for rest, impaired performance, stress intolerance, muscle pain, headache, concentration impairment, mental fatigue, and dizziness when standing up.

19 patients fulfilled the Canadian Consensus Criteria (CCC) for ME/CFS. These patients are referred to as post-COVID-19 Syndrome ME/CFS (PCS/ME/CFS), the other 23 patients are referred to as PCS/non-ME/CFS. Most PCS/non-ME/CFS patients (18 of 23) who did not fulfill ME/CFS criteria had exertion intolerance with a duration of PEM of less than 14 hours. Furthermore, eight of 23 patients did not fulfill the criteria for neurological/cognitive symptoms. Based on answers to the Patient Health Questionnaire 9 (PHQ9), we have no evidence of severe depression in our study cohort. In two patients with an Epworth Sleepiness Scale (ESS) score of >16 sleep apnea was excluded. From all postinfectious non-COVID-19 ME/CFS patients evaluated during the same period at our clinic (n=123) a sex- and

age-matched control cohort who had the shortest duration of illness (13 months, range 7 - 19 months, n = 19) was selected. Infectious trigger at disease onset are SARS-CoV-2 (n=42), respiratory tract, unspecified (n=9), EBV (n=3), gastroenteritis (n= 1), other infection (n=6). The majority of patients from both the PCS and non-COVID ME/CFS cohort was severely impaired in daily life with a median Bell disability score of 40 and 30 out of 100, respectively (Fig. 1). According to the Bell disability scale, patients with a score of 30-40 are able to perform light work 2-4 hours a day, thus requiring a reduced work schedule or are unable to work. Patients with a Bell disability score of 20 are confined to bed most of the day. PCS/non-ME/CFS patients had a median Bell Score of 50 and a higher SF-36 sub-score for role limitations compared to PCS/ME/CFS patients (Fig. 1). Twelve of the 42 patients were already seen three and four months after symptom onset without relevant changes in the Chalder Fatigue Scale (CFQ) and SF36 compared to six months after symptom onset.

Symptom severity: Fatigue as the leading symptom of PCS was assessed by the Chalder Fatigue Score (CFQ). PCS/non-ME/CFS patients reported less fatigue compared to non-COVID- 19 ME/CFS (Fig. 1). Frequency and severity of PEM as the cardinal symptom of ME/CFS was a strong discriminatory factor between PCS/non-ME/CFS and non-COVID ME/CFS patients, but differences were not significant between PCS/non-ME/CFS and PCS/ME/CFS. While according to diagnostic criteria all ME/CFS patients had PEM of 14 hours or more, 18 of 23 PCS/non-ME/CFS patients reported PEM of less than 14 hours as shown in Fig. 2. PEM severity, frequency and length was similar in the PCS/ME/CFS and non-COVID-19 ME/CFS patients. PEM, stress intolerance and hypersensitivity to temperature were less severe in PCS/non-ME/CFS compared to non-COVID ME/CFS and flu-like symptoms were less severe in both PCS cohorts compared to non-COVID ME/CFS.

Autonomic dysfunction: The majority of PCS patients suffered from autonomic dysfunction assessed by Composite Autonomic Symptom Score (COMPASS 31) with moderate (defined as a range between 20 and 40 out of 100) symptoms in 21 and severe (defined as a range of more than 40 from 100) in 11 patients. Severity of symptoms was not significantly different between the cohorts. The COMPASS 31 total score and sub166 scores comprise orthostatic, gastrointestinal, vasomotor, pupillomotor, secretory, and bladder symptoms.

Hand grip strength (HGS): Muscle fatigue and fatigability were assessed by ten repeat hand grips at maximum force (Fmax1 and Fmean1) and were repeated after 60 minutes (Fmax2 and Fmean2). Compared to reference values for age-matched healthy subjects, most patients were below the cut-off values for Fmax1/2 and Fmean1/2 discriminating healthy controls from ME/CFS as shown in Fig. 3 for the female patients. Differences between cohorts were not found after BH correction.

Sitting and standing heart rate and blood pressure: Heart rate and systolic and diastolic blood pressure sitting, standing and after two, five and ten minutes was assessed in PCS/non-ME/CFS and PCS/ME/CFS patients and is shown for females in Fig.4. Three patients with PCS/non-ME/CFS and seven with PCS/ME/CFS had a sitting blood pressure of >140mmHg systolic and/or >90mmHg diastolic sitting. Four patients (3 female, 1 male) with PCS/ME/CFS and one patient with PCS/non-ME/CFS fulfilled diagnostic criteria for postural ycardia syndrome (POTS). Postural hypotension was diagnosed in six patients with PCS/ME/CFS (five females and one male) and one with PCS/non- ME/CFS.

Laboratory parameters: Table 6 lists laboratory values in PCS/ME/CFS and PCS/non-ME/CFS patients, which were out of normal range in a subset of patients. Remarkably, mannose binding lectin (MBL) deficiency, which is associated with enhanced susceptibility to infections and was found previously 199 more frequent in ME/CFS 27 was more frequent in both PCS patient cohorts with 17% and 23%, too compared to 5% in the general population. While C-reactive protein (CRP) was slightly elevated in two patients only, another marker of inflammation interleukin-8 (IL-8), which we assessed in erythrocytes, was above the normal value in 37 and 48% of all patients indicating inflammation during the last three to four months. Elevated antinuclear antibodies (ANA) of 1:160 - 1:1280 were found in eight patients. Double-stranded DNA (ds- DNA) and extractable nuclear antigen antibodies (ENA) were negative in all patients and there was no evidence for a rheumatological disease. Four patients showed elevated thyroid peroxidase (TPO) antibodies with normal thyroid stimulating hormone (TSH) and free triiodothyronine/thyroxine (fT3/fT4). Three of them had been diagnosed with Hashimoto thyroiditis before COVID-19. Deficiencies of Vitamin D and folic acid were found in 17% and 19% of all patients, respectively. N-terminal pro-brain natriuretic peptide (NT-pro BNP) was slightly elevated in three patients. Angiotensin converting enzyme 1 (ACE1) and ACE2 were assessed due to presumably dysregulation in PCS. Indeed, ACE1 levels were below the normal range in 31% of all patients. There were no differences between laboratory findings in PCS/ME/CFS and PCS/non-ME/CFS patients. To investigate a potential pathophysiological role of laboratory parameters of relevance for oxygen supply, inflammation and vasoregulation, we analyzed associations with levels of fatigue assessed by questionnaires and muscle fatigue determined by HGS within a correlation analysis using spearman's p (Fig. 5). Remarkably, we found several significant correlations following BH correction. HGS parameters showed a positive correlation with levels of hemoglobin in both PCS/non- ME/CFS and PCS/ME/CFS. Further we observed a negative correlation of Fmax1 with IL-8 in erythrocytes and of Fmean2 with CRP and a positive correlation with angiotensin converting enzyme 2 (ACE2) levels in the PCS/non-ME/CFS cohort. In PCS/ME/CFS cohort 232 there was a positive correlation of HGS parameters with bilirubin and ferritin and a negative with NT-pro BNP levels. In non-COVID ME/CFS patients we observed a similar correlation of bilirubin with HGS which was not significant after BH correction. None of these biomarkers showed an association with fatigue severity assessed by questionnaires.

Table 6: Laboratory values in PCS cohorts

| | | PCS/ME/ CFS | | | | | | PCS/non-ME/CFS | | | | | | Reference range | |
|---|---|---|---|---|---|---|---|---|---|---|---|---|---|---|---|
| | unit | n | media n | min | max | % low | % hig h | n | media n | min low | max | % | % hig h | m | f |
| CD4 Tcells abs | /nl | 19 | 0.75 | 0.31 | 2.07 | 5 | 11 | 23 | 0.72 | 0.31 | 1.52 | 17 | 4 | 0.5-1.2 | |
| CD8 Tcells abs | /nl | 19 | 0.41 | 0.21 | 0.97 | 16 | 11 | 23 | 0.37 | 0.21 | 0.61 | 35 | 0 | 0.3-0.8 | |
| Erythroctes | /pl | 19 | 4.5 | 4 | 5 | 0 | 0 | 23 | 4.7 | 4 | 5.4 | 0 | 0 | 4.3-5.8 | 3.9-5.2 |
| Hb | g/dl | 19 | 13.7 | 12.2 | 15.4 | 0 | 0 | 23 | 13.7 | 12.2 | 17.6 | 0 | 4 | 13.5-17.0 | 12.0-15.6 |
| Thtomboyct es | /nl | 19 | 263 | 143 | 376 | 5 | 5 | 23 | 233 | 168 | 452 | 0 | 4 | 150-370 | |
| Ferritine | µg/l | 19 | 91.9 | 16.7 2 | 337. | 0 | 16 | 21 | 69.9 | 11.2 | 235.1 | 5 | 5 | 30-400 | 13-150 |
| Creatinine | mg/dl | 18 | 0.785 | 0.61 | 1.04 | 0 | 22 | 21 | 0.77 | 0.61 | 0.99 | 5 | 5 | 0.7-1.2 | 0.5-0.9 |
| Creatinkina se | U/l | 19 | 74 | 25 | 152 | - | 0 | 21 | 61 | 41 | 273 | - | 5 | <190 | <167 |
| LDH | U/l | 19 | 205 | 147 | 317 | 0 | 16 | 22 | 195 | 124 | 280 | 5 | 5 | 135-250 | |
| Bilirubin | mg/dl | 18 | 0.505 | 0.22 | 1.6 | - | 6 | 22 | 0.49 | 0.2 | 0.97 | - | 0 | <1.2 | |
| GPT | U/l | 18 | 17.5 | 11 | 49 | - | 11 | 21 | 18 | 10 | 49 | - | 10 | <41 | <31 |
| GOT | U/l | 18 | 24.5 | 17 | 33 | - | 0 | 21 | 22 | 15 | 37 | - | 0 | <49 | <35 |
| NT-pro BNP | ng/l | 18 | 47 | 7 | 125 | - | 11 | 21 | 39 | 6 | 181 | - | 5 | * | |
| ACE 1 | U/l | 17 | 26.8 | 10.3 | 52.5 | 29 | 0 | 22 | 24.85 | 12.2 | 37.6 | 32 | 0 | 20-70 | |
| ACE 2 | ng/ml | 18 | 4.05 | 2.5 7 | 541. | 6 | 17 | 20 | 4.6 | 2.7 | 387.6 | 0 | 25 | n.a. | |
| fT3 | ng/l | 18 | 3.325 | 2.54 | 3.98 | 0 | 0 | 23 | 3.27 | 2.57 | 4.08 | 0 | 0 | 2-4.4 | |
| fT4 | ng/l | 18 | 13.8 | 11 | 18.1 | 0 | 11 | 23 | 13.1 | 9.77 | 15.9 | 0 | 0 | 9.3-17 | |
| TSH basal | mU/l | 18 | 1.385 | 0.37 | 2.36 | 0 | 0 | 21 | 1.33 | 0.7 | 3.31 | 0 | 0 | 0.27-4.2 | |
| Anti- TPO | kU/l | 15 | 9 | 9 | 328 | - | 13 | 19 | 9 | 9 | 81 | - | 11 | <34 | |
| Ab | | | | | | | | | | | | | | | |
| IgG | g/l | 19 | 10.94 | 7.13 2 | 13.2 | 0 | 0 | 23 | 10.63 | 6.73 | 16.18 | 4 | 4 | 7-16 | |
| IgA | g/l | 19 | 1.75 | 1.01 | 4.09 | 0 | 11 | 23 | 1.92 | 0.32 | 4.62 | 4 | 4 | 0.7-4 | |
| IgM | g/l | 19 | 1.15 | 0.63 | 5.05 | 0 | 11 | 23 | 1.09 | 0.45 | 13.48 | 0 | 9 | 0.4-2.3 | |
| IgE | g/l | 19 | 46 | 2.4 | 1072 | 0 | 21 | 23 | 35.1 | 4 | 751 | 0 | 22 | 0-100 | |
| IgG1 | g/l | 19 | 5.797 | 3.637 6 | 7.22 | 0 | 0 | 22 | 6.034 | 3.13 | 8.209 | 0 | 5 | 2.8-8 | |
| IgG2 | g/l | 19 | 3.904 | 2.607 5 | 6.44 | 0 | 5 | 22 | 4.881 | 2.752 | 5.934 | 0 | 5 | 1.12-5.7 | |
| IgG3 | g/l | 19 | 0.473 | 0.15 2 | 1.34 | 5 | 5 | 22 | 0.505 | 0.19 | 1.062 | 5 | 0 | 0.24-1.25 | |
| IgG4 | g/l | 19 | 0.262 | 0.132 5 | 2.11 | 0 | 5 | 22 | 0.544 | 0.096 | 1.781 | 0 | 9 | 0.052-1.25 | |
| C3 | mg/l | 18 | 1080 | 850 | 1640 11 | | 0 | 22 | 1155 | 820 | 1720 | 14 | 0 | 900-1800 | |
| C4 | mg/l | 18 | 185 | 100 | 330 | 0 | 0 | 22 | 210 | 110 | 320 | 0 | 0 | 100-400 | |
| MBL | ng/ ml | 18 | 1452 | <50 | 4000 | 17 | - | 22 | 3734 | <50 | 4000 | 23 | - | >50 | |
| IL8 in | pg/ | 19 | 134.8 | 97 | 224 | - | 37 | 23 | 149.2 | 65.2 | 442 | - | 48 | <150 | |

(continued)

| | | PCS/ME/ CFS | | | | | | PCS/non-ME/CFS | | | | | | Reference range | |
|---|---|---|---|---|---|---|---|---|---|---|---|---|---|---|---|
| | unit | n | media n | min | max | % low | % hig h | n | media n | min low | max | % | % hig h | m | f |
| erythroc. | ml | | | | | | | | | | | | | | |
| Soluble | IU/ | 19 | 312 | 176 | 746 | - | 5 | 23 | 326 | 173 | 765 | - | 4 | <710 | |
| IL2R | ml | | | | | | | | | | | | | | |
| CRP | mg/l | 19 | 0.8 | 0.6 | 7.1 | - | 5 | 23 | 1 | 0.6 | 7.57 | - | 4 | <5 | |
| Vitamin D3 | nmo l/l | 19 | 80.4 | 46.2 6 | 109. | 11 | 0 | 23 | 62.4 | 16.7 | 217.6 | 22 | 4 | 50-150 | |
| Folic acid | µg/l | 16 | 7.3 | 2.5 | 20 | 19 | 13 | 21 | 9.7 | 2.7 | 20 | 19 | 5 | 4.6-18.7 | |

abs: absolute; R: receptor; * NT-pro BNP reference values according to age: ≤44 years = ≤97, ≤54years = ≤121, ≤64 years = ≤198

Conclusion: In this study, we provide evidence that a subset of PCS patients presenting with the hallmark of moderate to severe fatigue and exertion intolerance fulfill the Canadian Consensus Criteria (CCC) for ME/CFS. In PCS patients who did not fulfill these criteria, this was mostly due to shorter duration of PEM, while severity and frequency of other symptoms was not different compared to the PCS/ME/CFS cohort. A less severe symptom burden of the PCS/non-ME/CFS cohort became only evident in comparison to non-COVID ME/CFS patients with less fatigue, stress intolerance, hypersensitivity to temperature and flu-like symptoms. Severity of symptoms was similar in the PCS/ME/CFS compared to the non-COVID-19 ME/CFS patient cohort with the only exception of less severe flu-like symptoms.

Strikingly, while all PCS patients suffered from moderate to severe fatigue and exertion intolerance, a subset did not fulfill the CCC criteria for ME/CFS mostly due to a shorter PEM lasting less than 14 hours. We have not seen such a symptom constellation in other chronic postinfectious syndromes so frequently. Based on the average Bell disability scores, about two thirds of all patients are forced to reduce working hours or are unable to work. Most patients in our study presented with symptoms of autonomic dysfunction. Mental health is not relevantly impaired in most patients with PCS.

COVID-19 triggers a strong inflammatory response and there is evidence for autoimmunity triggered by COVID-19. We have no indication for ongoing overt inflammation as only two of the patients presented with mildly elevated CRP. Almost half of the patients had, however, elevated IL-8 levels in erythrocytes. Elevated ANA in eight patients (seven female/one male) and the preponderance of females may indicate an autoimmune mechanism similar to ME/CFS triggered by other infections.

Remarkably we found diminished HGS in the majority of patients and an association of several biomarker with muscle fatigue. First, positive correlations of HGS parameters with hemoglobin levels observed in both PCS cohorts, and with ACE2 in the PCS/non-ME/CFS and with bilirubin, ferritin and NT-pro BNP levels in the PCS/ME/CFS cohort point to endothelial dysfunction and hypoperfusion as cause of muscle fatigue. Negative association of NT-pro BNP levels with HGS is consistent with our hypothesis of hypoperfusion as cause of muscle fatigue. In the PCS/non-ME/CFS cohort, a negative correlation of Fmax1 with IL-8 in erythrocytes and CRP levels with Fmean2 was found which points to low level inflammation as mechanism of muscle fatigue.

We also found endothelial dysfunction in a subset of both PCS cohorts. In the non-COVID ME/CFS cohort analysed in this study we observed a similar correlation of HGS parameters with bilirubin. A possible explanation for the weaker correlations might be longer disease duration of the patients in the ME/CFS cohort (13 months) and several different infectious triggers.

Taken together, our study provides evidence that patients following mild COVID-19 develop a chronic syndrome fulfilling diagnostic criteria of ME/CFS in a subset. By defining and characterizing subgroups of PCS patients we could identify associations of HGS with biomarkers pointing to hypoperfusion and inflammation as potential pathomechanisms. We must anticipate that this pandemic has the potential to dramatically increase the number of ME/CFS patients. At the same time, it offers the unique chance to identify ME/CFS patients in a very early stage of disease and apply interventions such as pacing and coping early with a better therapeutic prognosis. Further, it is an unprecedented opportunity to understand the underlying pathomechanism and characterize targets for specific treatment approaches.

**Example 3: Endothelial dysfunction and endothelial biomarkers in two patient cohorts**

Study aim: In this study we aimed to characterise the peripheral endothelial function using post-occlusive reactive hyperaemia peripheral arterial tonometry (RH-PAT) in PCS patients following mild to moderate COVID-19. In addition, we analysed several endothelial biomarkers including Endothelin-1 (ET-1), Angiopoietin-2 (Ang-2) and Endocan (ESM-1) which play an important role in inflammatory and non-inflammatory diseases associated with ED. Further we assessed Interleukin 8 (IL-8), Angiotensin-Converting Enzyme (ACE) and ACE2. IL-8 is expressed by monocytes/macrophages, but also by endothelial cells and vascular smooth muscle cells. The chemokine plays an important role in (endothelial) inflammation and regulation of leukocyte rolling as well as vascular permeability. ACE and ACE2 are crucial actors in the maintenance of the blood pressure and vascular homeostasis. ACE converts angiotensin I (AngI) into the vasoconstrictive AngII, which activates the AT1/2 receptor. Counter-regulatory ACE2 cleaves AngI into Ang1-9 and metabolizes AngII to Ang1-7. Peptides generated by ACE2 are ligands of the receptor Mas stimulating a protective, vasodilative signalling.

Methods: Participants: 30 PCS patients with persistent fatigue and exertion intolerance following mild to moderate COVID-19 were recruited from an ongoing observational study for measurement of endothelial function by RH-PAT. 14 of the patients fulfilled diagnostic criteria for ME/CFS according to 2003 Canadian Consensus Criteria. In contrast to the original classification and in accordance with the studies of L. Jason and colleagues a minimum of 14 hours of PEM instead of 24 hours was required for diagnosis of ME/CFS. As a control group 15 age- and sex-matched HC without a known history of COVID-19 were characterised using RH-PAT. For biomarker analysis in addition 15 reconvalescent post COVID-19 healthy controls (PCHC) were recruited. The endothelial biomarkers were validated in a 2nd cohort of 57 PCS patients, 50 HC without a known history of COVID-19 and 20 PCHC with at least 5 months elapsed following

COVID-19 infection. HCs of both cohorts were recruited before SARS-COV-2 vaccination and were negative for SARS-CoV2 antibodies tested by Anti-SARS-CoV-2-spike IgG-ELISA (Euroimmune). The study was approved by the Ethics Committee of Charité - Universitätsmedizin Berlin in accordance with the 1964 Declaration of Helsinki and its later amendments (EA2/066/20). All study participants gave written informed consent.

Assessment of endothelial function: Peripheral endothelial function was assessed using post-occlusive reactive hyperaemia peripheral arterial tonometry (RH-PAT) (endoPAT2000 device; Itamar Medical Ltd.; Caesarea, Israel) as previously described (10). Endothelium-mediated changes in peripheral arterial tone were recorded using plethysmographic probes on the index finger of each hand during reactive hyperaemia. Hyperaemia was induced by occlusion of the left brachial artery over five minutes using an inflatable blood pressure cuff. The RHI was calculated from the change in the pulse wave amplitude (PWA) relative to baseline in the occluded arm and corrected for corresponding changes in PWA relative to baseline in the contra-lateral, non-occluded arm in order to minimize the influence of non-endothelial dependent systemic effects using the equation: RHI = (A/B) ÷ (C/D). Based on previous studies and the manufacturers analysis instructions ED was defined as RHI ≤1.67. The automatic batch analysis of individual measurements was performed using the manufacturer's analysis version 3.1.2 (2.0).

Measurements of peripheral endothelial function were performed under standardised conditions between 8:30 and 12:30 am after 15 minutes of supine rest in a quiet, dimly lit, air-conditioned room ensuring temperatures of 21-24°C. Blood pressure was measured using a digital blood pressure monitor on the right upper arm in order to provide orientation for the individual supra-systolic pressure of 60mmHg necessary for sufficient occlusion in the later procedure. The average heart rate was determined during the five-minute pre-occlusion period by the endoPAT2000 device. Venous blood samples were collected 10 minutes following the assessment of endothelial function from the participants control arm where no occlusion was performed.

Assessment of biomarkers: Serum ESM-1 concentration was measured by ELISA purchased from Aviscera Bioscience, Inc., serum Ang-2 was measured by Quantikine Human Angiopoietin-2 Immunoassay purchased from R&D Systems, Inc and serum ET-1 concentration was determined by QuantiGlo™ ELISA from R&D Systems, Inc. according to the manufacturers' protocols. ACE and IL-8 (post erythrocyte lysis (32)) were determined at the Charité diagnostics laboratory (Labor Berlin GmbH, Berlin, Germany). Serum ACE2 level was determined using enzyme-linked immunoassay (ELISA; R&D Systems). Biomarkers were assessed in RH-PAT study participants and further 15 PCHC subjects (Table 1A). In addition, biomarker analysis was validated in a 2nd cohort including post-COVID ME/CFS, PCS, HC and PCHC subjects.

Assessment of symptom severity: As part of their initial consultation at the Institute of Medical Immunology at Charité Berlin all patients completed the modified Symptom Questionnaire for PEM (DSQ-PEM), the Chalder Fatigue Scale and the Bell Disability Scale. The DSQ-PEM uses five different 5-point Likert scales each for assessing the frequency or severity of PEM symptoms and one 7-point Likert scale for assessing the duration of PEM (27) with a resulting maximum score of 46. The Chalder Fatigue Scale uses eleven different 4-point Likert scales to measure the severity of fatigue with a resulting maximum score of 33. The Bell Disability Scale is comprised of eleven statements in association with the level of physical function. The scale is scored from 0 (very severe, bedridden constantly) - 100 (healthy) in steps of ten.

Statistical analysis: For comparative analysis, Kruskal-Wallis test with Dunn's post-hoc multiple comparisons or Mann-Whitney-U rank-sum-test for quantitative parameters were used. ED distribution was analysed in a 2 x 2 contingency table and tested for significance by Fisher's exact test. Correlation analysis was performed using non-parametric Spearman coefficient. Statistical tests were performed using GraphPad Prism software (Version 6.07). A two-tailed p value ≤ 0.05 was considered statistically significant.

Study population: 30 PCS patients were enrolled for assessment of endothelial function by RH-PAT with 14 of them fulfilling diagnostic criteria for ME/CFS, the others were referred to as PCS. In addition, 15 age- and sex-matched HC without a history of COVID-19 were recruited. Biomarkers were additionally determined in 15 reconvalescent post COVID-19 healthy controls (PCHC). Fatigue assessed by the Chalder Fatigue Scale and severity of the patient's disability assessed by the Bell Disability Scale were comparable between both patient groups. The PEM score was higher in ME/CFS (median of 33.5) than in PCS patients (median 22.0). The patient groups did neither differ in heart rate nor blood pressure. The time interval since COVID-19 infection was median 9 months in patients but only 2 months in PCHC. Endothelial biomarkers were validated in a 2nd study cohort. Here the PEM score again was higher in ME/CFS (median of 36) than in PCS patients (median 24.5). Fatigue assessed by the Chalder Fatigue Scale was more severe in ME/CFS (median of 29) than in PCS (median of 24) and physical function assessed by the Bell Disability Scale was worse in ME/CFS (median of 30) than in PCS (median of 50). The time interval since COVID-19 infection was median 8 months in patients and 6 months in PCHC.

Assessment of ED by RH-PAT: Five of 14 patients with post COVID-19 ME/CFS and five of 16 patients with PCS had peripheral ED defined by diminished reactive hyperaemia index (RHI) (≤1.67), but none of HC (Figure 6). Patient cohorts showed a significantly higher frequency of ED compared to the HC group (Fisher's exact test; pME/CFS: 0.0169 and pPCS: 0.0434).

Correlation of clinical parameters with RHI: The RHI values correlated negative with age in the HC group (r= -0.5405; p: 0.0375). In contrast, in patients with PCS a positive correlation was found between RHI values and age (r= 0.5328; p: 0.0356). Further, the RHI positively correlated with systolic (r= 0.6490; p: 0.0078) and diastolic blood pressure (r= 0.5283; p: 0.0374) in PCS patients. None of these associations were found in the ME/CFS patient group. In addition, no associations of the RHI with Chalder Fatigue Scale, Bell Disability Scale or PEM score were observed.

Biomarkers for ED: The endothelial biomarkers ET-1, Ang-2 and ESM-1 were comparatively analysed in patient and control serum by ELISA in the RH-PAT study cohort. ET-1 concentrations in ME/CFS patients were significantly higher compared to PCHC analysed in the same assay (p=0.0051, Figure 7A, Table 7A). The time post infection was, however, much shorter in the PCHC cohort compared to patients. In the 2nd validation cohort with a median duration of 6 and 8 months post COVID-19 in the PCHC and patients, respectively higher ET-1 levels could be confirmed in ME/CFS compared to PCHC (p=0.0007, Figure 7B) and HC (p=0.0003, Figure 7B). In addition, ET-1 serum concentration was also significantly higher in PCS patients compared to PCHC (p=0.0365, Figure 3B) and HC (p=0.0384, Figure 7B).

In the RH-PAT study cohort no significant differences in Ang-2 and ESM-1 between the cohorts were observed (Table 7A). However, Ang-2 level tend to be lower in PCS patients compared to HC (p=0.1021) but not in ME/CFS (Figure 8A, Table 7A). Lower Ang-2 levels in PCS patients compared to HC could be confirmed in the validation cohort (p<0.0001, Figure 8B, Table 7B). Furthermore, Ang-2 levels were lower in PCS than in post COVID ME/CFS patients (p=0.0172). Interestingly, Ang-2 levels were lower in PCHC compared to HC (p=0.0204), too.

**Table 7: Levels of biomarker**

| (A) PAT study group | ME/CFS median (IQR) | n | PCS median (IQR) | n | HC median (IQR) | n | PCHC median (IQR) | n | *p* value |
|---|---|---|---|---|---|---|---|---|---|
| Serum ET-1 (pg/ml) | 1.434 (1.208-1.622) | 14 | 1.168 (1.019-1.328) | 16 | 1.141 (1.014-1.302) | 15 | 1.043 (0.950-1.241) | 15 | pi=0.1961<br>$p_2$=0.0619<br>$P_3$>0.9999<br>**$p_4$=0.0051**<br>$p_5$>0.9999<br>$P_6$>0.9999 |
| Serum Esm-1 (ng/ml) | 2.013 (1.199-4.890) | 14 | 2.013 (1.366-6.223) | 16 | 1.782 (0.984-3.399) | 15 | ND | | $p_1$>0.9999<br>$P_2$>0.9999<br>$P_3$>0.9999 |
| Serum Ang-2 (pg/ml) | 1866 (1502-2108) | 14 | 1621 (1283-1762) | 16 | 2058 (1566-3459) | 15 | 1696 (1274-2906) | 15 | $p_1$>0.9999<br>$P_2$>0.9999<br>$p_3$=0.1021<br>$P_4$>0.9999<br>$p_5$>0.9999<br>$P_6$>0.9999 |
| Serum ACE (U/l) | 25.20 (17.70-32.35) | 14 | 27.50 (23.65-32.65) | 16 | normal range*: 20-70 | | ND | | $p_1$=0.4043 |
| Serum ACE2 (U/ml) | 5.099 (4.192-6.162) | 14 | 4.476 (3.946-6.476) | 16 | 4.971 (4.23-6.792) | 15 | ND | | $p_1$>0.9999<br>$P_2$>0.9999<br>$p_3$=0.5715 |
| IL-8 post erythrocyte lysis (pg/ml) | 139.9 (116.7-197.6)<br>> 150 | 14<br>7 | 188.2 (150.2-217.0)<br>> 150 | 16<br>12 | normal range*: < 150 | | ND | | $p_1$=0.0592 |

(continued)

| (B) validation study group | ME/CFS *median (IQR)* | *n* | PCS *median (IQR)* | *n* | HC *median (IQR)* | *n* | PCHC *median (IQR)* | *n* | *p* value |
|---|---|---|---|---|---|---|---|---|---|
| Serum ET-1 (pg/ml) | 1.110 (0.980-1.325) | 25 | 1.130 (0.750-1.310) | 25 | 0.820 (0.770-0.940) | 47 | 0.830 (0.723-0.948) | 20 | $p_1$>0.9999<br>**$p_2$=0.0003**<br>**$p_3$=0.0384**<br>**$P_4$=0.0007**<br>**$p_5$=0.0365**<br>$P_6$>0.9999 |
| Serum Ang-2 (pg/ml) | 2759 (2193-3425) | 26 | 2149 (1564-2535) | 30 | 2977 (2355-4143) | 50 | 2356 (1802-3027) | 20 | **$p_1$=0.0188**<br>$P_2$>0.9999<br>**$p_3$<0.0001**<br>$p_4$=0.5336<br>$p_5$>0.9999<br>**$p_6$=0.0234** |
| Serum ACE (U/l) | 29.20 (20.10-42.20) | 23 | 23.70 (19.43-31.75) | 30 | normal range*: 20-70 | | ND | | $p_1$=0.1643 |
| IL-8 post erythrocyte lysis (pg/ml) | 172.6 (136.9-198.0)<br>> 150 | 25<br>17 | 150.2 (126.7-180.1)<br>> 150 | 29<br>15 | normal range*: < 150 | | ND | | $p_1$=0.0884 |

For comparative analysis of more than two groups, Kruskal-Wallis test with Dunn's post-hoc multiple comparisons was used, otherwise Mann-Whitney-U rank-sum-test were used. A p value ≤ 0.05 was considered statistically significant. $p_1$ = ME/CFS compared with HC; $p_2$ = PCS compared with HC; $p_3$ = ME/CFS compared with PCS [ACE = Angiotensin-converting enzyme; Ang-2 = Angiopoietin-2; Esm-1 = Endocan; ET-1 = Endothelin-1; HC = healthy control; IQR = interquartile range; NA = not assessed]
* normal range as stated by LaborBerlin; $p_1$: ME/CFS vs. PCS, $p_2$: ME/CFS vs HC; $p_3$: PCS vs HC, $p_4$: ME/CFS vs. PCHC, $p_5$: PCS vs. PCHC, p6: HC vs PCHC

Further, serum ACE1 and IL-8 (measured following erythrocyte lysis) were determined in the diagnostic laboratory in patients and ACE2 by ELISA (Table 7). Median levels of ACE1 did not differ between both cohorts, but were below the normal reference value in five of 14 ME/CFS and two of 16 PCS patients (< 20 U/l) as well as in eight of 23 ME/CFS and five of 30 PCS patients of the validation cohort. ACE2 levels were not different between both patient cohorts and HC. Median IL-8 concentration did not differ between patient cohorts but were above the normal reference value (> 150 pg/ml) in 12 of 16 PCS patients and seven of 14 ME/CFS patients in the PAT cohort as well as 17 of 25 ME/CFS and 15 of 29 PCS patients in the validation cohort.

Neither ET-1, Ang-2 and ESM-1 concentrations, nor ACE1, ACE2 and IL-8 levels correlated with the RHI.

Discussion: Long COVID is a poorly understood condition with multiple features and a broad range of symptoms. Endothelial infection in COVID-19 may have long-term consequences for vascular function. In our present study, we found ED and dysregulated levels of the endothelial marker ET-1 and Ang-2 in PCS patients on average 8 months after mild to moderate COVID.

In acute COVID-19 there is ample evidence for ED and impaired microcirculation. SARS-CoV-2 has been shown to alter vascular homeostasis by directly infecting endothelial cells via ACE2. ACE2 is expressed in arterial and venous endothelial cells and is internalized and downregulated after binding of the virus. Local angiotensin II hyperreactivity triggered by this leads to progression of inflammation and fibrosis. In addition to direct injury to the vascular endothelium by endothelial cell infection, inflammatory mediators could also contribute to endothelitis and endothelial cell injury. There is also first evidence for endothelial damage especially in pulmonary microvascular cells by apoptosis or autophagy post-acute COVID-19.

First studies have reported ED post COVID-19. A recent study analysed endothelial function using the EndoPAT technology in patients during acute as well as median 100 days post COVID-19 and reported impaired RHI in the post-infection stage only. However, in this study no information about the severity of acute COVID nor about symptom persistence was reported. Another study in patients post severe acute COVID-19 found impaired ED in half of the patients, which was associated with fatigue, chest pain and neuro-cognitive difficulties and severity of acute COVID-19. A recent study reported elevated levels of circulating endothelial cells, in COVID-19 convalescents on average 34 days post symptom onset as a biomarker for ED associated with levels of several cytokines.

ED has been described in non-COVID-19 post-infectious ME/CFS. In our previous study of 35 ME/CFS patients peripheral ED assessed by RH-PAT was observed in half of the patients and was associated with disease severity. A recent study in post-infectious ME/CFS confirmed these findings by demonstrating ED by both RH-PAT and flow mediated dilatation. ED resulting in muscle and cerebral hypoperfusion are considered as key pathomechanisms in ME/CFS.

RHI is usually inversely associated with age and blood pressure and known cardiovascular risk factors, which we also observed for RHI and age in our HC group. Surprisingly we found a paradox positive correlation of the RHI with both age and blood pressure in PCS suggesting that ED develops independently of classical cardiovascular risk factors in these patients and that vascular stiffness may even help to stabilize the vascular diameter.

Remarkably, PCS patients with and without ME/CFS showed elevated levels of the endothelial biomarker ET-1, while reconvalescents had normal levels. Endothelins are the most important and potent vasoconstrictors and are produced by endothelial cells. ET-1 mediates vasoconstriction via the ETA receptor, which is mainly located on vascular smooth muscle cells. Thus our findings may indicate hypoperfusion, which is in line with a recent study in PCS patients with exertion intolerance. Data from cardio-pulmonary exercise testing (CPET) provides evidence for a marked reduction in oxygen consumption during exercise attributed primarily to reduced oxygen diffusion in the peripheral microcirculation. Targeting the ETA and ETB receptors via an antagonist improved the peripheral endothelial function defined by RHI in patients with type 2 diabetes. Thus, ET-1 could be both a biomarker of endothelial involvement and therapeutic target in PCS.

Ang-2 belongs to the angiopoietin/tie-2 pathway regulating endothelial homeostasis and angiogenesis. In the present study, non-ME/CFS PCS patients unexpectedly showed reduced serum Ang-2 levels compared to HC. Ang-2 expression is increased during COVID-19 infection presumably due to endothelial inflammation. Ang-2 expression was shown to decrease under high flow and sheer stress. This may explain diminished levels in our PCS patients, probably due to an increase in vascular stiffness. Remarkably, Ang-2 was diminished in reconvalescent PCHC, too, which may indicate a longer lasting change in vascular perfusion.

As reported in our previous study, we found elevated levels of the pro-inflammatory mediator IL-8 in around 60 % of PCS patients. As IL-8 is rapidly degraded in serum, it concentration was determined in lysed erythrocytes, which bind IL-8 via a duffy antigen receptor. Endothelial cells and monocytes are the main producers of IL-8. IL-8 promotes endothelial cell migration, proliferation as well as survival and endothelial permeability. Elevated IL-8 levels were described in patients with severe as well as mild COVID-19 and correlated with disease prognosis. Thus, IL-8 might indicate ongoing endothelial inflammation in our PCS patients.

Conclusion: In conclusion, our study found that a subset of PCS patients had a diminished RHI indicating peripheral ED. A limitation of our study is the lack of a reconvalescent cohort for RHI assessment, thus we do not know if diminished RHI is associated with PCS symptoms. Elevated ET-1 levels were, however, found in the PCS patients

only, indicating that endothelial hypoperfusion plays a role in PCS and provides a rational for therapeutic targeting. The paradox association of RHI with age and BP and diminished Ang-2 indicates a distinct pathomechanism in the non-ME/CFS PCS subgroup.

**References:**

1. Nacul L, Authier FJ, Scheibenbogen C, et al. EUROPEAN ME NETWORK (EUROMENE) Expert Consensus on the Diagnosis, Service Provision and Care of People with ME/CFS in Europe. 2020

2. Sotzny, F., Blanco, J., Capelli, E., Castro-Marrero, J., Steiner, S., Murovska, M., Scheibenbogen C. European Network on, M. C.. Myalgic Encephalomyelitis/Chronic Fatigue Syndrome - Evidence for an autoimmune disease. Autoimmun Rev. 17, 601-609. 2018

3. Steiner S, Becker SC, Hartwig J, et al. Autoimmunity-Related Risk Variants in PTPN22 and CTLA4 Are Associated With ME/CFS With Infectious Onset. Front Immunol 2020;11:578. doi: 10.3389/fimmu.2020.00578

4. Szklarski M, Freitag H, Lorenz S, Becker SC, Sotzny F, Bauer S, Hartwig J, Heidecke H, Wittke K, Kedor C, Hanitsch LG, Grabowski P, Sepúlveda N, Scheibenbogen C. Delineating the Association Between Soluble CD26 and Autoantibodies Against G-Protein Coupled Receptors, Immunological and Cardiovascular Parameters Identifies Distinct Patterns in Post-Infectious vs. Non-Infection-Triggered Myalgic Encephalomyelitis/Chronic Fatigue Syndrome. Front Immunol. 2021 Apr6;12:644548. doi: 10.3389/fimmu.2021.644548. PMID: 33889154; PMCID: PMC8056217.

**Claims**

1. A pharmaceutical composition comprising a soluble guanylate cyclase stimulator for use in the treatment of a medical condition comprising a chronic vascular dysfunction occurring or persisting after onset of an infection in a human subject.

2. The pharmaceutical composition for use according to claim 1, wherein the chronic vascular dysfunction occurs after the onset of first symptoms of the infection, or after the acute phase of the infection, for example within six months after the acute phase of said infection.

3. The pharmaceutical composition for use according to any of the preceding claims, wherein the chronic vascular dysfunction persists from the onset of first symptoms of the infection, or from the onset of the dysfunction, for at least one month, preferably three months, more preferably six months.

4. The pharmaceutical composition for use according to any of the preceding claims, wherein the chronic vascular dysfunction is associated with vascular constriction, endothelial dysfunction and/or hypo-perfusion.

5. The pharmaceutical composition for use according to any of the preceding claims, wherein the chronic vascular dysfunction comprises muscle weakness, muscle fatigue, muscle pain, moderate fatigue, severe fatigue, exercise intolerance, neurocognitive impairment (e.g. memory disturbance), post exertional malaise, hypersensitivity, and/or pain (e.g. headache), preferably decreased handgrip strength.

6. The pharmaceutical composition for use according to any of the preceding claims, wherein the chronic vascular dysfunction is associated with a medical score above or below a reference level, for example a reference level for a healthy control subject or healthy patient population, wherein the medical score is selected from the group consisting of SF-36 health score, Chalder Fatigue Score, CCC Symptom Score, hand grip strength test, Bell disabling scale, MBSQ score, PEM score, COMPASS-31, cognitive function (SDMT), Blood pressure (BP), Heart Rate (HR) regulation in passive standing test [CDE NIH], HRV and in stress index (SI), pain scale, and reactive hyperemia index (RHI).

7. The pharmaceutical composition for use according to any of the preceding claims, wherein the soluble guanylate cyclase stimulator is selected from the group consisting of vericiguat, riociguat, neliciguat, BAY-41-2272, BAY

41-8543, and etriciguat, preferably vericiguat.

8. The pharmaceutical composition for use according to any of the preceding claims, wherein the treatment comprises administering the soluble guanylate cyclase stimulator, preferably vericiguat, or salt thereof, at 0.1-500 mg/day to a human subject, preferably 0.1-50 mg/day or at 1-10 mg/day.

9. The pharmaceutical composition for use according to any of the preceding claims, wherein the treatment comprises administration of vericiguat once daily for at least one week, up to at least twelve weeks, preferably once daily for 10 weeks, wherein the route of administration is preferably oral, preferably 1 to 10 mg per dose via oral delivery.

10. The pharmaceutical composition for use according to any of the preceding claims, wherein the subject had or was suspected to have had an infection, preferably a viral infection selected from the group consisting of a SARS corona infection, SARS-CoV-2 infection, influenza virus infection, epstein barr virus infection, HSV-1 infection, HHV6 infection, dengue fever virus, and entero virus infection, preferably a SARS-CoV-2 infection.

11. The pharmaceutical composition for use according to any of the preceding claims, wherein the treatment comprises administering vericiguat to a subject who has had or is suspected of having had a SARS-CoV-2 infection and said subject has chronic vascular dysfunction comprising fatigue, muscle fatigue, muscle pain, exercise intolerance, post exertional malaise, hypersensitivity, neurocognitive impairment (e.g. memory disturbance), headache, and/or decreased hand-grip strength compared to reference level for a healthy control subject or healthy patient population, wherein said chronic vascular dysfunction occurs or persists after the onset of first symptoms of SARS-CoV-2 infection, or after the acute phase of SARS-CoV-2 infection.

12. The pharmaceutical composition for use according to any of the preceding claims, wherein the onset of the viral infection is **characterized by** the presence of symptoms of the viral infection, and/or by detection of the viral infection in said subject comprising detection of viral components, such as by a PCR-test or an antigen-test, and wherein said viral infection may be undetectable while the chronic vascular dysfunction persists.

13. The pharmaceutical composition for use according to any of the preceding claims, wherein the subject has an elevated level of one or more of biomarkers selected from ET-1, IL-8, CrP, hemoglobin, and/or NT-proBNP, and/or a decreased level of one or more of biomarkers selected from ferritin, ANG-2, ACE2, ACE-1, and/or bilirubin, wherein said level is compared to a level in a healthy control subject or healthy patient population and is determined in a sample comprising and/or derived from a bodily fluid and/or cells from the subject, preferably from blood, serum or plasma.

14. The pharmaceutical composition for use according to any of the preceding claims, wherein the treatment comprises administering vericiguat orally at 2.5 to 10 mg/day to a subject diagnosed with ME/CFS or Post COVID Syndrome and said subject has chronic vascular dysfunction occurring or persisting after the onset of first symptoms of SARS-CoV-2 infection, or after the acute phase of SARS-CoV-2 infection.

15. The pharmaceutical composition for use according to any of the preceding claims, wherein the subject is or has been treated additionally with a guideline treatment for Post-COVID/Long-COVID and/or for ME/CSF.

Fig. 1

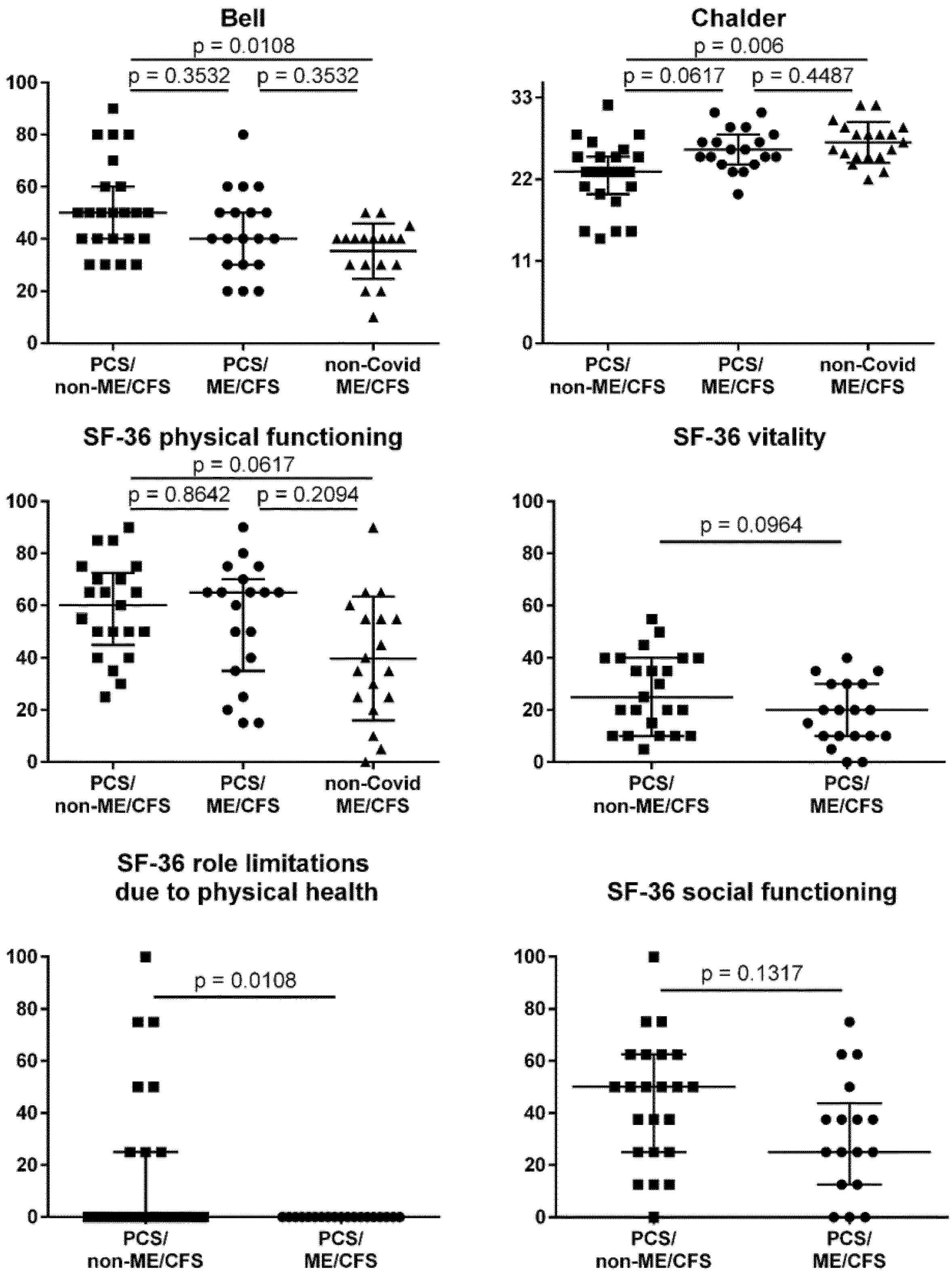
Bell
p = 0.0108
p = 0.3532
p = 0.3532
PCS/ non-ME/CFS
PCS/ ME/CFS
non-Covid ME/CFS
Chalder
p = 0.006
p = 0.0617
p = 0.4487
SF-36 physical functioning
p = 0.0617
p = 0.8642
p = 0.2094
SF-36 vitality
p = 0.0964
SF-36 role limitations due to physical health
p = 0.0108
SF-36 social functioning
p = 0.1317

Fig. 2

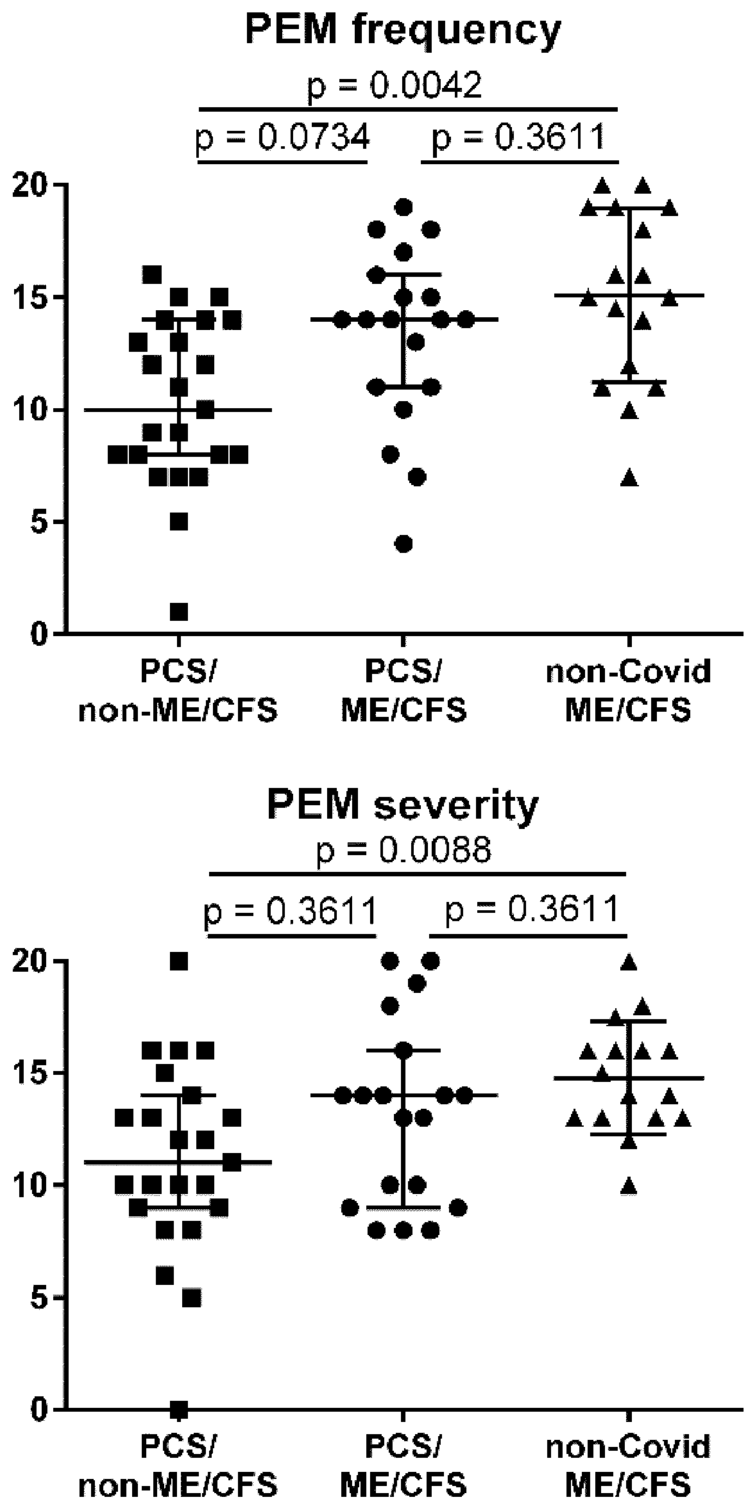
PEM frequency
p = 0.0042
p = 0.0734
p = 0.3611
20
15
10
5
0
PCS/
non-ME/CFS
PCS/
ME/CFS
non-Covid
ME/CFS
PEM severity
p = 0.0088
p = 0.3611
p = 0.3611
20
15
10
5
0
PCS/
non-ME/CFS
PCS/
ME/CFS
non-Covid
ME/CFS

Fig. 3

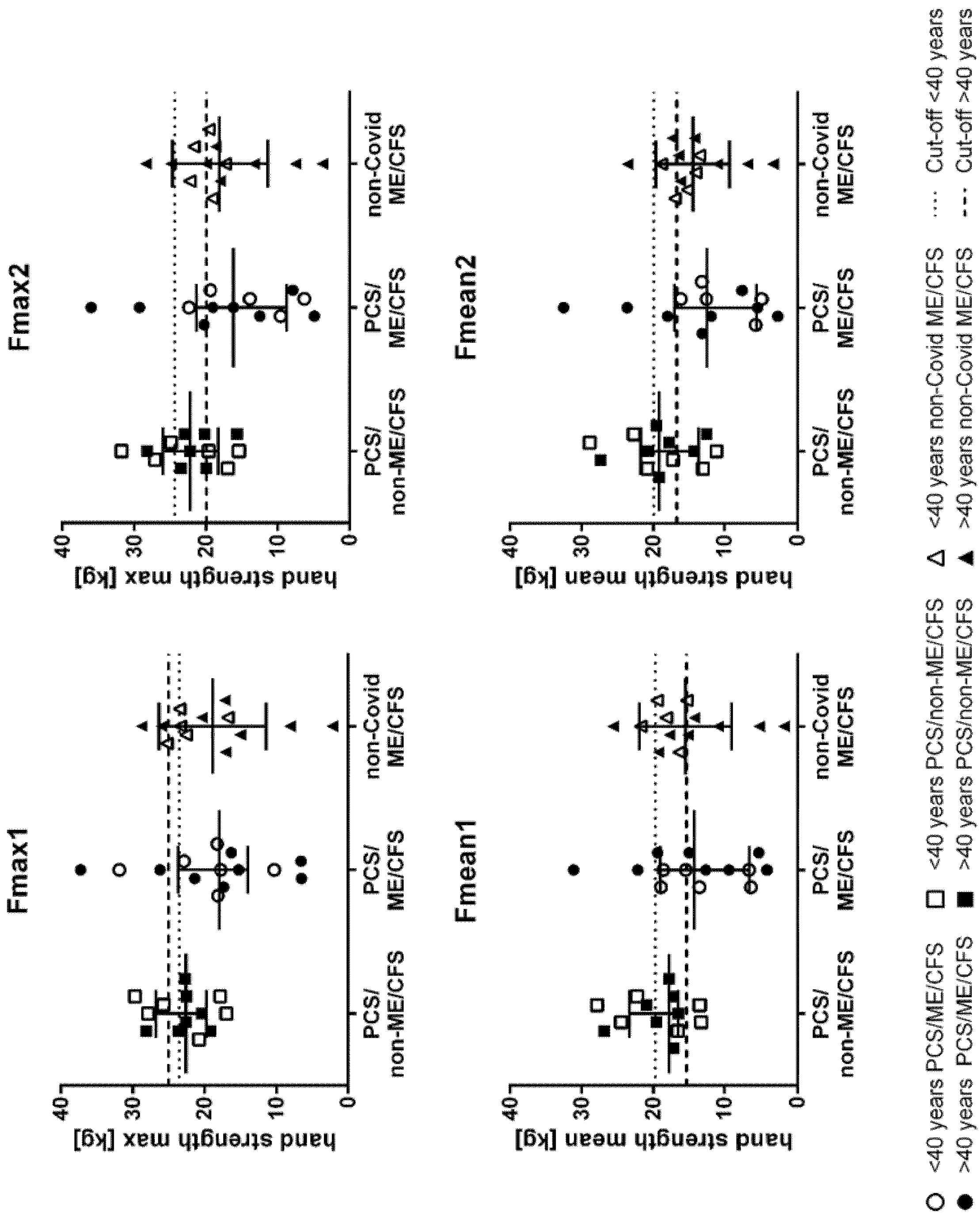
Fmax1
Fmax2
Fmean1
Fmean2
hand strength max [kg]
hand strength mean [kg]
0
10
20
30
40
PCS/ non-ME/CFS
PCS/ ME/CFS
non-Covid ME/CFS
<40 years PCS/ME/CFS
>40 years PCS/ME/CFS
<40 years PCS/non-ME/CFS
>40 years PCS/non-ME/CFS
<40 years non-Covid ME/CFS
>40 years non-Covid ME/CFS
Cut-off <40 years
Cut-off >40 years

Fig. 4

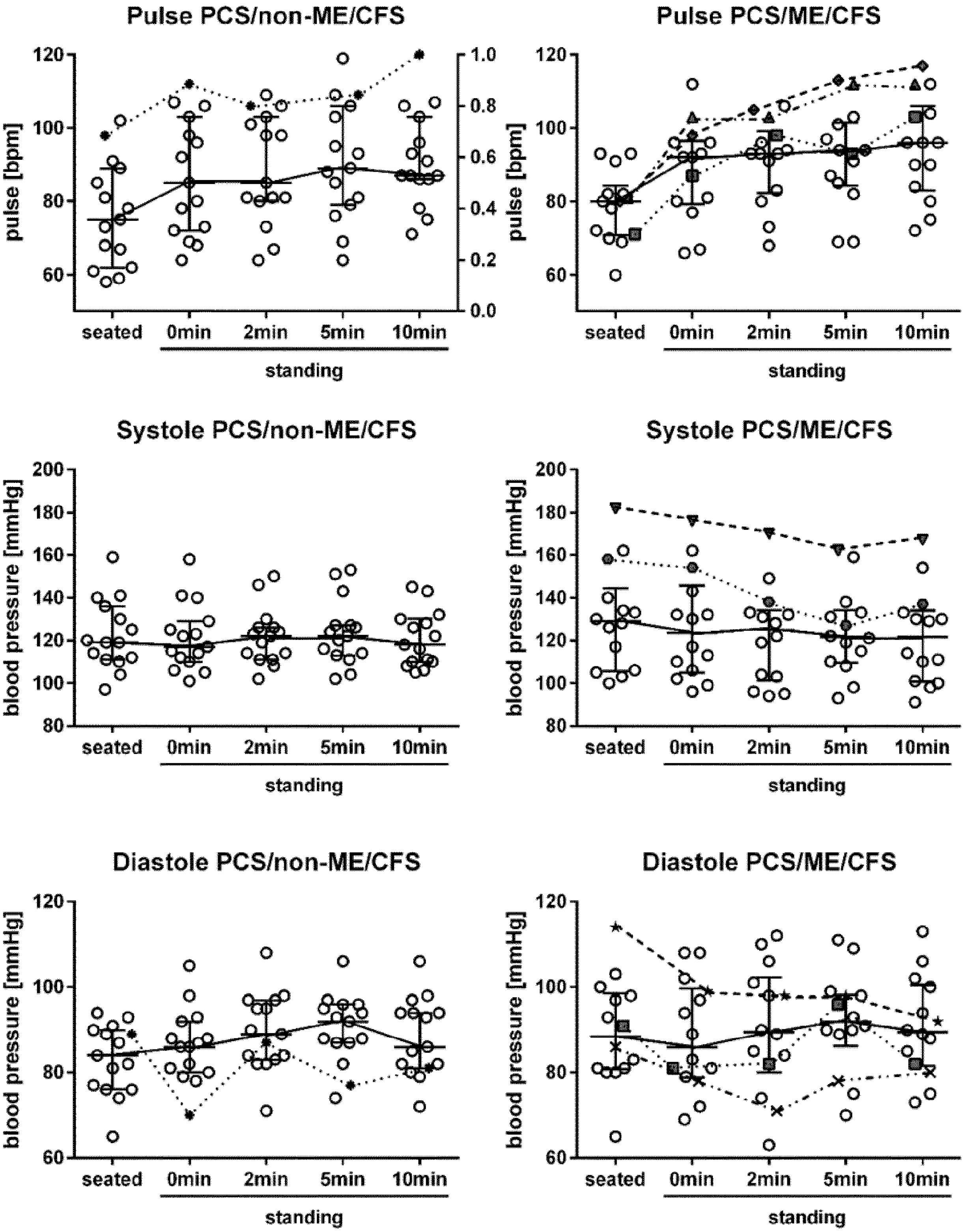

Pulse PCS/non-ME/CFS
pulse [bpm]
120
100
80
60
1.0
0.8
0.6
0.4
0.2
0.0
seated
0min
2min
5min
10min
standing
Pulse PCS/ME/CFS
pulse [bpm]
Systole PCS/non-ME/CFS
blood pressure [mmHg]
200
180
160
140
120
100
80
Systole PCS/ME/CFS
Diastole PCS/non-ME/CFS
Diastole PCS/ME/CFS

Fig. 5

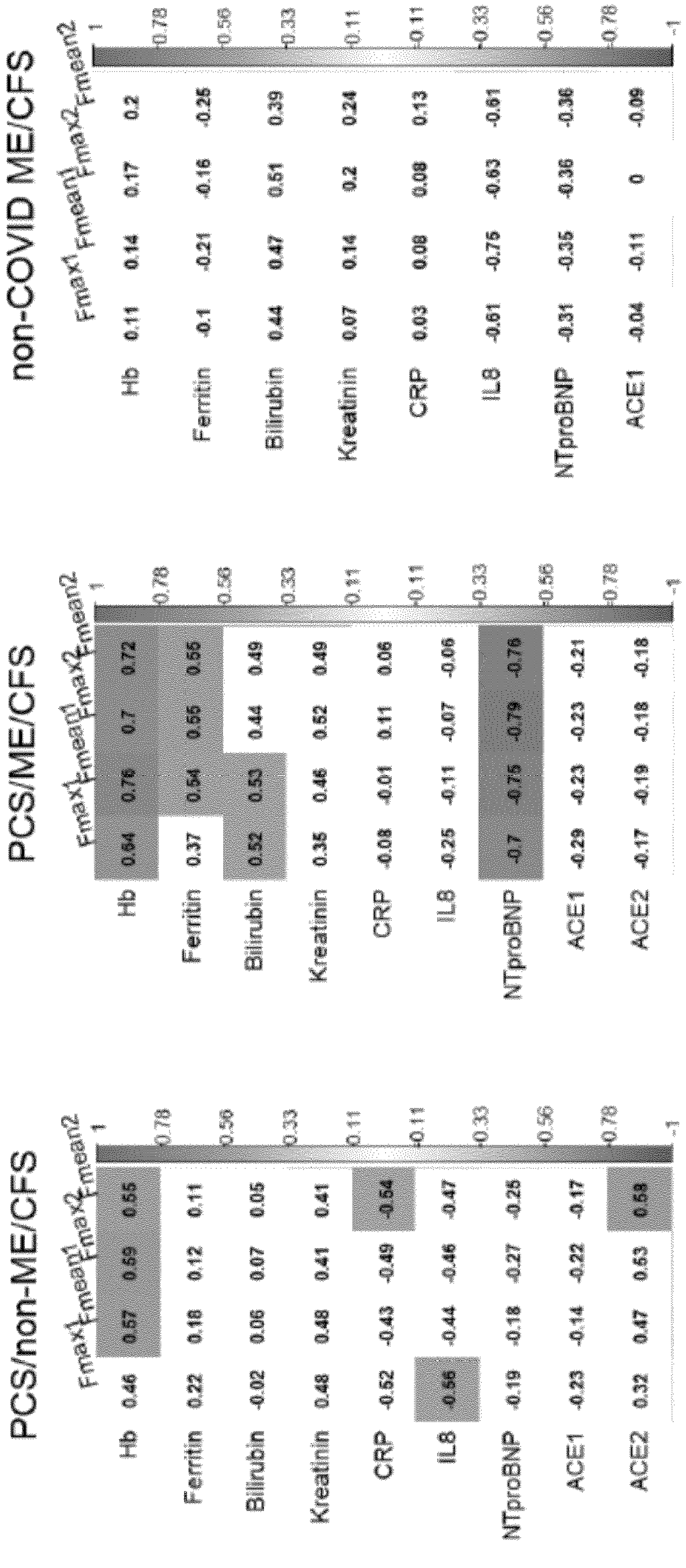
PCS/non-ME/CFS
PCS/ME/CFS
non-COVID ME/CFS
Fmax1
Fmean1
Fmax2
Fmean2
Hb
Ferritin
Bilirubin
Kreatinin
CRP
IL8
NTproBNP
ACE1
ACE2

Fig. 6

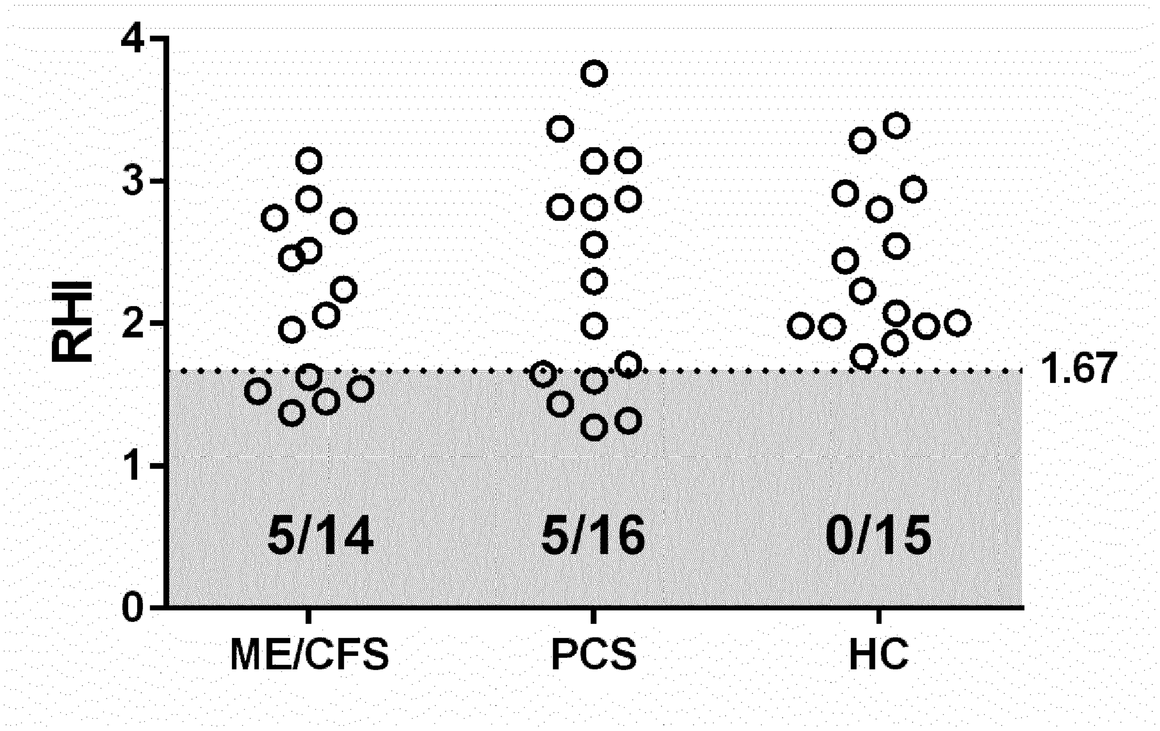

Fig. 7

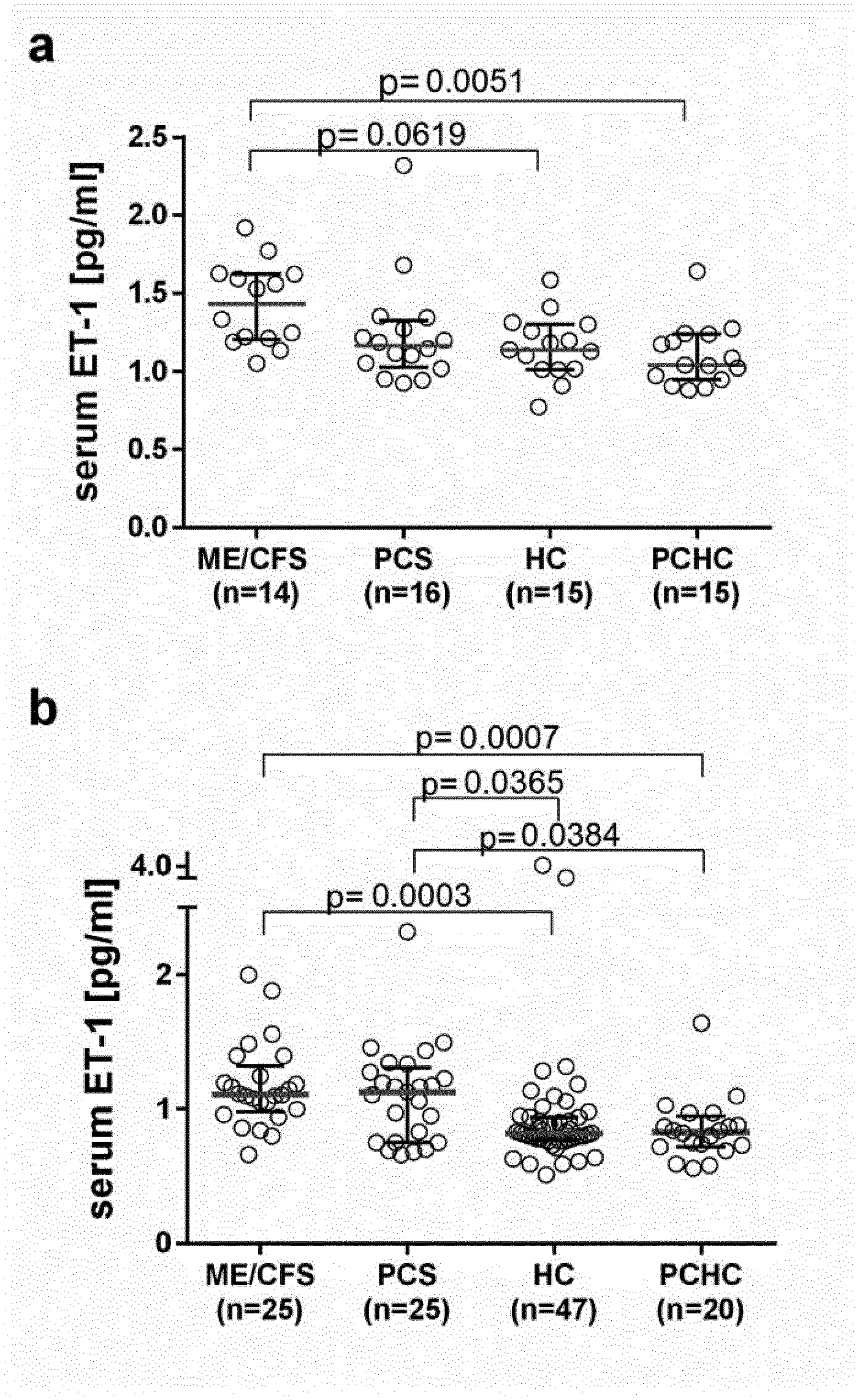
a
p= 0.0051
p= 0.0619
serum ET-1 [pg/ml]
2.5
2.0
1.5
1.0
0.5
0.0
ME/CFS (n=14)
PCS (n=16)
HC (n=15)
PCHC (n=15)
b
p= 0.0007
p= 0.0365
p= 0.0384
p= 0.0003
4.0
2
1
0
serum ET-1 [pg/ml]
ME/CFS (n=25)
PCS (n=25)
HC (n=47)
PCHC (n=20)

Fig. 8

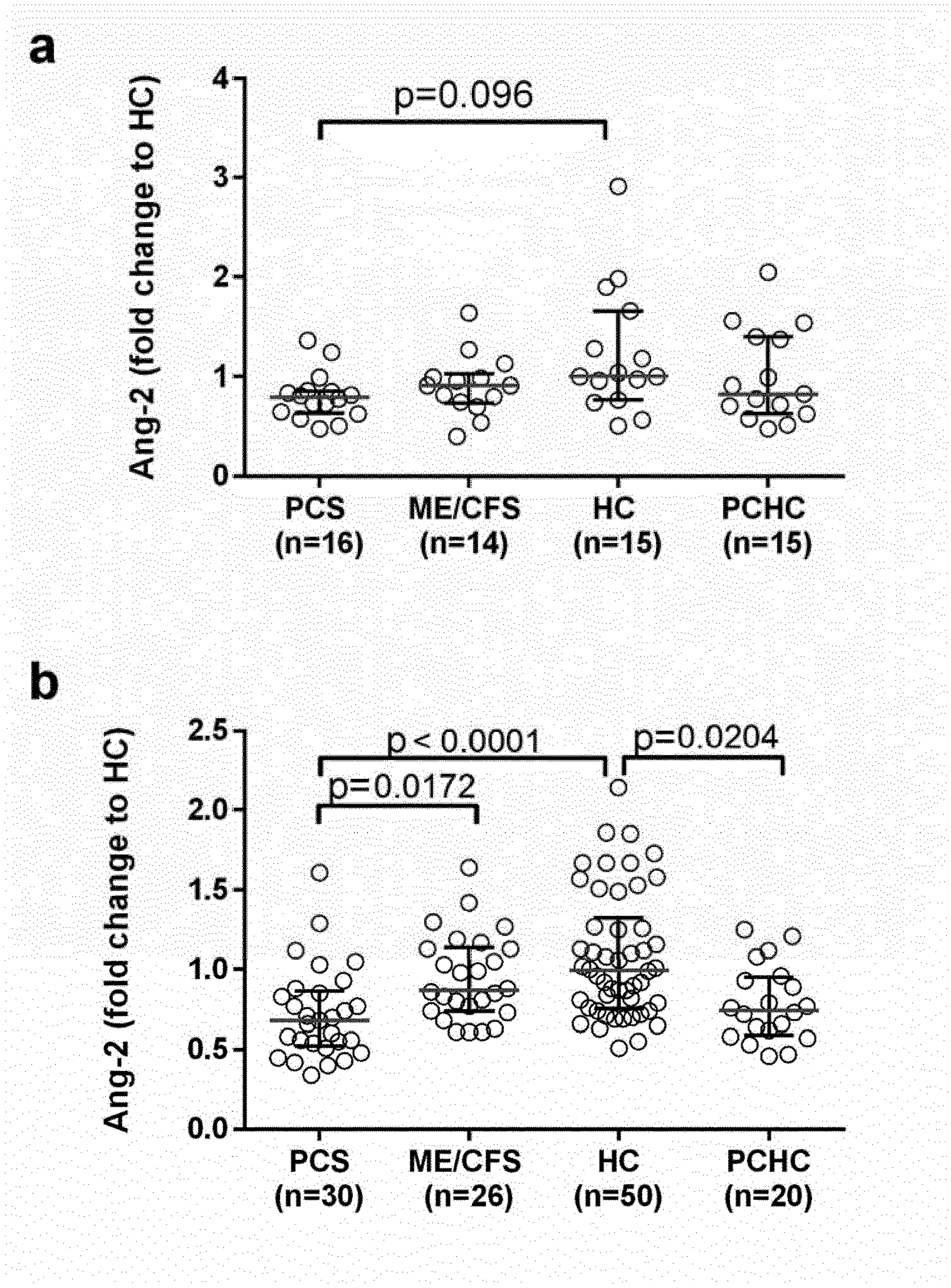
a
Ang-2 (fold change to HC)
p=0.096
PCS (n=16)
ME/CFS (n=14)
HC (n=15)
PCHC (n=15)
b
Ang-2 (fold change to HC)
p < 0.0001
p=0.0204
p=0.0172
PCS (n=30)
ME/CFS (n=26)
HC (n=50)
PCHC (n=20)

Europäisches Patentamt
European Patent Office
Office européen des brevets

# EUROPEAN SEARCH REPORT

Application Number
EP 22 15 8721

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2015/063287 A1 (BERGEN TEKNOLOGIOVERFØRING AS [NO])<br>7 May 2015 (2015-05-07)<br>* page 1, paragraph 1 *<br>* page 2, line 7 - line 8 *<br>* page 6, paragraph 2 - page 17, paragraph 2 *<br>* page 18, paragraph 3 - page 19, paragraph 1; claims; examples *<br>----- | 1-10,12, 13,15<br><br>11,14 | INV.<br>A61K31/00<br>A61K31/506<br>A61K31/5377<br>A61P9/00<br>A61P21/00<br>A61P25/28<br>A61P43/00 |
| Y | WONG TIMOTHY L. ET AL: "Long COVID and Myalgic Encephalomyelitis/Chronic Fatigue Syndrome (ME/CFS)-A Systemic Review and Comparison of Clinical Presentation and Symptomatology",<br>MEDICINA,<br>vol. 57, no. 5, 26 April 2021 (2021-04-26), page 418, XP055872731,<br>DOI: 10.3390/medicina57050418<br>* the whole document *<br>----- | 11,14 | |
| A | WO 2021/202546 A1 (CYCLERION THERAPEUTICS INC [US]) 7 October 2021 (2021-10-07)<br>* abstract; claims 6-15 *<br>* page 30, line 15 - line 29 *<br>----- | 1-15 | TECHNICAL FIELDS SEARCHED (IPC)<br>A61K<br>A61P |
| A | WO 2019/211081 A1 (BAYER AG [DE])<br>7 November 2019 (2019-11-07)<br>* abstract; claims *<br>----- | 1-15 | |

The present search report has been drawn up for all claims

1

| Place of search | Date of completion of the search | Examiner |
|---|---|---|
| The Hague | 11 July 2022 | Hoff, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 22 15 8721

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2022

| Patent document cited in search report | Publication date | Patent family member(s) | Publication date |
|---|---|---|---|
| WO 2015063287 A1 | 07-05-2015 | CN 105813637 A | 27-07-2016 |
| | | EP 3062780 A1 | 07-09-2016 |
| | | US 2016256460 A1 | 08-09-2016 |
| | | WO 2015063287 A1 | 07-05-2015 |
| WO 2021202546 A1 | 07-10-2021 | NONE | |
| WO 2019211081 A1 | 07-11-2019 | CA 3098475 A1 | 07-11-2019 |
| | | CN 112055584 A | 08-12-2020 |
| | | EP 3787610 A1 | 10-03-2021 |
| | | JP 2021522291 A | 30-08-2021 |
| | | US 2021052528 A1 | 25-02-2021 |
| | | WO 2019211081 A1 | 07-11-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003086407 A
- DE 19834044
- WO 2003095451 A
- DE 19834047
- DE 19942809

### Non-patent literature cited in the description


- **BOETTCHER, M. ; THOMAS, D. ; MUECK, W. et al.** Safety, pharmacodynamic, and pharmacokinetic characterization of vericiguat: results from six phase I studies in healthy subjects. *Eur J Clin Pharmacol,* 2021, vol. 77, 527-537
- *Eur J Clin Pharmacol,* 2021, vol. 77, 527-537
- **HARALD RENZ.** Praktische Labordiagnostik - Lehrbuch zur Laboratoriumsmedizin, klinische Chemie und Hämatologie. De Gruyter Verlag
- **CERAVOLO MG ; ARIENTI C ; DE SIRE A et al.** Rehabilitation and COVID-19: the CochraneRehabilitation 2020 rapid living systematic review. *Eur J Phys Rehabil Med,* 2020, vol. 56, 642-651
- **KEDOR C ; FREITAG H ; MEYER-ARNDT L ; WITTKE K ; ZOLLER T ; STEINBEIS F et al.** Chronic COVID-19 Syndrome and Chronic Fatigue Syndrome (ME/CFS) following the first pandemic wave in Germany - a first analysis of a prospective observational study. *medRxiv,* 2021
- **NACUL L,AUTHIER FJ, SCHEIBENBOGEN C, LORUSSO L, HELLAND IB, MARTIN JA.** European Network on Myalgic Encephalomyelitis/Chronic Fatigue Syndrome (EUROMENE): Expert Consensus on the Diagnosis, Service Provision, and Care of People with ME/CFS in Europe. *Medicina,* 2021, vol. 57 (5), 510
- **CARRUTHERS BM et al.** *Journal of Chronic Fatigue Syndrome,* 2003, vol. 11, 7-115
- **NACUL L et al.** European Network on Myalgic Encephalomyelitis/Chronic Fatigue Syndrome (EUROMENE): Expert Consensus on the Diagnosis, Service Provision, and Care of People with ME/CFS in Europe. *Medicina,* 19 May 2021, vol. 57 (5), 510
- **COTLER J ; HOLTZMAN C ; DUDUN C ; JASON LA.** A Brief Questionnaire to Assess Post-Exertional Malaise. *Diagnostics (Basel, Switzerland),* 2018, vol. 8 (3
- **CHALDER T ; BERELOWITZ G ; PAWLIKOWSKA T ; WATTS L ; WESSELY S ; WRIGHT D et al.** Development of a fatigue scale. *Journal of psychosomatic research,* 1993, vol. 37 (2), 147-53
- **SLETTEN et al.** COMPASS 31: a refined and abbreviated Composite Autonomic Symptom Score. *Mayo Clinic proceedings,* 2012, vol. 87 (12), 1196-201
- **SMITH A.** Symbol digit modalities test: Manual. Western Psychological Services, 1982
- **WARE, J.E., JR. ; SHERBOURNE, C.D.** *The MOS 36-item short-form health survey (SF-36)*
- **CELLA, M. ; CHALDER, T.** Measuring fatigue in clinical and community settings. *Journal of psychosomatic research,* 2010, vol. 69, 17-22
- **CHALDER T et al.** Development of a fatigue scale. *J Psychosom Res.,* 1993, vol. 37 (2), 147-53
- **CARRUTHERS ; BRUCE M. et al.** Myalgic Encephalomyelitis/Chronic Fatigue Syndrome: Clinical Working Case Definition, Diagnostic and Treatment Protocols. *Journal of Chronic Fatigue Syndrome,* 2003, vol. 11 (2), 7-115
- **JAKEL, B. et al.** Hand grip strength and fatigability: correlation with clinical parameters and diagnostic suitability in ME/CFS. *J Transl Med,* 2021, vol. 19, 159
- **BELL DS.** The Doctor's Guide to Chronic Fatigue Syndrome: Understanding, Treating and Living with CFIDS. Da Capo Lifelong Books, 1995
- **SUNNQUIST M ; LAZARUS S ; JASON LA.** The development of a short form of the DePaul Symptom Questionnaire. *Rehabil Psychol,* November 2019, vol. 64 (4), 453-462
- **SLETTEN DM et al.** COMPASS 31: A refined and abbreviated Composite Autonomic Symptom Score. *Mayo Clinic Proceedings,* 2012, vol. 87, 1196-120
- **NACUL L et al.** European Network on Myalgic Encephalomyelitis/Chronic Fatigue Syndrome (EUROMENE): Expert Consensus on the Diagnosis, Service Provision, and Care of People with ME/CFS in Europe. *Medicina,* 2021, vol. 57 (5), 510
- **STROBER LB et al.** *A new look at an old test: Normative data of the symbol digit modalities test - Oral version Multiple Sclerosis and Related Disorders,* August 2020, vol. 43, 102154

- **BAEVSKY RM ; CHERNIKOVA AG.** Heart rate variability analysis: physiological foundations and main methods. *Cardiometry,* 10 May 2017, 66-76
- **WAGNER J et al.** Functional aging in health and heart failure: the COmPLETE Study. *BMC Cardiovascular Disorderns,* 2019, vol. 19, 180
- **DE CARLO T E ; ROMANO A ; WAHEED N K ; DUKER J S.** A review of optical coherence tomography angiography (OCTA). *International Journal of Retina and Vitreous,* 2015, vol. 1, 5
- **BERG.** Pharmaceutical Salts. *J. Pharm. ScL,* 1977, vol. 66, 1-19
- **NACUL L ; AUTHIER FJ ; SCHEIBENBOGEN C et al.** EUROPEAN ME NETWORK (EUROMENE). *Expert Consensus on the Diagnosis, Service Provision and Care of People with ME/CFS in Europe,* 2020
- **SOTZNY, F. ; BLANCO, J. ; CAPELLI, E. ; CASTRO-MARRERO, J. ; STEINER, S. ; MUROVSKA, M. ; SCHEIBENBOGEN C.** European Network on, M. C.. Myalgic Encephalomyelitis/Chronic Fatigue Syndrome - Evidence for an autoimmune disease. *Autoimmun Rev.,* 2018, vol. 17, 601-609
- **STEINER S ; BECKER SC ; HARTWIG J et al.** Autoimmunity-Related Risk Variants in PTPN22 and CTLA4 Are Associated With ME/CFS With Infectious Onset. *Front Immunol,* 2020, vol. 11, 578
- **SZKLARSKI M ; FREITAG H ; LORENZ S ; BECKER SC ; SOTZNY F ; BAUER S ; HARTWIG J ; HEIDECKE H ; WITTKE K ; KEDOR C.** Delineating the Association Between Soluble CD26 and Autoantibodies Against G-Protein Coupled Receptors, Immunological and Cardiovascular Parameters Identifies Distinct Patterns in Post-Infectious vs. Non-Infection-Triggered Myalgic Encephalomyelitis/Chronic Fatigue Syndrome. *Front Immunol.,* 06 April 2021, vol. 12, 644548